# EUROPEAN PATENT SPECIFICATION

(11) **EP 0 915 880 B1**
(45) Date of publication and mention of the grant of the patent: **10.10.2007**
(21) Application number: 97936222.5
(22) Date of filing: 23.07.1997
(51) Int. Cl.: C07D 487/04, A61K 31/505

(54) **AZOLO TRIAZINES AND PYRIMIDINES**
AZOLOTRIAZINE UND PYRIMIDINE
AZOLO TRIAZINES ET PYRIMIDINES

(30) Priority: 24.07.1996 US 23290 P; 24.07.1996 US 686047
(43) Date of publication of application: 19.05.1999
(60) Divisional application: 07015514.8
(73) Proprietor: Bristol-Myers Squibb Pharma Company, Princeton, New Jersey 08443-4000 (US)
(72) Inventor: ARVANITIS, Argyrios, Georgious, Kennett Square, PA 19348 (US); CHORVAT, Robert, John, West Chester, PA 19382 (US); HE, Liqi, West Chester, Pennsylvania 19380 (US); GILLIGAN, Paul Joseph, Wilmington, Delaware 19810 (US); Liqi HE, Wilmington, Delaware 19807 (US); Paul Joseph Gilligan, West Chester, Pennsylvania 19380 (US)
(74) Representative: Reitstötter - Kinzebach
(86) International application number: PCT/US1997/013072
(87) International publication number: WO 1998/003510

(56) References cited:
- EP-A- 0 503 099
- EP-A- 0 531 901
- EP-A- 0 591 528
- EP-A- 0 714 898
- WO-A-94/13676
- WO-A-96/35689
- WO-A-97/29109
- US-A- 3 910 907
- US-A- 3 920 652
- US-A- 3 995 039
- US-A- 4 824 834
- US-A- 4 892 576
- US-A- 5 484 760
- CHEMICAL ABSTRACTS, vol. 68, no. 25, 17 June 1968 Columbus, Ohio, US; abstract no. 114635v, TAKAMIZAWA: "7-Haloacylaminopyrazolo(1,5-a)pyrimidines " XP002048048
- CHEMICAL ABSTRACTS, vol. 67, no. 23, 4 December 1967 Columbus, Ohio, US; abstract no. 108663r, TAKAMIZAWA: "7-Methylaminopyrazolo...." XP002048049
- CHEMICAL ABSTRACTS, vol. 74, no. 5, 1 February 1971 Columbus, Ohio, US; abstract no. 22872r, TAKAMIZAWA : "7-Aminoalkylaminopyrazolo..." XP002048050
- DATABASE WPI Section Ch, Week 9142 Derwent Publications Ltd., London, GB; Class B02, AN 91-306726 XP002048051 & JP 03 204 877 A (OTSUKA SEIYAKU KOGYO KK) , 6 September 1991
- DATABASE WPI Section Ch, Week 8618 Derwent Publications Ltd., London, GB; Class B02, AN 86-116678 XP002048052 & JP 61 057 587 A (SHIONOGI & CO LTD) , 24 March 1986
- J.MED.CHEM, vol. 25, 1982, pages 243-249,
- J.HETEROCYC.CHEM., vol. 22, 1985, pages 601-633,

## Description

### FIELD OF THE INVENTION

This invention relates a treatment of psychiatric disorders and neurological diseases including major depression, anxiety-related disorders, post-traumatic stress disorder, supranuclear palsy and feeding disorders as well as treatment of immunological, cardiovascular or heart-related diseases and colonic hypersensitivity associated with psychopathological disturbance and stress, by using certain [1,5-a]-pyrazolo-1,3,5-triazines.

### BACKGROUND OF THE INVENTION

Corticotropin releasing factor (herein referred to as CRF), a 41 amino acid peptide, is the primary physiological regulator of proopiomelanocortin(POMC) -derived peptide secretion from the anterior pituitary gland (J. Rivier et al., Proc. Nat. Acad. Sci. (USA) 80:4851 (1983); W. Vale et al., Science 213:1394 (1981)). In addition to its endocrine role at the pituitary gland, immunohistochemical localization of CRF has demonstrated that the hormone has a broad extrahypothalamic distribution in the central nervous system and produces a wide spectrum of autonomic, electrophysiological and behavioral effects consistent with a neurotransmitter or neuromodulator role in brain (W. Vale et al., Rec. Prog. Horm. Res. 39:245 (1983); G.F. Koob, Persp. Behav. Med. 2:39 (1985); E.B. De Souza et al., J: Neurosci. 5:3189 (1985)]. There is also evidence that CRF plays a significant role in integrating the response of the immune system to physiological, psychological, and immunological stressors (J.E. Blalock, Physiological Reviews 69:1 (1989); J.E. Morley, Life Sci. 41:527 (1987)).

Clinical data provide evidence that CRF has a role in psychiatric disorders and neurological diseases including depression, anxiety-related disorders and feeding disorders. A role for CRF has also been postulated in the etiology and pathophysiology of Alzheimer's disease, Parkinson's disease, Huntington's disease, progressive supranuclear palsy and amyotrophic lateral sclerosis as they relate to the dysfunction of CRF neurons in the central nervous system (for review see E.B. De Souza, Hosp. Practice 23:59 (1988)].

In affective disorder, or major depression, the concentration of CRF is significantly increased in the cerebral spinal fluid (CSF) of drug-free individuals [C.B. Nemeroff et al., Science 226:1342 (1984); C.M. Banki et al., Am. J. Psychiatry 144:873 (1987); R.D. France et al., Biol. Psychiatry 28:86 (1988); M. Arato et al., Biol Psychiatry 25:355 (1989)]. Furthermore, the density of CRF receptors is significantly decreased in the frontal cortex of suicide victims, consistent with a hypersecretion of CRF [C.B. Nemeroff et al., Arch. Gen. Psychiatry 45:577 (1988)]. In addition, there is a blunted adrenocorticotropin (ACTH) response to CRF (i.v. administered) observed in depressed patients [P.W. Gold et al., Am J. Psychiatry 141:619 (1984); F. Holsboer et al., Psychoneuroendocrinology 9:147 (1984); P.W. Gold et al., New Eng. J. Med. 314:1129 (1986)]. Preclinical studies in rats and non-human primates provide additional support for the hypothesis that hypersecretion of CRF may be involved in the symptoms seen in human depression [R.M. Sapolsky, Arch. Gen. Psychiatry 46:1047 (1989)]. There is preliminary evidence that tricyclic antidepressants can alter CRF levels and thus modulate the numbers of CRF receptors in brain [Grigoriadis et al., Neuropsychopharmacology 2:53 (1989)].

There has also been a role postulated for CRF in the etiology of anxiety-related disorders. CRF produces anxiogenic effects in animals and interactions between benzodiazepine / non-benzodiazepine anxiolytics and CRF have been demonstrated in a variety of behavioral anxiety models [D.R. Britton et al., Life Sci. 31:363 (1982); C.W. Berridge and A.J. Dunn Regul. Peptides 16:83 (1986)]. Preliminary studies using the putative CRF receptor antagonist a-helical ovine CRF (9-41) in a variety of behavioral paradigms demonstrate that the antagonist produces "anxiolytic-like" effects that are qualitatively similar to the benzodiazepines [C.W. Berridge and A.J. Dunn Horm. Behav. 21:393 (1987), Brain Research Reviews 15:71 (1990)]. Neurochemical, endocrine and receptor binding studies have all demonstrated interactions between CRF and benzodiazepine anxiolytics providing further evidence for the involvement of CRF in these disorders. Chlordiazepoxide attenuates the "anxiogenic" effects of CRF in both the conflict test [K.T. Britton et al., Psychopharmacology 86:170 (1985); K.T. Britton et al., Psychopharmacology 94:306 (1988)] and in the acoustic startle test [N.R. Swerdlow et al., Psychopharmacology 88:147 (1986)] in rats. The benzodiazepine receptor antagonist (Ro15-1788), which was without behavioral activity alone in the operant conflict test, reversed the effects of CRF in a dose-dependent manner while the benzodiazepine inverse agonist (FG7142) enhanced the actions of CRF [K.T. Britton et al., Psychopharmacology 94:306 (1988)].

The mechanisms and sites of action through which the standard anxiolytics and antidepressants produce their therapeutic effects remain to be elucidated. It has been hypothesized however, that they are involved in the suppression of the CRF hypersecretion that is observed in these disorders. Of particular interest is that preliminary studies examining the effects of a CRF receptor antagonist (α-helical CRF₉₋₄₁) in a variety of behavioral paradigms have demonstrated that the CRF antagonist produces "anxiolytic-like" effects qualitatively similar to the benzodiazepines [for review see G.F. Koob and K.T. Britton, In: Corticotropin-Releasing Factor: Basic and Clinical Studies of a Neuropeptide, E.B. De Souza and C.B. Nemeroff eds., CRC Press p221 (1990)].

Several publications describe corticotropin releasing factor antagonist compounds and their use to treat psychiatric disorders and neurological diseases. Examples of such publications include DuPont Merck PCT application US94/11050 , Pfizer WO 95/33750, Pfizer WO 95/34563, Pfizer WO 95/33727 and Pfizer EP 0778 277 Al.

Insofar as is known, [1,5-*a*]-pyrazolo-1,3,5-triazines, [1,5-*a*]-1,2,3-triazolo-1,3,5-triazines, [1,5-*a*]-pyrazolo-pyrimidines and [1,5-*a*]-1,2,3-triazolo-pyrimidines, have not been previously reported as corticotropin releasing factor antagonist compounds useful in the treatment of psychiatric disorders and neurological diseases. However, there have been publications which teach some of these compounds for other uses.

For instance, EP 0 269 859 (Ostuka, 1988) discloses pyrazolotriazine compounds of the formula 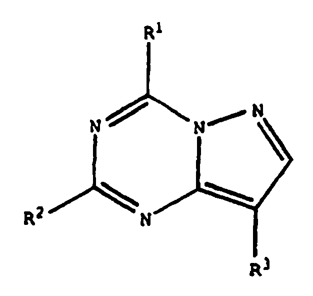 where R¹ is OH or alkanoyl, R² is H, OH, or SH, and R³ is an unsaturated heterocyclic group, naphthyl or substituted phenyl, and states that the compounds have xanthine oxidase inhibitory activity and are useful for treatment of gout.

EP 0 594 149 (Ostuka, 1994) discloses pyrazolotriazine and pyrazolopyrimidine compounds of the formula 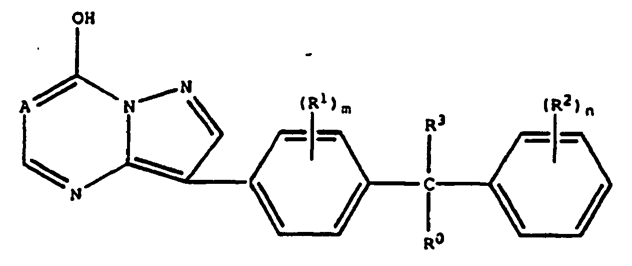 where A is CH or N, R⁰ and R³ are H or alkyl, and R¹ and R² are H, alkyl, alkoxyl, alkylthio, nitro, etc., and states that the compounds inhibit androgen and are useful in treatment of benign prostatic hypertrophy and prostatic carcinoma.

US 3,910,907 (ICI, 1975) discloses pyrazolotriazines of the formula: 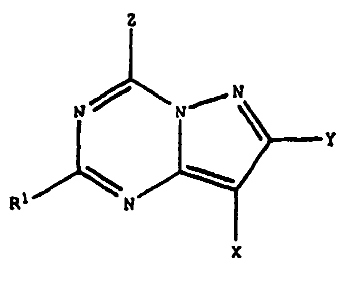 where R1 is CH₃, C₂H₅ or C₆H₅, X is H, C₆H₅, m-CH₃C₆H₄, CN, COOEt, Cl, I or Br, Y is H, C₆H₅, o-CH₃C₆H₄, or p-CH₃C₆H₄, and Z is OH, H, CH₃, C₂H₅, C₆H₅, n-C₃H₇, i-C₃H₇, SH, SCH₃, NHC₄H₉, or N(C₂H₅)₂, and states that the compounds are c-AMP phosphodiesterase inhibitors useful as bronchodilators.

US 3,995,039 discloses pyrazolotriazines of the formula: 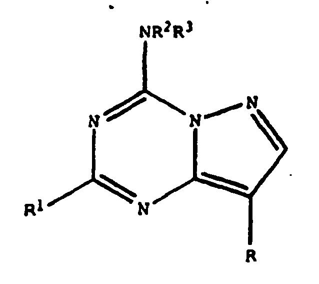 where R¹ is H or alkyl, R² is H or alkyl, R³ is H, alkyl, alkanoyl, carbamoyl, or lower alkylcarbamoyl, and R is pyridyl, pyrimidinyl, or pyrazinyl, and states that the compounds are useful as bronchodilators.

US 5,137,887 discloses pyrazolotriazines of the formula 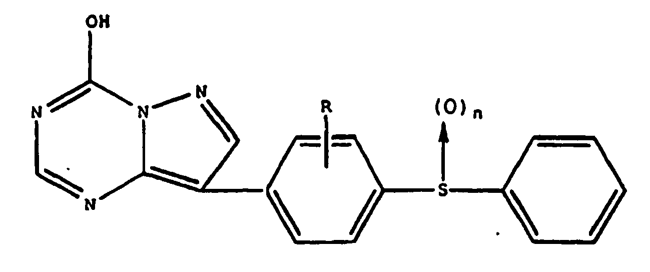 where R is lower alkoxy, and teaches that the compounds are xanthine oxidase inhibitors and are useful for treatment of gout.

US 4,892,576 discloses pyrazolotriazines of the formula 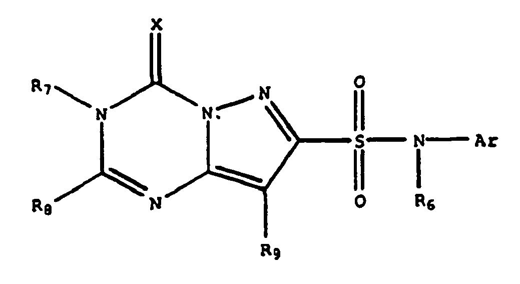 where X is O or S, Ar is a phenyl, naphthyl, pyridyl or thienyl group, R₆-R₈ are H, alkyl, etc., and R₉ is H, alkyl, phenyl, etc. The patent states that the compounds are useful as herbicides and plant growth regulants.

US 5,484,760 and WO 92/10098 discloses herbicidal compositions containing, among other things, a herbicidal compound of the formula 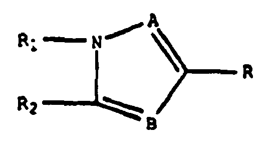 where A can be N, B can be CR₃, R₃ can be phenyl or substituted phenyl, etc., R is -N(R₄)SO₂R₅ or -SO₂N(R₆)R₇, and R₁ and R₂ can be taken together to form 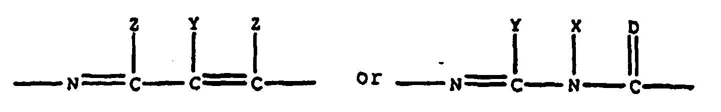 where X, Y and Z are H, alkyl, acyl, etc. and D is O or S.

US 3,910,907 and Senga et al., J. Med. Chem., 1982, 25, 243-249, disclose triazolotriazines cAMP phosphodiesterase inhibitors of the formula 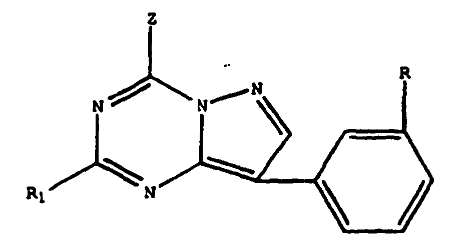 where Z is H, OH, CH₃, C₂H₅, C₆H₅, n-C₃H₇, iso-C₃H₇, SH, SCH₃, NH(n-C₄H₉), or N(C₂H₅)₂, R is H or CH₃, and R₁ is CH₃ or C₂H₅. The reference lists eight therapeutic areas where inhibitors of cAMP phosphodiesterase could have utility: asthma, diabetes mellitus, female fertility control, male infertility, psoriasis, thrombosis, anxiety, and hypertension.

WO95/35298 (Otsuka, 1995) discloses pyrazolopyrimidines and states that they are useful as analgesics. The compounds are represented by the formula 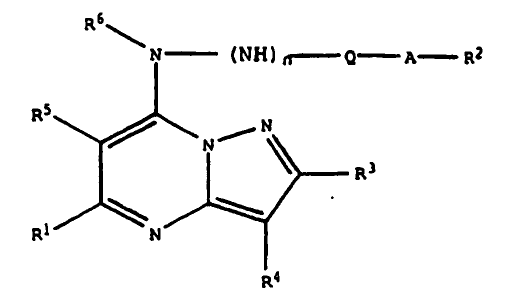 where Q is carbonyl or sulfonyl, n is 0 or 1, A is a single bond, alkylene or alkenylene, R¹ is H, alkyl, etc., R² is naphthyl, cycloalkyl, heteroaryl, substituted phenyl or phenoxy, R³ is H, alkyl or phenyl, R⁴ is H, alkyl, alkoxycarbonyl, phenylalkyl, optionally phenylthio-substituted phenyl, or halogen, R⁵ and R⁶ are H or alkyl.

EP 0 591 528 (Otsuka, 1991) discloses antiinflammatory use of pyrazolopyrimidines represented by the formula 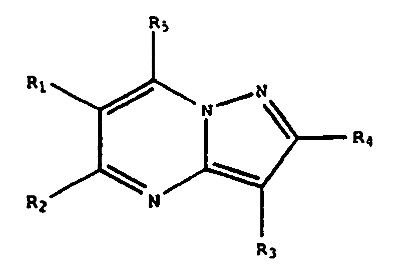 where R₁, R₂, R₃ and R₄ are H, carboxyl, alkoxycarbonyl, optionally substituted alkyl, cycloalkyl, or phenyl, R₅ is SR₆ or NR₇R₈, R₆ is pyridyl or optionally substituted phenyl, and R₇ and R₈ are H or optionally substituted phenyl.

Springer et al, J. Med. Chem., 1976, vol. 19, no. 2, 291-296 and Springer U.S. patents 4021,556 and 3,920,652 disclose pyrazolopyrimidines of the formula 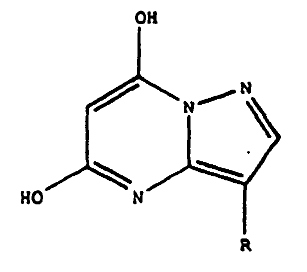 where R can be phenyl, substituted phenyl or pyridyl, and their use to treat gout, based on their ability to inhibit xanthine oxidase.

Joshi et al., J. Prakt. Chemie, 321, 2, 1979, 341-344, discloses compounds of the formula 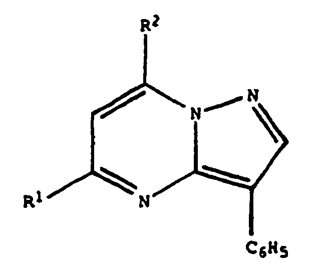 where R¹ is CF₃, C₂F₅, or C₆H₄F, and R² is CH₃, C₂H₅, CF₃, or C₆H₄F.

Maquestiau et al., Bull. Soc. Belg., vol.101, no. 2, 1992, pages 131-136 discloses a pyrazolo[1,5-*a*]pyrimidine of the formula

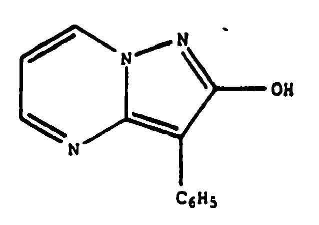

Ibrahim et al., Arch. Pharm. (weinheim) 320, 487-491 (1987) discloses pyrazolo[1,5-a]pyrimidines of the formula 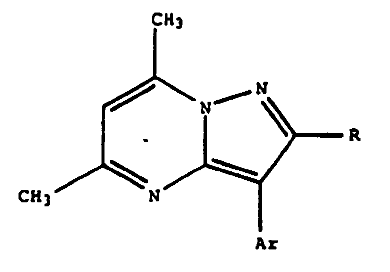 where R is NH2 or OH and Ar is 4-phenyl-3-cyano-2-aminopyrid-2-yl.

Other references which disclose azolopyrimidines inclued EP 0 511 528 (Otsuka, 1992), US 4,997,940 (Dow, 1991), EP 0 374 448 (Nissan, 1990), US 4,621,556 (ICN,1997), EP 0 531 901 (Fujisawa, 1993), US 4,567,263 (BASF, 1986), EP 0 662 477 (Isagro, 1995), DE 4 243 279 (Bayer, 1994), US 5,397,774 (Upjohn, 1995), EP 0 521 622 (Upjohn, 1993), WO 94/109017 (Upjohn, 1994), J. Med. Chem., 24, 610-613 (1981), and J. Het. Chem., 22, 601 (1985).

### SUMMARY OF THE INVENTION

In accordance with one aspect, the present invention provides novel compounds, pharmaceutical compositions and the use of said compounds in the treatment of affective disorder, anxiety, depression, irritable bowel syndrome, post-traumatic stress disorder, supranuclear palsy, immune suppression, Alzheimer's disease, gastrointestinal disease, anorexia nervosa or other feeding disorder, drug or alcohol withdrawal symptoms, drug addiction, inflammatory disorder, fertility problems, disorders, the treatment of which can be effected or facilitated by antagonizing CRF, including but not limited to disorders induced or facilitated by CRF, or a disorder selected from inflammatory disorders such as rheumatoid arthritis and osteoarthritis, pain, asthma, psoriasis and allergies; generalized anxiety disorder; panic, phobias, obsessive-compulsive disorder; post-traumatic stress disorder; sleep disorders induced by stress; pain perception such as fibromyalgia; mood disorders such as depression, including major depression, single episode depression, recurrent depression, child abuse induced depression, and postpartum depression; dysthemia; bipolar disorders; cyclothymia; fatigue syndrome; stress-induced headache; cancer, human immunodeficiency virus (HIV) infections; neurodegenerative diseases such as Alzheimer's disease, Parkinson's disease and Huntington's disease; gastrointestinal diseases such as ulcers, irritable bowel syndrome, Crohn's disease, spastic colon, diarrhea, and post operative ilius and colonic hypersensitivity associated by psychopathological disturbances or stress; eating disorders such as anorexia and bulimia nervosa; hemorrhagic stress; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; cardiovascular and hear related disorders including hypertension, tachycardia and congestive heart failure; stroke; immune dysfunctions including stress induced immune dysfunctions (e.g., stress induced fevers, porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; chemical dependencies and addictions (e.g., dependencies on alcohol, cocaine, heroin, benzodiazepines, or other drugs); drug and alcohol withdrawal symptoms; osteoporosis; psychosocial dwarfism and hypoglycemia in a mammal.

The present invention provides novel compounds which bind to corticotropin releasing factor receptors, thereby altering the anxiogenic effects of CRF secretion. The compounds of the present invention are useful for the treatment of psychiatric disorders and neurological diseases, anxiety-related disorders, post-traumatic stress disorder, supranuclear palsy and feeding disorders as well as treatment of immunological, cardiovascular or heart-related diseases and colonic hypersensitivity associated with psychopathological disturbance and stress in a mammal.

According to another aspect, the present invention provides novel-compounds of Formula (1) (described below) which are useful as antagonists of the corticotropin releasing factor. The compounds of the present invention exhibit activity as corticotropin releasing factor antagonists and appear to suppress CRF hypersecretion. The present invention also includes pharmaceutical compositions containing such compounds of Formula (1), and the use of such compounds for the suppression of CRF hypersecretion, and/or for the treatment of anxiogenic disorders.

According to yet another aspect of the invention, the compounds provided by this invention (and especially labelled compounds of this invention) are also useful as standards and reagents in determining the ability of a potential pharmaceutical to bind to the CRF receptor.

### DETAILED DESCRIPTION OF INVENTION

[1] The present invention comprises [1,5-a]-pyrazolo-1,3,5-triazines of the formula (1) 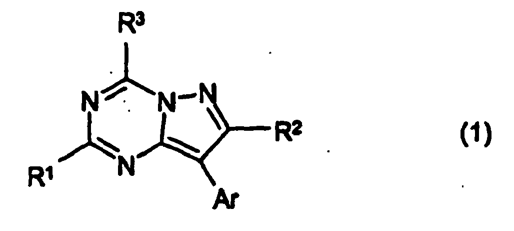 wherein
   - Ar: is phenyl, pyridyl or 2,3-dihydrobenzofuranyl, each (Ar) optionally substituted with 1 to 4 R⁴ substituents.
   - R¹: is independently selected at each occurrence from H, C₁-C₄ alkyl, C₂-C₄ alkenyl, C₂-C₄ alkynyl, halo, CN, C₁-C₄ haloalkyl, C₁-C₁₂ hydroxyalkyl, C₂-C₁₂ alkoxyalkyl, C₂-C₁₀ cyanoalkyl, C₃-C₆ cycloalkyl, C₄-C₁₀ cycloalkylalkyl, NR⁹R¹⁰, C₁-C₄ alkyl-NR⁹R¹⁰, NR⁹COR¹⁰, OR¹¹, SH or S(O)ₙR¹²;
   - R²: is selected from H, C₁-C₄ alkyl, C₂-C₄ alkenyl, C₂-C₄ alkynyl, C₃-C₆ cycloalkyl, C₄-C₁₀ cycloalkylalkyl, C₁-C₄ hydroxyalkyl, halo, CN, -NR⁶R⁷, NR⁹COR¹⁰, -NR⁶S(O)ₙR⁷, S(O)ₙNR⁶R⁷, C₁-C₄ haloalkyl, -OR⁷, SH or -S(O)ₙR¹²;
   - R³: is NR^{6a}R^{7a};
   - R⁴: is independently selected at each occurrence from: C₁-C₁₀ alkyl, C₂-C₁₀ alkenyl, C₂-C₁₀ alkynyl, C₃-C₆ cycloalkyl, C₄-C₁₂ cycloalkylalkyl, NO₂, halo, CN, C₁-C₄ haloalkyl, NR⁶R⁷, NR⁸COR⁷, NR⁸CO₂R⁷, COR⁷, OR⁷, CONR⁶R⁷, CO(NOR⁹)R⁷, CO₂R⁷, or S(O)ₙR⁷, where each such C₁-C₁₀ alkyl, C₂-C₁₀ alkenyl, C₂-C₁₀ alkynyl, C₃-C₆ cycloalkyl and C₄-C₁₂ cycloalkylalkyl are optionally substituted with 1 to 3 substituents independently selected at each occurrence from C₁-C₄ alkyl, NO₂, halo, CN, NR⁶R⁷, NR⁸COR⁷, NR⁸CO₂R⁷, COR⁷ OR⁷, CONR⁶R⁷, CO₂R⁷, CO(NOR⁹)R⁷, or S(O)ₙR⁷;
   - R⁶ and R⁷, R^{6a} and R^{7a}: are independently selected at each occurrence from:
   - H,
   - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl. C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
   - aryl, aryl(C₁-C₄ alkyl), heteroaryl, heteroaryl(C₁-C₄ alkyl), heterocyclyl or heterocyclyl(C₁-C₄ alkyl);
   alternatively, NR⁶R⁷ and NR^{6a}R^{7a} are independently piperidine, pyrrolidine, piperazine, N-methylpiperazine, morpholine or thiomorpholine, each optionally substituted with 1 to 3 C₁-C₄ alkyl groups;
   - R⁸: is independently selected at each occurrence from H or C₁-C₄ alkyl;
   - R⁹ and R¹⁰: are independently selected at each occurrence from H, C₁-C₄ alkyl, or C₃-C₆ cycloalkyl;
   - R¹¹: is selected from H, C₁-C₄ alkyl, C₁-C₄ haloalkyl, or C₃-C₆ cycloalkyl;
   - R¹²: is C₁-C₄ alkyl or C₁-C₄ haloalkyl;
   - R¹³: is selected from C₁-C₄ alkyl, C₁-C₄ haloalkyl, C₂C₈ alkoxyalkyl, C₃-C₆ cycloalkyl, C₄-C₁₂ cycloalkylalkyl, aryl, aryl(C₁-C₄ alkyl)-, heteroaryl or heteroaryl(C₁-C₄ alkyl)-;
   - R¹⁴: is selected from C₁-C₁₀ alkyl,C₃-C₁₀ alkenyl, C₃-C₁₀ alkynyl, C₃-C₈ cycloalkyl, or C₄-C₁₂ cycloalkylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, CONR¹⁶R¹⁵, and C₁-C₆ alkylthio, C₁-C₆ alkylsulfinyl and C₁-C₆ alkylsulfonyl;
   - R¹⁵ and R¹⁶: are independently selected at each occurrence from H, C₁-C₆ alkyl, C₃-C₁₀ cycloalkyl, C₄-C₁₆ cycloalkylalkyl, except that for S(O)ₙR¹⁵, R¹⁵ cannot be H;
   aryl is phenyl or naphthyl, each optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, and CONR¹⁶R¹⁵;
   heteroaryl is pyridyl, pyrimidinyl, triazinyl, furanyl, pyranyl, quinolinyl, isoquinolinyl, thienyl, imidazolyl, thiazolyl, indolyl, pyrrolyl, oxazolyl, benzofuranyl, benzothienyl, benzothiazolyl, isoxazolyl, pyrazolyl, 2,3 dihydrobenzothienyl or 2,3-dihydrobenzofuranyl, each being optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, -COR¹⁵, CO₂R¹⁵, CO(O)R¹⁵, NR⁸COR¹⁵, N(COR¹⁵)₂, NR⁸CONR¹⁶R¹⁵, NR⁸CO₂R¹⁵, NR¹⁶R¹⁵ and CONR¹⁶R¹⁵;
   heterocyclyl is saturated or partially saturated heteroaryl, optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁵R¹⁶, and CONR¹⁶R¹⁵;
   - n: is independently at each occurrence 0,1 or 2.
   with the provisos that
   (1) when R¹ is methyl or ethyl, R² is H, and R³ is NH(n-C₄H₉), or N(C₂H₅)₂, then Ar is not unsubstituted phenyl or m-methylphenyl; and
   (2) when R² is H, and Ar is pyridyl, and R³ is NR^{6a}R^{7a}, then R^{6a} and R^{7a} are not H or alkyl,
   and geometric Isomers thereof, stereoisomeric forms thereof, or mixtures of stereoisomeric forms thereof, and pharmaceutically acceptable salt or pro-drug forms thereof.
[2] The present invention further comprises the use of said compounds for treating affective disorder, anxiety, depression, headache, irritable bowel syndrome, post-traumatic stress disorder, supranuclear palsy, immune suppression, Alzheimer's disease, gastrointestinal diseases, anorexia nervosa or other feeding disorder, drug addiction, drug or alcohol withdrawal symptoms, inflammatory diseases, cardiovascular or heart-related diseases, fertility problems, human immunodeficiency virus infections, hemorrhagic stress, obesity, infertility, head and spinal cord traumas, epilepsy, stroke, ulcers, amyotrophic lateral sclerosis, hypoglycemia or a disorder the treatment of which can be effected or facilitated by antagonizing CRF, including but not limited to disorders induced or facilitated by CRF, in mammals.
[3] Preferred compounds of the above invention are compounds of Formula (1) and isomers thereof, stereoisomeric forms thereof, or mixtures of stereoisomeric forms thereof, and pharmaceutically acceptable salt or pro-drug forms thereof with the additional provisos that: (1) when R¹ is H, C₁-C₄ alkyl, halo, CN, C₁-C₁₂ hydroxyalkyl, C₁-C₄ alkoxyalkyl or SO₂(C₁-C₄ alkyl), and R^{6a} is unsubstituted C₁-C₄ alkyl, then R^{7a} is not phenyl, naphthyl, thienyl, benzothienyl, pyridyl, quinolyl, pyrazinyl, furanyl, benzofuranyl, benzothiazolyl, indolyl or C₃-C₆ cycloalkyl; and (2) R¹ is H, C₁-C₄ alkyl, halo, CN, C₁-C₁₂ hydroxyalkyl, C₁-C₄ alkoxyalkyl or SO₂(C₁-C₄ alkyl), and R^{7a} is unsubstituted C₁-C₄ alkyl, then R^{6a} is not phenyl, naphthyl, thienyl, benzothienyl, pyridyl, quinolyl, pyrazinyl, furanyl, benzofuranyl, benzothiazolyl, indolyl or C₃-C₆ cycloalkyl.
[4] Preferred compounds of the above invention also include compounds of Formula (1) and isomers thereof, stereoisomeric forms thereof, or mixtures of stereoisomeric forms thereof, and pharmaceutically acceptable salt or pro-drug forms thereof wherein Ar is phenyl, pyridyl or 2,3-dihydrobenzofuranyl, each optionally substituted with 1 to 4 R⁴ substituents.
[5] Preferred compounds of the above invention also include compounds of Formula (1) and isomers thereof, stereoisomeric forms thereof, or mixtures of stereoisomeric forms thereof, and pharmaceutically acceptable salt or pro-drug forms thereof wherein Ar is 2,4-dichlorophenyl, 2,4-dimethylphenyl or 2,4,6-trimethylphenyl, R¹ and R² are CH₃, and R³ is NR^{6a}R^{7a}.
[6] More preferred compounds of the above invention also include compounds and isomers thereof, stereoisomeric forms thereof, or mixtures of stereoisomeric forms thereof, and pharmaceutically acceptable salt or pro-drug forms thereof wherein
   R^{6a} is independently selected from:
      - H,
      - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl)-, heteroaryl, heteroaryl(C₁-C₄ alkyl)-, heterocyclyl or heterocyclyl(C₁-C₄ alkyl)-; and
   R^{7a} is independently selected at each occurrence from:
      - H,
      - C₅-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl), heteroaryl, heteroaryl(C₁-C₄ alkyl), heterocyclyl or heterocyclyl(C₁-C₄ alkyl);
   alternatively, NR⁶R⁷ and NR^{6a}R^{7a} are independently piperidine, pyrrolidine, piperazine, N-methylpiperazine, morpholine or thiomorpholine, each optionally substituted with 1-3 C₁-C₄ alkyl groups.
[7] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are identical and are selected from:
   - C₁-C₄ alkyl or C₃-C₆ cycloalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, -COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl, and
   - aryl or heteroaryl.
[8] More preferred compounds of the above invention also include compounds and isomers thereof, stereoisomeric forms thereof, or mixtures of stereoisomeric forms thereof, and pharmaceutically acceptable salt or pro-drug forms thereof wherein
   R^{6a} is selected from:
      - H,
      - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl), heteroaryl, heteroaryl(C₁-C₄ alkyl), heterocyclyl or heterocyclyl(C₁-C₄ alkyl);
   R^{7a} is selected from:
      - C₁-C₄ alkyl and each such C₁-C₄ alkyl is substituted with 1-3 substituents independently selected at each occurrence from C₁-C₆ alkyl, C₃-C₆ cycloalkyl, halo, C₁-C₄ haloalkyl, cyano, OR¹⁵, SH, S(O)nR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl.
[9] More preferred compounds of the above invention also include compounds and isomers thereof, stereoisomeric forms thereof, or mixtures of stereoisomeric forms thereof, and pharmaceutically acceptable salt or pro-drug forms thereof wherein one of R^{6a} and R^{7a} is selected from:
   - C₃-C₆ cycloalkyl, each such C₃-C₆ cycloalkyl optionally substituted with 1-3 substituents independently selected at each occurrence from C₁-C₆ alkyl, C₃-C₆ cycloalkyl, halo, C₁-C₄ haloalkyl, cyano, OR¹⁵, SH, S(O)nR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
   - aryl,
   - heteroaryl or
   - heterocyclyl,
   and the other of R^{6a} and R^{7a} is unsubstituted C₁-C₄ alkyl.
[10] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are independently H or C₁-C₁₀ alkyl, each such C₁-C₁₀ alkyl optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, R⁸CONR¹⁶R¹⁵, NR⁸CO₂R¹³, NR¹⁶R¹⁵, CONR¹⁶R¹⁵, aryl, heteroaryl or heterocyclyl.
[11] More preferred compounds of the above invention also include compounds and isomers thereof, stereoisomeric forms thereof, or mixtures of stereoisomeric forms thereof, and pharmaceutically acceptable salt or pro-drug forms thereof wherein
   R^{6a} is independently selected from:
      - H,
      - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl)-, heteroaryl, heteroaryl(C₁-C₄ alkyl), heterocyclyl or heterocyclyl(C₁-C₄ alkyl);
   R^{7a} is independently selected at each occurrence from:
      - H,
      - C₅-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³; COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl), heteroaryl, heteroaryl(C₁-C₄ alkyl), heterocyclyl or heterocyclyl(C₁-C₄ alkyl),
   alternatively, NR⁶R⁷ and NR^{6a}R^{7a} are independently piperidine, pyrrolidine, piperazine, N-methylpiperazine, morpholine or thiomorpholine, each optionally substituted with 1-3 C₁-C₄ alkyl groups.
[12] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are identical and are selected from:
   - C₁-C₄ alkyl or C₃-C₆ cycloalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, -COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl, and
   - aryl or heteroaryl.
[13] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are identical and are
   - C₁-C₄ alkyl, each such C₁-C₄ alkyl
      optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, -COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl.
[14] More preferred compounds of the above invention also include compounds and isomers thereof, stereoisomeric forms thereof, or mixtures of stereoisomeric forms thereof, and pharmaceutically acceptable salt or pro-drug forms thereof wherein
   R^{6a} is selected from:
      - H,
      - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl), heteroaryl, heteroaryl(C₁-C₄ alkyl), heterocyclyl or heterocyclyl(C₁-C₄ alkyl);
   R^{7a} is:
      - C₁-C₄ alkyl and each such C₁-C₄ alkyl is substituted with 1-3 substituents independently selected at each occurrence from C₁-C₆ alkyl, C₃-C₆ cycloalkyl, halo, C₁-C₄ haloalkyl, cyano, OR¹⁵, SH, S(O)nR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl.
[15] More preferred compounds of the above invention also include compounds and isomers thereof, stereoisomeric forms thereof, or mixtures of stereoisomeric forms thereof, and pharmaceutically acceptable salt or pro-drug forms thereof wherein one of R^{6a} and R^{7a} is selected from:
   - C₃-C₆ cycloalkyl, each such C₃-C₆ cycloalkyl optionally substituted with 1-3 substituents independently selected at each occurrence from C₁-C₆ alkyl, C₃-C₆ cycloalkyl, halo, C₁-C₄ haloalkyl, cyano, OR¹⁵, SH, S(O)nR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
   - aryl,
   - heteroaryl or
   - heterocyclyl,
   and the other of R^{6a} and R^{7a} is unsubstituted C₁-C₄ alkyl.
[16] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are independently H or C₁-C₁₀ alkyl, each such C₁-C₁₀ alkyl optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, R⁸CONR¹⁶R¹⁵, NR⁸CO₂R¹³, NR₁₆R¹⁵, CONR¹⁶R¹⁵, aryl, heteroaryl or heterocyclyl.
[17] More preferred compounds of the above invention also include compounds and isomers thereof, stereoisomeric forms thereof, or mixtures of stereoisomeric forms thereof, and pharmaceutically acceptable salt or pro-drug forms thereof wherein
   - Ar is phenyl, pyridyl or 2,3-dihydrobenzofuranyl, and each Ar is optionally substituted with 1 to 4 R⁴ substituents,
   - R³ is NR^{6a}R^{7a} and
   - R¹ and R² are independently selected from H, C₁-C₄ alkyl, C₃-C₆ cycloalkyl, C₄-C₁₀ cycloalkylalkyl.
[18] More preferred compounds of the above invention also include compounds and isomers thereof, stereoisomeric forms thereof, or mixtures of stereoisomeric forms thereof, and pharmaceutically acceptable salt or pro-drug forms thereof wherein
   R^{6a} is independently selected from:
      - H,
      - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl)-, heteroaryl, heteroaryl(C₁-C₄ alkyl), heterocyclyl or heterocyclyl(C₁-C₄ alkyl);
   R^{7a} is independently selected at each occurrence from:
      - H,
      - C₅-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl), heteroaryl, heteroaryl(C₁-C₄ alkyl), heterocyclyl or heterocyclyl(C₁-C₄ alkyl),
   alternatively, NR⁶R⁷ and NR^{6a}R^{7a} are independently piperidine, pyrrolidine, piperazine, N-methylpiperazine, morpholine or thiomorpholine, each optionally substituted with 1-3 C₁-C₄ alkyl groups.
[19] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are identical and are selected from:
   - C₁-C₄ alkyl or C₃-C₆ cycloalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, -COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl, and
   - aryl or heteroaryl.
[20] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are identical and are
   - C₁-C₄ alkyl, each such C₁-C₄ alkyl optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, -COR¹⁵, CO₂R¹⁵, OC(O)R¹³, NR⁸COR¹⁵, N(COR¹⁵)2, NR⁸CONR¹⁶R¹⁵, NR⁸CO₂R¹³, NR¹⁶R¹⁵, CONR¹⁶R¹⁵, aryl, heteroaryl or heterocyclyl.
[21] More preferred compounds of the above invention also include compounds and isomers thereof, stereoisomeric forms thereof, or mixtures of stereoisomeric forms thereof, and pharmaceutically acceptable salt or pro-drug forms thereof wherein
   R^{6a} is selected from:
      - H,
      - C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
      - aryl, aryl(C₁-C₄ alkyl), heteroaryl, heteroaryl(C₁-C₄ alkyl), heterocyclyl or heterocyclyl(C₁-C₄ alkyl);
   R^{7a} is:
      - C₁-C₄ alkyl and each such C₁-C₄ alkyl is substituted with 1-3 substituents independently selected at each occurrence from C₁-C₆ alkyl, C₃-C₆ cycloalkyl, halo, C₁-C₄ haloalkyl, cyano, OR¹⁵, SH, S(O)nR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl.
[22] More preferred compounds of the above invention also include compounds and isomers thereof, stereoisomeric forms thereof, or mixtures of stereoisomeric forms thereof, and pharmaceutically acceptable salt or pro-drug forms thereof wherein one.of R^{6a} and R^{7a} is selected from:
   - C₃-C₆ cycloalkyl, each such C₃-C₆ cycloalkyl optionally substituted with 1-3 substituents independently selected at each occurrence from C₁-C₆ alkyl, C₃-C₆ cycloalkyl, halo, C₁-C₄ haloalkyl, cyano, OR¹⁵, SH, S(O)nR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
   - aryl,
   - heteroaryl or
   - heterocyclyl,
   and the other of R^{6a} and R^{7a} is unsubstituted C₁-C₄ alkyl.
[23] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are independently H or C₁-C₁₀ alkyl, each such C₁-C₁₀ alkyl optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, R⁸CONR¹⁶R¹⁵, NR⁸CO₂R¹³, NR¹⁶R¹⁵, CONR¹⁶R¹⁵, aryl, heteroaryl or heterocyclyl.
[24] Specifically preferred compounds of the above invention are compounds of Formula (50) 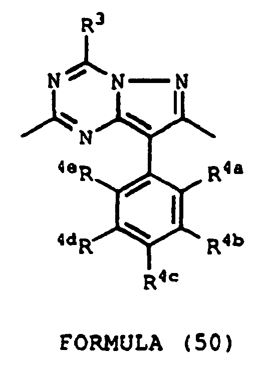 and isomers thereof, stereoisomeric forms thereof, or mixtures of stereoisomeric forms thereof, and pharmaceutically acceptable salt or pro-drug forms thereof, selected from the group consisting of:
   a compound of Formula (50) wherein R³ is -NHCH(n-Pr)₂, R^{4a} is Cl, R^{4b} is H, R^{4c} is Cl, R^{4d} is H and R^{4e} is H;
   a compound of Formula (50) wherein R³ is -N(Et)(n-Bu), R^{4a} is Cl, R^{4b} is H, R^{4c} is Cl, R^{4d} is H and R^{4e} is H;
   a compound of Formula (50) wherein R³ is -N(CH₂CH₂OMe)₂, R^{4a} is Cl, R^{4b} is H, R^{4c} is Cl, R^{4d} is H and R^{4e} is H;
   a compound of Formula (50) wherein R³ is -NHCH(Et)(n-Bu), R^{4a} is Cl, R^{4b} is H, R^{4c} is Cl, R^{4d} is H and R^{4e} is H;
   a compound of Formula (50) wherein R³ is -NHCH(Et)(CH₂OMe), R^{4a} is Cl, R^{4b} is H, R^{4c} is Cl, R^{4d} is H and R^{4e} is H;
   a compound of Formula (50) wherein R³ is -NHCH(CH₂OMe)₂, R^{4a} is Cl, R^{4b} is H, R^{4c} is Cl, R^{4d} is H and R^{4e} is H;
   a compound of Formula (50) wherein R³ is -N(Et)₂, R^{4a} is Cl, R^{4b} is H, R^{4c} is Cl, R^{4d} is H and R^{4e} is H;
   a compound of Formula (50) wherein R³ is -NHCH(CH₂OEt)₂, R^{4a} is Cl, R^{4b} is H, R^{4c} is Cl, R^{4d} is H and R^{4e} is H;
   a compound of Formula (50) wherein R³ is -NHCH(Et)₂, R^{4a} is Cl, R^{4b} is H, R^{4c} is Cl, R^{4d} is H and R^{4e} is H;
   a compound of Formula (50) wherein R³ is -N(Me)(Ph), R^{4a} is Cl, R^{4b} is H, R^{4c} is Cl, R^{4d} is H and R^{4e} is H;
   a compound of Formula (50) wherein R³ is -N(n-Pr)₂, R^{4a} is Cl, R^{4b} is H, R^{4c} is Cl, R^{4d} is H and R^{4e} is H;
   a compound of Formula (50) wherein R³ is -NHCH(Et)(n-Pr), R^{4a} is Cl, R^{4b} is H, R^{4c} is Cl, R^{4d} is H and R^{4e} is H;
   a compound of Formula (50) wherein R³ is -NHCH(CH₂OMe)₂, R^{4a} is Me, R^{4b} is H, R^{4c} is Me, R^{4d} is H and R^{4e} is Me;
   a compound of Formula (50) wherein R³ is -NHCH(CH₂OMe)₂, R^{4a} is Me, R^{4b} is H, R^{4c} is Me, R^{4d} is H and R^{4e} is H;
   a compound of Formula (50) wherein R³ is -N(CH₂CH₂OMe)₂, R^{4a} is Me, R^{4b} is H, R^{4c} is Me, R^{4d} is H and R^{4e} is H;
   a compound of Formula (50) wherein R³ is -NHCH(Et)(CH₂OMe), R^{4a} is Me, R^{4b} is H, R^{4c} is Me, R^{4d} is H and R^{4e} is H;
   a compound of Formula (50) wherein R³ is -NHCH(Et)₂, R^{4a} is Me, R^{4b} is H, R^{4c} is Me, R^{4d} is H and R^{4e} is H;
   a compound of Formula (50) wherein R³ is -N(Et)₂, R^{4a} is Me, R^{4b} is H, R^{4c} is Me, R^{4d} is H and R^{4e} is H;
   a compound of Formula (50) wherein R³ is -N(CH₂CN)₂, R^{4a} is Me, R^{4b} is H, R^{4c} is Me, R^{4d} is H and R^{4e} is H;
   a compound of Formula (50) wherein R³ is -NHCH(Me)(CH₂OMe), R^{4a} is Me, R^{4b} is H, R^{4c} is Me, R^{4d} is Hand R^{4e} is H;
   a compound of Formula (50) wherein R³ is -N(n-Pr) (CH₂cPr), R^{4a} is Me, R^{4b} is H, R^{4c} is Me, R^{4d} is H and R^{4e} is H;
   a compound of Formula (50) wherein R³ is -NHCH(Me) (CH₂N(Me)₂), R^{4a} is Me, R^{4b} is H, R^{4c} is Me, R^{4d} is H and R^{4e} is H;
   a compound of Formula (50) wherein R³ is -N(cPr) (CH₂CH₂CN), R^{4a} is Me, R^{4b} is H, R^{4c} is Me, R^{4d} is H and R^{4e} is H;
   a compound of Formula (50) wherein R³ is -N(n-Pr)(CH₂CH₂CN), R^{4a} is Me, R^{4b} is H, R^{4c} is Me, R^{4d} is H and R^{4e} is H;
   a compound of Formula (50) wherein R³ is -N(n-Bu) (CH₂CN), R^{4a} is Me, R^{4b} is H, R^{4c} is Me, R^{4d} is H and R^{4e} is H;
   a compound of Formula (50) wherein R³ is -NHCH (Et) (CH₂OMe), R^{4a} is Me, R^{4b} is H, R^{4c} is Me, R^{4d} is H and R^{4e} is Me;
   a compound of Formula (50) wherein R³ is -NHCH(Et)₂, R^{4a} is Me, R^{4b} is H, R^{4c} is Me, R^{4d} is H and R^{4e} is Me;
   a compound of Formula (50) wherein R³ is -N(CH₂CH₂OMe)₂, R^{4a} is Me, R^{4b} is H, R^{4c} is Me, R^{4d} is H and R^{4e} is Me;
   a compound of Formula (50) wherein R³ is -NHCH(CH₂OMe)₂, R^{4a} is Br, R^{4b} is H, R^{4c} is OMe, R^{4d} is H and R^{4e} is H;
   a compound of Formula (50) wherein R³ is -NHCH(Et)(CH₂OMe), R^{4a} is Br, R^{4b} is H, R^{4c} is OMe, R^{4d} is H and R^{4e} is H;
   a compound of Formula (50) wherein R³ is -N(Et)₂, R^{4a} is Me, R^{4b} is H, R^{4c} is Me, R^{4d} is H and R^{4e} is Me;
   a compound of Formula (50) wherein R³ is -NHCH(CH₂OEt)₂, R^{4a} is Me, R^{4b} is H, R^{4c} is Me, R^{4d} is H and R^{4e} is Me;
   a compound of Formula (50) wherein R³ is -NHCH(CH₂CH2OMe) (CH₂OMe)₂, R^{4a} is Me, R^{4b} is H, R^{4c} is Me, R^{4d} is H and R^{4e} is Me;
   a compound of Formula (50) wherein R³ is morpholino, R^{4a} is Me, R^{4b} is H, R^{4c} is Me, R^{4d} is H and R^{4e} is H;
   a compound of Formula (50) wherein R³ is -N(CH₂CH₂OMe)₂, R^{4a} is Br, R^{4b} is H, R^{4c} is OMe, R^{4d} is H and R^{4e} is H;
   a compound of Formula (50) wherein R³ is -NHCH(Et)₂, R^{4a} is Br, R^{4b} is H, R^{4c} is OMe, R^{4d} is H and R^{4e} is H;
   a compound of Formula (50) wherein R³ is -N(Et)₂, R^{4a} is Br, R^{4b} is H, R^{4c} is OMe, R^{4d} is H and R^{4e} is H;
   a compound of Formula (50) wherein R³ is -NH(c-Pr), R^{4a} is Me, R^{4b} is H, R^{4c} is Me, R^{4d} is H and R^{4e} is H;
   a compound of Formula (50) wherein R³ is -NHCH(CH₂OMe)₂, R^{4a} is CN, R^{4b} is H, R^{4c} is OMe, R^{4d} is H and R^{4e} is H;
   a compound of Formula (50) wherein R³ is -N(c-Pr) (CH₂CH₂CN), R^{4a} is Me, R^{4b} is H, R^{4c} is Me, R^{4d} is H and R^{4e} is Me;
   a compound of Formula (50) wherein R³ is -NCH(CH₂OMe)₂, R^{4a} is Me, R^{4b} is H, R^{4c} is Br, R^{4d} is H and R^{4e} is H;
   a compound of Formula (50) wherein R³ is -NHCH(CH₂OMe)(CH₂CH₂OMe), R^{4a} is Me, R^{4b} is H, R^{4c} is Br, R^{4d} is H and R^{4e} is H;
   a compound of Formula (50) wherein R³ is -NHCH(CH₂OMe)₂, R^{4a} is Me, R^{4b} is H, R^{4c} is OMe, R^{4d} is Me and R^{4e} is H;
   a compound of Formula (50) wherein R³ is -N(CH₂CH₂OMe)₂ R^{4a} is Me, R^{4b} is H, R^{4c} is OMe, R^{4d} is Me and R^{4e} is H;
   a compound of Formula (50) wherein R³ is -NHCH(Et)₂, R^{4a} is Me, R^{4b} is H, R^{4c} is OMe, R^{4d} is Me and R^{4e} is H;
   a compound of Formula (50) wherein a compound of Formula (50) wherein R³ is -N(Et)₂, R^{4a} is Me, R^{4b} is H, R^{4c} is OMe, R^{4d} is Me and R^{4e} is H;
   a compound of Formula (50) wherein R³ is -NHCH(CH₂OMe)₂, R^{4a} is Cl, R^{4b} is H, R^{4c} is Me, R^{4d} is H and R^{4e} is H;
   a compound of Formula (50) wherein R³ is -NHCH(Et)(CH₂OMe), R^{4a} is Cl, R^{4b} is H, R^{4c} is Me, R^{4d} is H and R^{4e} is H;
   a compound of Formula (50) wherein R³ is -N(CH₂CH₂OMe)₂, R^{4a} is Cl, R^{4b} is H, R^{4c} is Me, R^{4d} is H and R^{4e} is H;
   a compound of Formula (50) wherein R³ is -NHCH(CH₂OMe)(CH₂CH₂OMe), R^{4a} is Cl, R^{4b} is H, R^{4c} is Me, R^{4d} is H and R^{4e} is H;
   a compound of Formula (50) wherein R³ is -N(c-Pr)(CH₂CH₂CN), R^{4a} is Me, R^{4b} is H, R^{4c} is OMe, R^{4d} is Me and R^{4e} is H;
   a compound of Formula (50) wherein R³ is -N(c-Pr)(CH₂CH₂CN), R^{4a} is Cl, R^{4b} is H, R^{4c} is Cl, R^{4d} is H and R^{4e} is H;
   a compound of Formula (50) wherein R³ is (*S*)-NHCH(CH₂OMe)(CH₂CH₂OMe), R^{4a} is Cl, R^{4b} is H, R^{4c} is Cl, R^{4d} is H and R^{4e} is H;
   a compound of Formula (50) wherein R³ is -NHCH(CH₂OMe)(CH₂CH₂OMe), R^{4a} is Cl, R^{4b} is H, R^{4c} is Cl, R^{4d} is H and R^{4e} is H;
   a compound of Formula (50) wherein R³ is -NHCH(Et)₂, R^{4a} is Me, R^{4b} is H, R^{4c} is Br, R^{4d} is H and R^{4e} is H;
   a compound of Formula (50) wherein R³ is -N(CH₂CH₂OMe)₂, R^{4a} is Me, R^{4b} is H, R^{4c} is Br, R^{4d} is H and R^{4e} is H;
   a compound of Formula (50) wherein R³ is -NH(CH₂OMe)(CH₂-iPr), R^{4a} is Me, R^{4b} is H, R^{4c} is Me, R^{4d} is H and R^{4e} is H;
   a compound of Formula (50) wherein R³ is -N(CH₂CH₂OMe)₂, R^{4a} is Me, R^{4b} is H, R^{4c} is H, R^{4d} is H and R^{4e} is H;
   a compound of Formula (50) wherein R³ is -N(CH₂CH₂OMe)₂, R^{4a} is Me, R^{4b} is H, R^{4c} is NMe₂, R^{4d} is H and R^{4e} is H;
   a compound of Formula (50) wherein R³ is -NHCH(CH₂OMe) (n-Pr), R^{4a} is Me, R^{4b} is H, R^{4c} is Me, R^{4d} is H and R^{4e} is H;
   a compound of Formula (50) wherein R³ is -NHCH(CH₂OEt) (Et), R^{4a} is Me, R^{4b} is H, R^{4c} is Me, R^{4d} is H and R^{4e} is H;
   a compound of Formula (50) wherein R³ is -NHCH(CH₂OMe) (CH₂CH₂OMe), R^{4a} is Me, R^{4b} is H, R^{4c} is NMe2, R^{4d} is H and R^{4e} is H;
   a compound of Formula (50) wherein R³ is -N(Et)₂, R^{4a} is Me, R^{4b} is H, R^{4c} is Cl, R^{4d} is H and R^{4e} is H;
   a compound of Formula (50) wherein R³ is -NHCH(Et)₂, R^{4a} is Me, R^{4b} is H, R^{4c} is Cl, R^{4d} is H and R^{4e} is H;
   a compound of Formula (50) wherein R³ is -N(CH₂CH₂OMe)₂, R^{4a} is Me, R^{4b} is H, R^{4c} is Cl, R^{4d} is H and R^{4e} is H;
   a compound of Formula (50) wherein R³ is -NHCH(CH₂OMe)₂, R^{4a} is Me, R^{4b} is H, R^{4c} is Cl, R^{4d} is H and R^{4e} is H;
   a compound of Formula (50) wherein R³ is -N(Et)₂, R^{4a} is Me, R^{4b} is H, R^{4c} is Br, R^{4d} is H and R^{4e} is H;
   a compound of Formula (50) wherein R³ is -N(Et)₂, R^{4a} is Cl, R^{4b} is H, R^{4c} is Me, R^{4d} is H and R^{4e} is H;
   a compound of Formula (50) wherein R³ is -NHCH(Et)₂, R^{4a} is Cl, R^{4b} is H, R^{4c} is Me, R^{4d} is H and R^{4e} is H;
   a compound of Formula (50) wherein R³ is -NHCH(Et)₂, R^{4a} is Me, R^{4b} is H, R^{4c} is NMe2, R^{4d} is H and R^{4e} is H;
   a compound of Formula (50) wherein R³ is (*S*)-NHCH(CH₂OMe)(CH₂CH₂OMe), R^{4a} is Me, R^{4b} is H, R^{4c} is Me, R^{4d} is H and R^{4e} is H;
   a compound of Formula (50) wherein R³ is -NHCH(CH₂OMe)(CH₂CH₂OMe), R^{4a} is Me, R^{4b} is H, R^{4c} is Me, R^{4d} is H and R^{4e} is H;
   a compound of Formula (50) wherein R³ is (*S*)-NHCH(CH₂OMe)(CH₂CH₂OMe), R^{4a} is Me, R^{4b} is H, R^{4c} is Cl, R^{4d} is H and R^{4e} is H;
   a compound of Formula (50) wherein R³ is -NHCH(CH₂OMe)(CH₂CH₂OMe), R^{4a} is Me, R^{4b} is H, R^{4c} is Cl, R^{4d} is H and R^{4e} is H;
   a compound of Formula (50) wherein R³ is -N(c-Pr)(CH₂CH₂CN), R^{4a} is Me, R^{4b} is H, R^{4c} is Cl, R^{4d} is H and R^{4e} is H;
   a compound of Formula (50) wherein R³ is -NH(Et) (CH₂CN), R^{4a} is Me, R^{4b} is H, R^{4c} is Cl, R^{4d} is H and R^{4e} is H;
   a compound of Formula (50) wherein R³ is -N(Et)₂, R^{4a} is Me, R^{4b} is Me, R^{4c} is OMe, R^{4d} is H and R^{4e} is H;
   a compound of Formula (50) wherein R³ is -N(CH₂CH₂OMe)(CH₂CH₂OH), R^{4a} is Cl, R^{4b} is H, R^{4c} is Cl, R^{4d} is H and R^{4e} is H;
   a compound of Formula (50) wherein R³ is -N(CH₂CH₂OMe)₂, R^{4a} is Me, R^{4b} is Me, R^{4c} is OMe, R^{4d} is H and R^{4e} is H;
   a compound of Formula (50) wherein R³ is -NHCH(Et)₂, R^{4a} is Me, R^{4b} is Me, R^{4c} is OMe, R^{4d} is H and R^{4e} is H;
   a compound of Formula (50) wherein R³ is -N(CH₂c-Pr) (n-Pr), R^{4a} is Me, R^{4b} is H, R^{4c} is Cl, R^{4d} is H and R^{4e} is H;
   a compound of Formula (50) wherein R³ is -N(c-Pr) (CH₂CH₂CN), R^{4a} is Me, R^{4b} is Me, R^{4c} is OMe, R^{4d} is H and R^{4e} is H;
   a compound of Formula (50) wherein R³ is -NHCH(Et)₂, R^{4a} is Cl, R^{4b} is H, R^{4c} is OMe, R^{4d} is H and R^{4e} is H;
   a compound of Formula (50) wherein R³ is -N(Et)₂, R^{4a} is Cl, R^{4b} is H, R^{4c} is OMe, R^{4d} is H and R^{4e} is H;
   a compound of Formula (50) wherein R³ is -N(CH₂CH₂OMe)₂, R^{4a} is Cl, R^{4b} is H, R^{4c} is OMe, R^{4d} is H and R^{4e} is H;
   a compound of Formula (50) wherein R³ is -NHCH(Et)(CH₂OMe), R^{4a} is Cl, R^{4b} is H, R^{4c} is OMe, R^{4d} is H and R^{4e} is H;
   a compound of Formula (50) wherein R³ is -N(Et)₂, R^{4a} is Cl, R^{4b} is H, R^{4c} is CN, R^{4d} is H and R^{4e} is H;
   a compound of Formula (50) wherein R³ is -N(c-Pr)(CH₂CH₂CN), R^{4a} is Cl, R^{4b} is H, R^{4c} is OMe, R^{4d} is H and R^{4e} is H;
   a compound of Formula (50) wherein R³ is -NHCH(CH₂OH)₂, R^{4a} is Cl, R^{4b} is H, R^{4c} is Cl, R^{4d} is H and R^{4e} is H; and
   a compound of Formula (50) wherein R³ is N(CH₂CH₂OMe)₂, R^{4a} is Me, R^{4b} is H, R^{4c} is OMe, R^{4d} is H and R^{4e} is H.
[25] More specifically preferred is 4-(bis-(2-methoxyethyl)amino)-2,7-dimethyl-8-(2-methyl-4-methoxyphenyl)-[1,5-a]-pyrazolo-1,3,5-triazine and isomers thereof, stereoisomeric forms thereof, or mixtures of stereoisomeric forms thereof, and pharmaceutically acceptable salt or pro-drug forms thereof.
[26] More specifically preferred is 4-(bis-(2-methoxyethyl)amino)-2,7-dimethyl-8-(2,5-dimethyl-4-methoxyphenyl)-[1,5-a]-pyrazolo-1,3,5-triazine and isomers thereof, stereoisomeric forms thereof, or mixtures of stereoisomeric forms thereof, and pharmaceutically acceptable salt or pro-drug forms thereof.
[27] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are independently H or C₁-C₁₀ alkyl, and each such C₁-C₁₀ alkyl is optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, R⁸CONR¹⁶R¹⁵, NR⁸CO₂R¹³, NR¹⁶R¹⁵, CONR¹⁶R¹⁵, aryl, heteroaryl or heterocyclyl.
[28] More preferred compounds of the above invention also include compounds and isomers thereof, stereoisomeric forms thereof, or mixtures of stereoisomeric forms thereof, and pharmaceutically acceptable salt or pro-drug forms thereof wherein
   - Ar is phenyl, pyridyl or 2,3-dihydrobenzofuranyl, and each Ar is optionally substituted with 1 to 4 R⁴ substituents,
   - R³ is NR^{6a}R^{7a} and
   - R¹ and R² are independently selected from H, C₁-C₄ alkyl, C₃-C₆ cycloalkyl, C₄-C₁₀ cycloalkylalkyl.
[29] More preferred compounds of the above invention also include compounds and isomers thereof, stereoisomeric forms thereof, or mixtures of stereoisomeric forms thereof, and pharmaceutically acceptable salt or pro-drug forms thereof wherein R^{6a} and R^{7a} are independently H or C₁-C₁₀ alkyl, and each such C₁-C₁₀ alkyl is optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, R⁸CONR¹⁶R¹⁵, NR⁸CO₂R¹³, NR¹⁶R¹⁵, CONR¹⁶R¹⁵, aryl, heteroaryl or heterocyclyl.

The present invention also provides pharmaceutical compositions comprising compounds of Formula (1) and a pharmaceutically acceptable carrier.

Many compounds of this invention have one or more asymmetric centers or planes. Unless otherwise indicated, all chiral (enantiomeric and diastereomeric) and racemic forms are included in the present invention. Many geometric isomers of olefins, C=N double bonds, and the like can also be present in the compounds, and all such stable isomers are contemplated in the present invention. The term "isomer" as used above refers to such geometric isomers. The compounds may be isolated in optically active or racemic forms. It is well known in the art how to prepare optically active forms, such as by resolution of racemic forms or by synthesis from optically active starting materials. All chiral, (enantiomeric and diastereomeric) and racemic forms and all geometric isomeric forms of a structure are intended, unless the specific stereochemistry or isomer form is specifically indicated.

The term "alkyl" includes both branched and straight-chain alkyl having the specified number of carbon atoms. Commonly used abbreviations have the following meanings: Me is methyl, Et is ethyl, Pr is propyl, Bu is butyl. The prefix "n" means a straight chain alkyl. The prefix "c" means a cycloalkyl. The prefix "(*S*)" means the S enantiomer and the prefix "(*R*)" means the R enantiomer. Alkenyl" includes hydrocarbon chains of either a straight or branched configuration and one or more unsaturated carbon-carbon bonds which may occur in any stable point along the chain, such as ethenyl, propenyl, and the like. "Alkynyl" includes hydrocarbon chains of either a straight or branched configuration and one or more triple carbon-carbon bonds which may occur in any stable point along the chain, such as ethynyl, propynyl and the like. "Haloalkyl" is intended to include both branched and straight-chain alkyl having the specified number of carbon atoms, substituted with 1 or more halogen; "alkoxy" represents an alkyl group of indicated number of carbon atoms attached through an oxygen bridge; "cycloalkyl" is intended to include saturated ring groups, including mono-,bi- or poly-cyclic ring systems, such as cyclopropyl, cyclobutyl, cyclopentyl, cyclohexyl, and so forth. "Halo" or "halogen" includes fluoro, chloro, bromo, and iodo.

The term "substituted", as used herein, means that one or more hydrogen on the designated atom is replaced with a selection from the indicated group, provided that the designated atom's normal valency is not exceeded, and that the substitution results in a stable compound. When a substitent is keto (i.e., =O), then 2 hydrogens on the atom are replaced.

Combinations of substituents and/or variables are permissible only if such combinations result in stable compounds. By "stable compound" or "stable structure" is meant a compound that is sufficiently robust to survive isolation to a useful degree of purity from a reaction mixture, and formulation into an efficacious therapeutic agent.

The term "appropriate amino acid protecting group" means any group known in the art of organic synthesis for the protection of amine or carboxylic acid groups. Such amine protecting groups include those listed in Greene and Wuts, "Protective Groups in Organic Synthesis" John Wiley & Sons, New York (1991) and "The Peptides: Analysis, Synthesis, Biology, Vol. 3, Academic Press, New York (1981), the disclosure of which is hereby incorporated by reference. Any amine protecting group known in the art can be used. Examples of amine protecting groups include, but are not limited to, the following: 1) acyl types such as formyl, trifluoroacetyl, phthalyl, and p-toluenesulfonyl; 2) aromatic carbamate types such as benzyloxycarbonyl (Cbz) and substituted benzyloxycarbonyls, 1-(p-biphenyl)-1-methylethoxycarbonyl, and 9-fluorenylmethyloxycarbonyl (Fmoc); 3) aliphatic carbamate types such as tert-butyloxycarbonyl (Boc), ethoxycarbonyl, diisopropylmethoxycarbonyl, and allyloxycarbonyl; 4) cyclic alkyl carbamate types such as cyclopentyloxycarbonyl and adamantyloxycarbonyl; 5) alkyl types such as triphenylmethyl and benzyl; 6) trialkylsilane such as trimethylsilane; and 7) thiol containing types such as phenylthiocarbonyl and dithiasuccinoyl.

The term "pharmaceutically acceptable salts" includes acid or base salts of the compounds of Formulae (1) and (2). Examples of pharmaceutically acceptable salts include, but are not limited to, mineral or organic acid salts of basic residues such as amines; alkali or organic salts of acidic residues such as carboxylic acids; and the like.

Pharmaceutically acceptable salts of the compounds of the invention can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

"Prodrugs" are considered to be any covalently bonded carriers which release the active parent drug of formula (I) or (II) *in vivo* when such prodrug is administered to a mammalian subject. Prodrugs of the compounds of formula (I) and (II) are prepared by modifying functional groups present in the compounds in such a way that the modifications are cleaved, either in routine manipulation or *in vivo,* to the parent compounds. Prodrugs include compounds wherein hydroxy, amine, or sulfhydryl groups are bonded to any group that, when administered to a mammalian subject, cleaves to form a free hydroxyl, amino, or sulfhydryl group, respectively. Examples of prodrugs include, but are not limited to, acetate, formate and benzoate derivatives of alcohol and amine functional groups in the compounds of formulas (I) and (II); and the like.

The term "therapeutically effective amount" of a compound of this invention means an amount effective to antagonize abnormal level of CRF or treat the symptoms of affective disorder, anxiety or depression in a host.

### Syntheses

Some compounds of Formula (1) may be prepared from intermediate compounds of Formula (7), using the procedures outlined in Scheme 1: 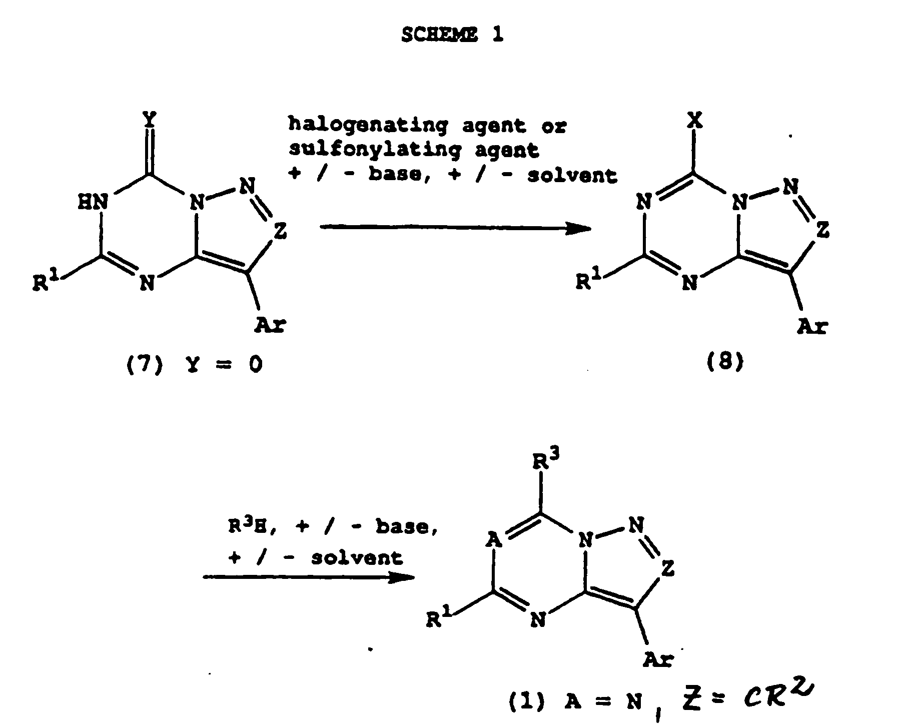 Compounds of Formula (7) (where Y is O) may be treated with a halogenating agent or sulfonylating agent in the presence or absence of a base in the presence or absence of an inert solvent at reaction temperatures ranging from -80°C to 250°C to give products of Formula (8) (where X is halogen, alkanesulfonyloxy, arylsulfonyloxy or haloalkane-sulfonyloxy). Halogenating agents include, but are not limited to, SOCl₂, POCl₃, PCl₃, PCl₅, POBr₃, PBr₃ or PBr₅. Sulfonylating agents include, but are not limited to, alkanesulfonyl halides or anhydrides (such as methanesulfonyl chloride or methanesulfonic acid anhydride), arylsulfonyl halides or anhydrides (such as p-toluenesulfonyl chloride or anhydride) or haloalkylsulfonyl halides or anhydrides (preferably trifluoromethanesulfonic anhydride). Bases may include, but are not limited to, alkali metal hydrides (preferably sodium hydride), alkali metal alkoxides (1 to 6 carbons) (preferably sodium methoxide or sodium ethoxide), alkaline earth metal hydrides, alkali metal dialkylamides (preferably lithium di-isopropylamide), alkali metal bis(trialkylsilyl)amides (preferably sodium bis(trimethylsilyl)amide), trialkyl amines (preferably N,N-di-isopropyl-N-ethyl amine or triethylamine) or aromatic amines (preferably pyridine). Inert solvents may include, but are not limited to, lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from -20°C to 100°C.

Compounds of Formula (8) may be reacted with compounds of Formula R³H (where R³ is defined as above except R³ is not SH, COR⁷, CO₂R⁷, aryl or heteroaryl) in the presence or absence of a base in the presence or absence of an inert solvent at reaction temperatures ranging from -80 to 250°C to generate compounds of Formula (1). Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal carbonates, alkali metal bicarbonates, alkali metal bis(trialkylsilyl)amides (preferably sodium bis(trimethylsilyl)amide), trialkyl amines (preferably N,N-di-isopropyl-N-ethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from 0°C to 140°C.

Scheme 2 delineates the procedures for converting intermediate compounds of Formula (7) (where Y is-S) to some compounds of Formula (1). 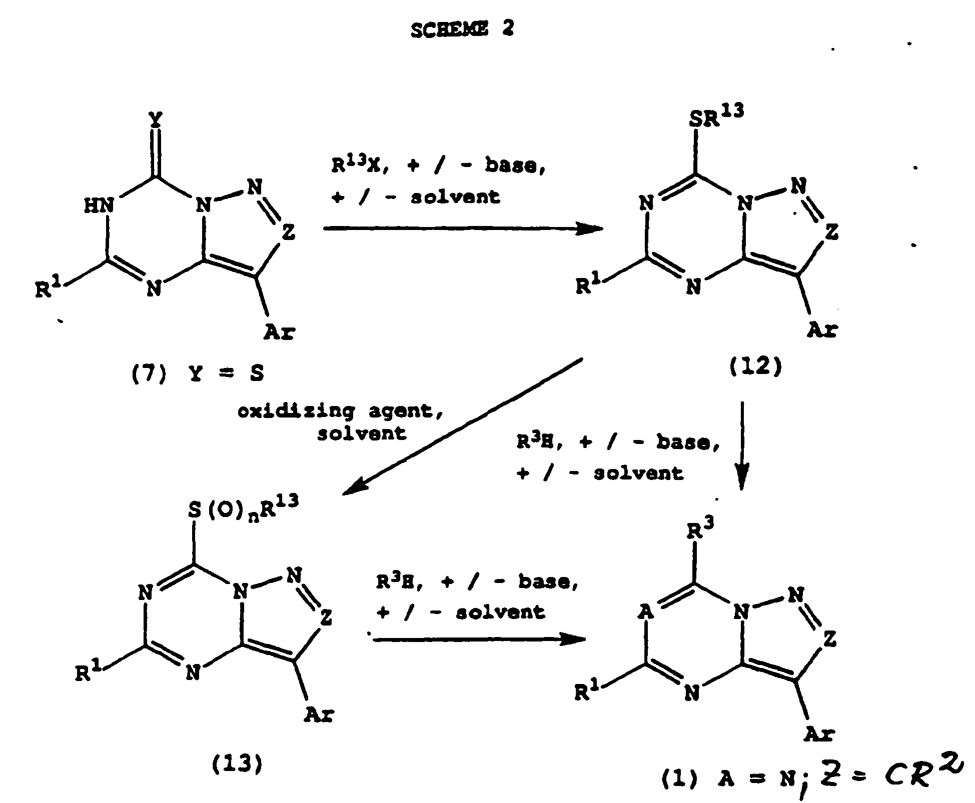 Compounds of Formula (7) (where Y is S) may be treated with an alkylating agent R¹³X (where R¹³ is defined as above, except R¹³ is not aryl or heteroaryl) in the presence or absence of a base in the presence or absence of an inert solvent at reaction temperatures ranging from -80°C to 250°C. Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal carbonates, alkali metal hydroxides, alkali metal bis(trialkylsilyl)amides (preferably sodium bis(trimethylsilyl)amide), trialkyl amines (prefereably N,N-di-isopropyl-N-ethyl amine or triethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from -80°C to 100°C.

Compounds of Formula (12) (Formula (1) where R³ is SR¹³) may then be reacted with compounds of Formula R³H to give compounds of Formula (1), using the same conditions and reagents as were used for the conversion of compounds of Formula (8) to compounds of Formula (1) as outlined for Scheme 1 above. Alternatively, compounds of Formula (12) (Formula (1) where R³ is SR¹³) may be oxidized to compounds of Formula (13) (Formula (1) where R³ is S(O)ₙR¹³, n is 1,2) by treatment with an oxidizing agent in the presence of an inert solvent at temperatures ranging from -80°C to 250°C. Oxidizing agents include, but are not limited to, hydrogen peroxide, alkane or aryl peracids (preferably peracetic acid or m-chloro-perbenzoic acid), dioxirane, oxone, or sodium periodate. Inert solvents may include, but are not limited to, alkanones (3 to 10 carbons, preferably acetone), water, alkyl alcohols (1 to 6 carbons), aromatic hydrocarbons (preferably benzene or toluene) or haloalkanes of 1 to 10 carbons and 1 to 10 halogens (preferably dichloromethane) or combinations thereof. The choices of oxidant and solvent are known to those skilled in the art (cf. Uemura, S., Oxidation of Sulfur, Selenium and Tellurium, in Comprehensive Organic Synthesis, Trost, B.M. ed., (Elmsford, NY: Pergamon Press, 1991), 7, 762-769). Preferred reaction temperatures range from -20°C to 100°C. Compounds of Formula (13) (Formula (1) where R³ is S(O)ₙR¹³, n is 1,2) may then be reacted with compounds of Formula R³H to give compounds of Formula (1), using the same conditions and reagents as were used for the conversion of compounds of Formula (8) co compounds of Formula (1) as outlined for Scheme (1) above.

Compounds of Formula (1) may be prepared from compounds of Formula (7), where Y is NH, by the procedures depicted in Scheme 3. 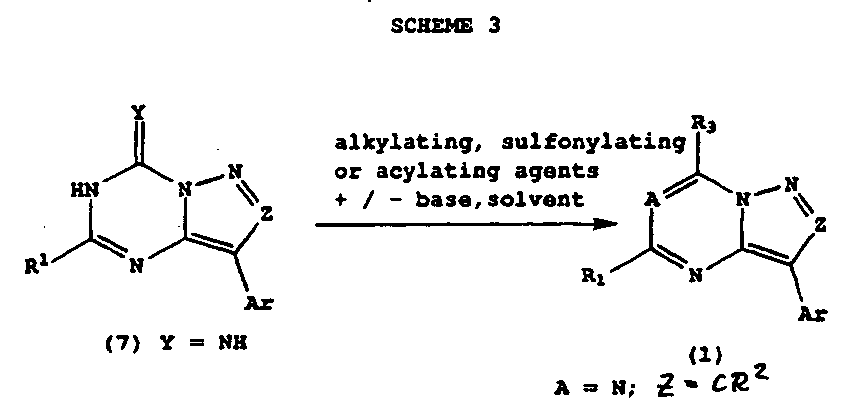 Reaction of compounds of Formula (7), where Y is NH, with alkylating agents, sulfonylating agents or acylating agents or sequential reactions with combinations thereof, in the presence or absence of a base in an inert solvent at reaction temperatures ranging from -80°C to 250°C may afford compounds of Formula (1). Alkylating agents may include, but are not limited to, C₁-C₁₀ alkyl -halides, -tosylates, -mesylates or -triflates; C₁-C₁₀ haloalkyl(1- 10 halogens)-halides, -tosylates, -mesylates or -triflates; C₂-C₈ alkoxyalkyl-halides, -tosylates, -mesylates or -triflates; C₃-C₆ cycloalkyl-halides, -tosylates, -mesylates or -triflates; C₄-C₁₂ cycloalkylalkyl-halides, -tosylates, -mesylates or -triflates; aryl(C₁-C₄ alkyl)-halides, -tosylates, -mesylates or -triflates; heteroaryl(C₁-C₄ alkyl)-halides, -tosylates, -mesylates or -triflates; or heterocyclyl(C₁-C₄ alkyl)-halides, -tosylates, -mesylates or -triflates. Acylating agents may include, but are not limited to, C₁-C₁₀ alkanoyl halides or anhydrides, C₁-C₁₀ haloalkanoyl halides or anhydrides with 1-10 halogens, C₂-C₈ alkoxyalkanoyl halides or anhydrides, C₃-C₆ cycloalkanoyl halides or anhydrides, C₄-C₁₂ cycloalkylalkanoyl halides or anhydrides, aroyl halides or anhydrides, aryl(C₁-C₄) alkanoyl halides or anhydrides, heteroaroyl halides or anhydrides, heteroaryl(C₁-C₄) alkanoyl halides or anhydrides, heterocyclylcarboxylic acid halides or anhydrides or heterocyclyl(C₁-C₄) alkanoyl halides or anhydrides. Sulfonylating agents include, but are not limited to, C₁-C₁₀ alkylsulfonyl halides or anhydrides, C₁-C₁₀ haloalkylsulfonyl halides or anhydrides with 1 - 10 halogens, C₂-C₈ alkoxyalkylsulfonyl halides or anhydrides, C₃-C₆ cycloalkylsulfonyl halides or anhydrides, C₄-C₁₂ cycloalkylalkylsulfonyl halides or anhydrides, arylsulfonyl halides or anhydrides, aryl(C₁-C₄ alkyl)-, heteroarylsulfonyl halides or anhydrides, heteroaryl(C₁-C₄ alkyl)sulfonyl halides or anhydrides, heterocyclylsulfonyl halides or anhydrides or heterocyclyl(C₁-C₄ alkyl)sulfonyl halides or anhydrides. Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal carbonates, alkali metal bis(trialkylsilyl)amides (preferably sodium bis(trimethylsilyl)amide), trialkyl amines (prefereably di-isopropylethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred reaction temperatures range from 0°C to 100°C.

Scheme 4 delineates procedures, which may be employed to prepare intermediate compounds of Formula (7), where Y is O, S and Z is CR². 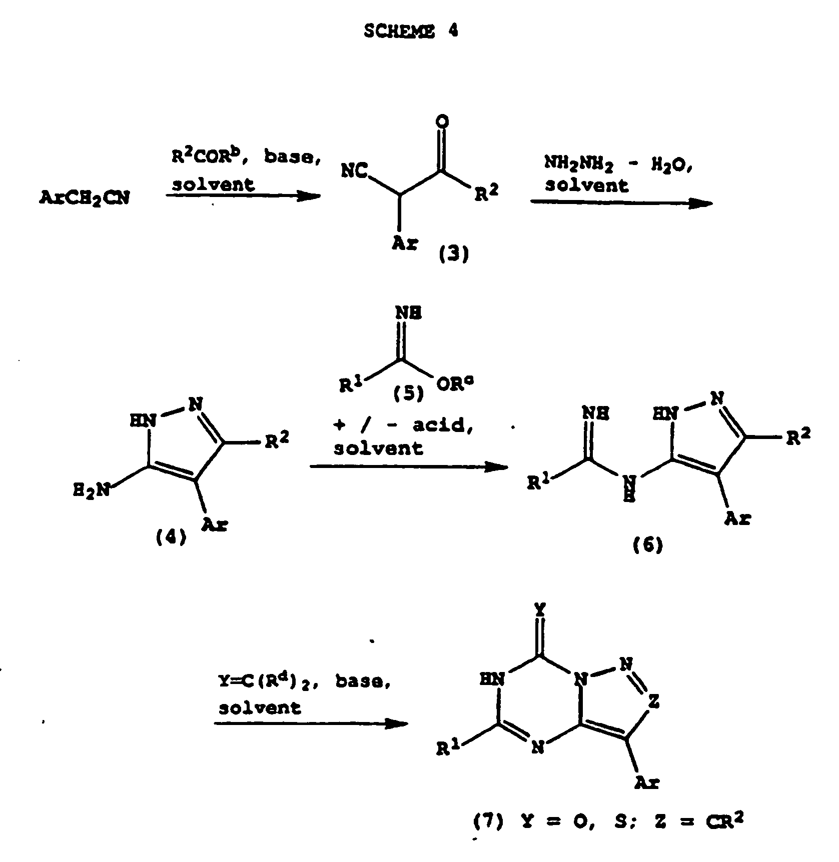 Compounds of the formula ArCH₂CN are reacted with compounds of the formula R²COR^{b}, where R² is defined above and R^{b} is halogen, cyano, lower alkoxy (1 to 6 carbons) or lower alkanoyloxy (1 to 6 carbons), in the presence of a base in an inert solvent at reaction temperatures ranging from -78°C to 200°C to afford compounds of Formula (3). Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal carbonates, alkali metal hydroxides, alkali metal bis(trialkylsilyl)amides (preferably sodium bis(trimethylsilyl)amide), trialkyl amines (preferably N,N-di-isopropyl-N-ethyl amine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), water, dialkyl ethers (preferably diethyl ether), cyclic ethers (preferably tetrahydrofuran or 1,4-dioxane), N;N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred reaction temperatures range from 0°C to 100°C.

Compounds of Formula (3) may be treated with hydrazine-hydrate in the presence of an inert solvent at temperatures ranging from 0°C to 200°C, preferably 70°C to 150°C, to produce compounds of Formula (4). Inert solvents may include, but are not limited to, water, alkyl alcohols (1 to 8 carbons, preferably methanol or ethanol), lower alkanenitriles (1 to 6 carbons, preferably acetonitrile), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Compounds of Formula (4) may be reacted with compounds of Formula (5) (where R^{c} is alkyl (1-6 carbons)) in the presence or absence of an acid in the presence of an inert solvent at temperatures ranging from 0°C to 200°C to produce compounds of Formula (6). Acids may include, but are not limited to alkanoic acids of 2 to 10 carbons (preferably acetic acid), haloalkanoic acids (2 - 10 carbons, 1-10 halogens, such as trifluoroacetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Inert solvents may include, but are not limited to, water, alkanenitriles (1 to 6 carbons, preferably acetonitrile), halocarbons of 1 to 6 carbons and 1 to 6 halogens (preferably dichloromethane or chloroform), alkyl alcohols of 1 to 10 carbons (preferably ethanol), dialkyl ethers (4 to 12 carbons, preferably diethyl ether or di-isopropylether) or cyclic ethers such as dioxan or tetrahydrofuran. Preferred temperatures range from ambient temprature to 100°C.

Compounds of Formula (6) may be converted to intermediate compounds of Formula (7) by treatment with compounds C=Y(R^{d})₂ (where Y is O or S and R^{d} is halogen (preferably chlorine), alkoxy (1 to 4 carbons) or alkylthio (1 to 4 carbons)) in the presence or absence of a base in an inert solvent at reaction temperatures from -50°C to 200°C. Bases may include, but are not limited to, alkali metal hydrides (preferably sodium hydride), alkali metal alkoxides (1 to 6 carbons) (preferably sodium methoxide or sodium ethoxide), alkali metal carbonates, alkali metal hydroxides, trialkyl amines (preferably N,N-diisopropyl-N-ethyl amine or triethylamine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred temperatures are 0°C to 150°C.

Intermediate compounds of Formula (7), where Z is N, may be synthesized according the methods outlined in Schema 5 (for reference purposes): 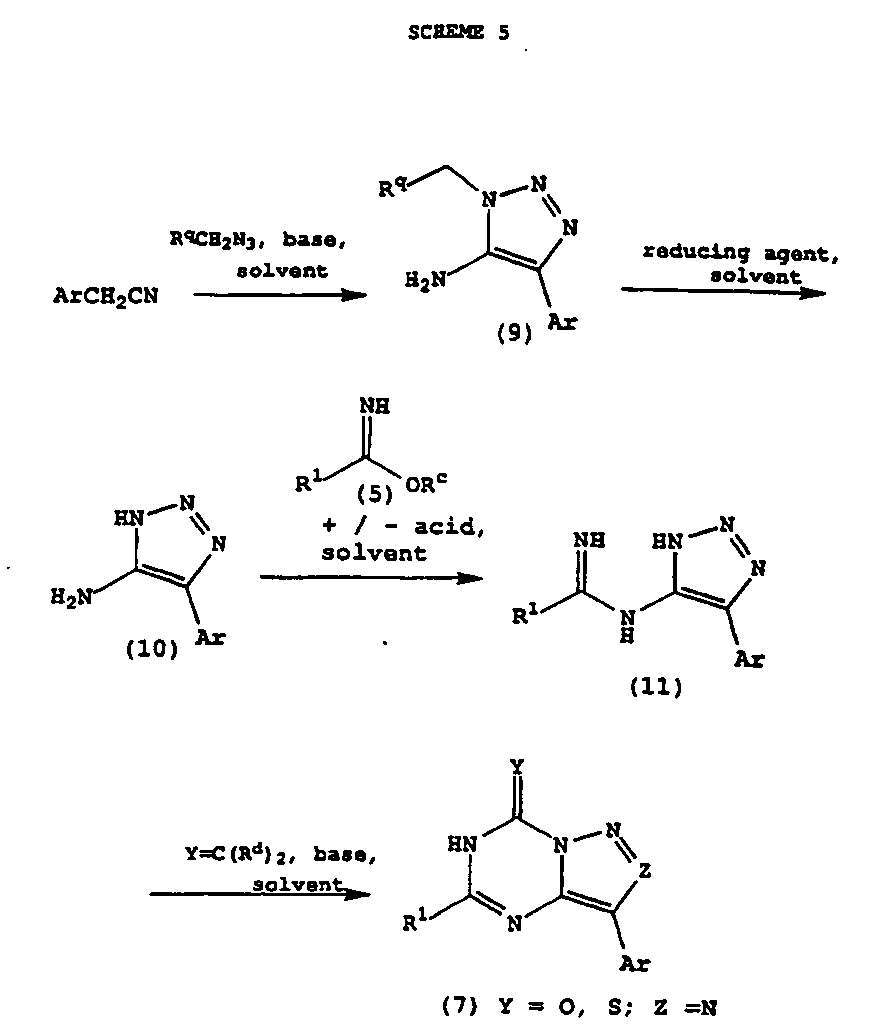 Compounds of ArCH₂CN are reacted with compounds of Formula R^{q}CH₂N₃ (where R^{q} is a phenyl group optionally substituted by H, alkyl (1 to 6 carbons) or alkoxy (1 to 6 carbons) in the presence or absence of a base in an inert solvent at temperatures ranging from 0°C to 200°C to generate compounds of Formula (9). Bases may include, but are not limited to, alkali metal hydrides (preferably sodium hydride), alkali metal alkoxides (1 to 6 carbons)(preferably sodium methoxide, sodium ethoxide or potassium t-butoxide), alkaline earth metal hydrides, alkali metal dialkylamides (preferably lithium di-isopropylamide), alkali metal carbonates, alkali metal hydroxides, alkali metal bis(trialkylsilyl)amides (preferably sodium bis(trimethylsilyl)amide), trialkyl amines (preferably N,N-di-isopropyl-N-ethyl amine or triethylamine) or aromatic amines (preferably pyridine). Inert solvents may include, but are not limited to, alkyl alcohols (1 to 8 carbons, preferably methanol or ethanol), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). Preferred reaction temperatures range from ambient temperature to 100°C.

Compounds of Formula (9) may be treated with a reducing agent in an inert solvent at -100°C to 100°C to afford products of Formula (10). Reducing agents include, but are not limited to, (a) hydrogen gas in combination with noble metal catalysts such as Pd-on-carbon, PtO₂, Pt-on-carbon, Rh-on-alumina or Raney nickel, (b) alkali metals (preferably sodium) in combination with liquid ammonia or (c) ceric ammonium nitrate. Inert solvents may include, but are not limited to, alkyl alcohols (1 to 8 carbons, preferably methanol or ethanol), lower alkanenitriles (1 to 6 carbons, preferably acetonitrile), water, dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide) or aromatic hydrocarbons (preferably benzene or toluene). The preferred reaction temperatures are -50°C to 60°C. Compounds of Formula (9) are then converted to compounds of Formula (7) (where Z is N) via intermediates of Formula (11) using the reagents and reaction conditions outlined in Scheme 4 for the conversion of compounds of Formula (4) to compounds of Formula (7) (where Z is CR²).

Compounds of Formula (1) may also be prepared from compounds of Formula (7) (where Y is O, S and Z is defined above) as outlined in Scheme 6: 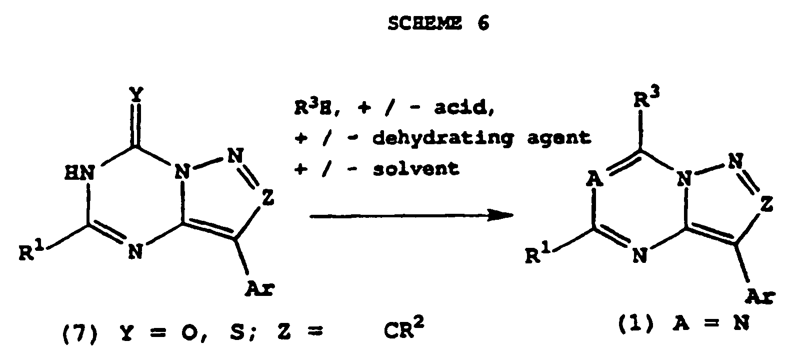 Compounds of Formula (7) may be reacted with compounds of Formula R³H in the presence of a dehydrating agent in an inert solvent at reaction temperatures ranging from 0°C to 250°C. Dehydrating agents include, but are not limited to, P₂O₅, molecular sieves or inorganic or organic acids. Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Inert solvents may include, but are not limited to, alkyl alcohols (1 to 8 carbons, preferably methanol or ethanol), lower alkanenitriles (1 to 6 carbons, preferably acetonitrile), dialkyl ethers (preferably glyme or diglyme), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or halocarbons of 1 to 10 carbons and 1 to 10 halogens (preferably chloroform) Preferred reaction temperatures range from ambient temperature to 150°C.

Some compounds of Formula (1) (where A is N) may also be prepared by the methods shown in Scheme 7: 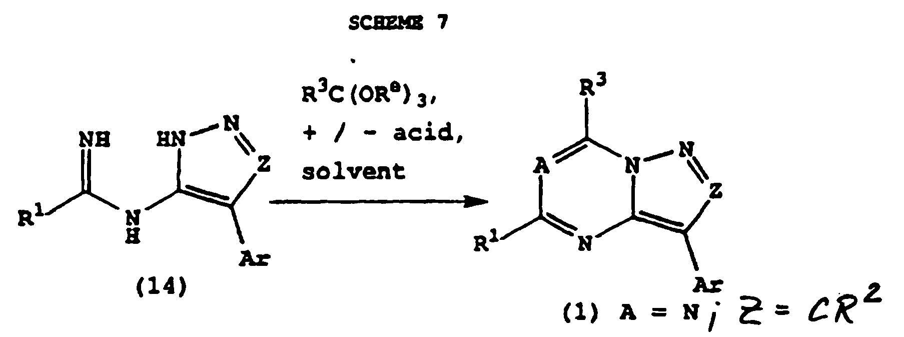 Intermediate compounds of Formula (14), where Z is defined above, may be reacted with compounds of Formula R³C(OR^{e})3, where R^{e} may be alkyl (1 to 6 carbons) in the presence or absence of an acid in an inert solvent at temperatures ranging from 0°C to 250°C. Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Inert solvents may include, but are not limited to, lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from 50°C to 150°C.

Intermediate compounds of Formula (7) may also be synthesized by the reactions displayed in Scheme 8. 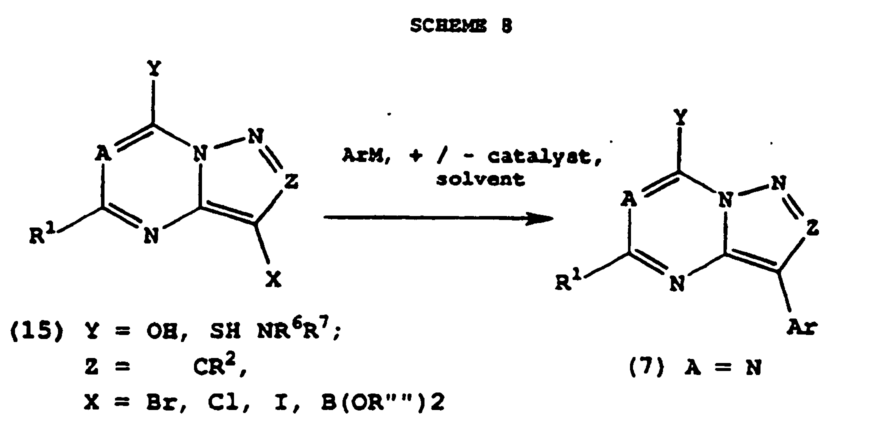 Compounds of Formula (15), (where Y is OH, SH, NR⁶R⁷; Z is defined above, X is Br, Cl, I, O₃SCF₃ or B(OR"")₂ and R"" is H or alkyl (1 to 6 carbons)) may be reacted with a compound of Formula ArM (where M is halogen, alkali metal, ZnCl, ZnBr, ZnI, MgBr, MgCl, MgI, CeCl₂, CeBr₂ or copper halides) in the presence or absence of an organometallic catalyst in the presence or absence of a base in an inert solvents at temperatures ranging from -100°C to 200°C. Those skilled in the art will recognize that the reagents ArM may be generated in situ. Organometallic catalysts include, but are not limited to, palladium phosphine complexes (such as Pd(PPh₃)₄), palladium halides or alkanoates (such as PdCl₂(PPh₃)₂ or Pd(OAc)₂) or nickel complexes (such as NiCl₂(PPh₃)₂). Bases may include, but are not limited to, alkali metal carbonates or trialkyl amines (preferably N,N-di-isopropyl-N-ethyl amine or triethylamine). Inert solvents may include, but are not limited to, dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or water. Preferred reaction temperatures range from -80°C to 100°C. The choices of M and X are known to those skilled in the art (cf. Imamoto, T., Organocerium Reagents in Comprehensive Organic Synthesis, Trost, B.M. ed., (Elmsford, NY: Pergamon Press, 1991), 1, 231-250; Knochel, P., Organozinc, Organocadmium and Organomercury Reagents in Comprehensive Organic Synthesis, Trost, B.M. ed., (Elmsford, NY: Pergamon Press, 1991), 1, 211-230; Knight, D.W., Coupling Reactions between sp2 Carbon Centers, in Comprehensive Organic Synthesis, Trost, B.M. ed., (Elmsford, NY: Pergamon Press, 1991), 3, 481-520).

Compounds of Formula (1) may also be prepared using the methods shown in Scheme 9. 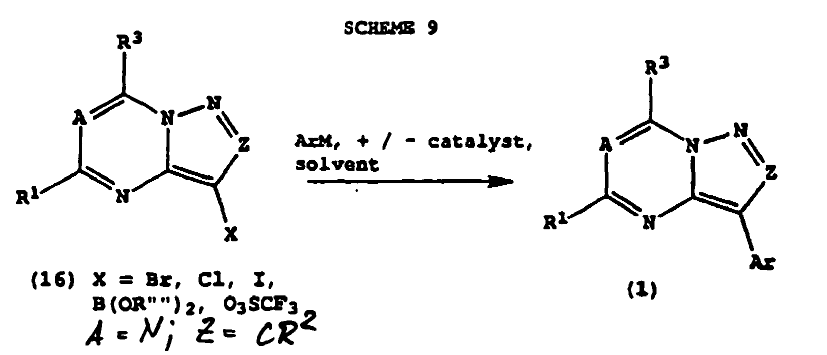 Compounds of Formula (16), where R¹ and R³ are defined above and X is Br, Cl, I, O₃SCF₃ or B(OR"")₂ and R"" is H or alkyl (1 to 6 carbons)) may be reacted with a compound of Formula ArM (where M is halogen, alkali metal, ZnCl, ZnBr, ZnI, MgBr, MgCl, MgI, CeCl₂, CeBr₂ or copper halides) in the presence or absence of an organometallic catalyst in the presence or absence of a base in an inert solvents at temperatures ranging from -100°C to 200°C. Those skilled in the art will recognize that the reagents ArM may be generated in situ (see the above references in Comprehensive Organic Synthesis). Organometallic catalysts include, but are not limited to, palladium phosphine complexes (such as Pd(PPh₃)₄), palladium halides or alkanoates (such as PdCl₂(PPh₃)₂ or Pd(OAc)₂) or nickel complexes (such as NiCl₂(PPh₃)₂). Bases may include, but are not limited to, alkali metal carbonates or trialkyl amines (preferably N,N-di-isopropyl-N-ethyl amine or triethylamine). Inert solvents may include, but are not limited to, dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or water. Preferred reaction temperatures range from -80°C to 100°C.

Intermediate compounds of Formula (7)(where Y is O, S, NH, Z is CR² and R¹, R² and Ar are defined as above) may be prepared as illustrated in Scheme 10. 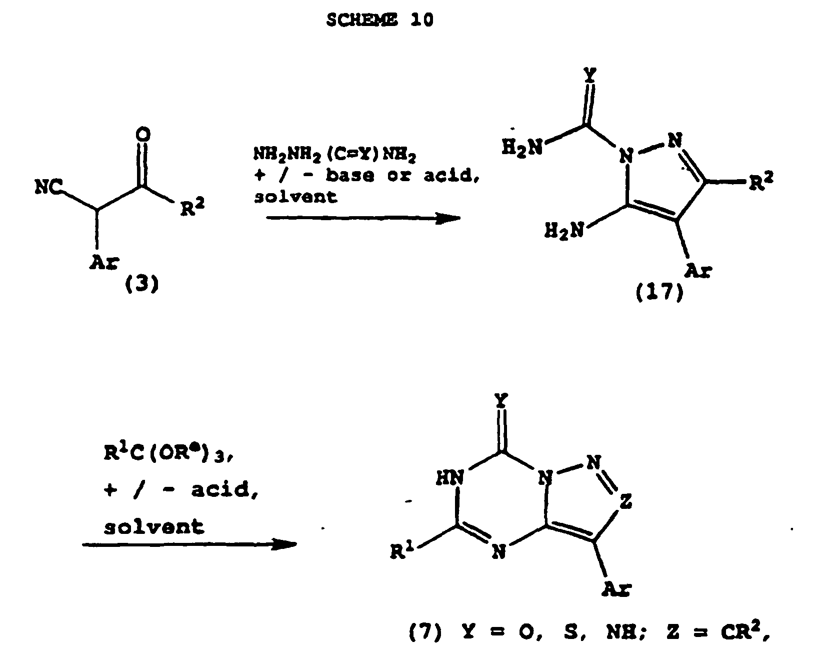 Compounds of Formula (3) may be reacted with compounds of Formula H₂NNH(C=Y)NH₂, where Y is O, S or NH, in the presence or absence of a base or acid in an inert solvent at temperatures from 0°C to 250°C to produce compounds of Formula (17). Acids may include, but are not limited to alkanoic acids of 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Bases may include, but are not limited to, alkali metal hydrides (preferably sodium hydride), alkali metal alkoxides (1 to 6 carbons)(preferably sodium methoxide or sodium ethoxide), alkaline earth metal hydrides, alkali metal dialkylamides (preferably lithium di-isopropylamide), alkali metal bis(trialkylsilyl)amides (preferably sodium bis(trimethylsilyl)amide), trialkyl amines (preferably N,N-di-isopropyl-N-ethyl amine or triethylamine) or aromatic amines (preferably pyridine). Inert solvents may include, but are not limited to, alkyl alcohols (1 to 6 carbons), lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane).

Preferred reaction temperatures range from 0°C to 150°C. Compounds of Formula (17) may then be reacted with compounds of Formula R³C(OR^{e})3, where R^{e} may be alkyl (1 to 6 carbons) in the presence or absence of an acid in an inert solvent at temperatures ranging from 0°C to 250°C. Acids may include, but are not limited to alkanoic acids of. 2 to 10 carbons (preferably acetic acid), arylsulfonic acids (preferably p-toluenesulfonic acid or benzenesulfonic acid), alkanesulfonic acids of 1 to 10 carbons (preferably methanesulfonic acid), hydrochloric acid, sulfuric acid or phosphoric acid. Stoichiometric or catalytic amounts of such acids may be used. Inert solvents may include, but are not limited to, lower alkanenitriles (1 to 6 carbons, preferably acetonitrile), dialkyl ethers (preferably diethyl ether), cyclic ethers (preferably tetrahydrofuran or 1,4-dioxane), N,N-dialkylformamides (preferably dimethylformamide), N,N-dialkylacetamides (preferably dimethylacetamide), cyclic amides (preferably N-methylpyrrolidin-2-one), dialkylsulfoxides (preferably dimethylsulfoxide), aromatic hydrocarbons (preferably benzene or toluene) or haloalkanes of 1 to 10 carbons and 1 to 10 halogens (preferably dichloromethane). Preferred reaction temperatures range from 50°C to 150°C.

Some compounds of Formula (1) may also be prepared using the methods shown in Scheme 15. 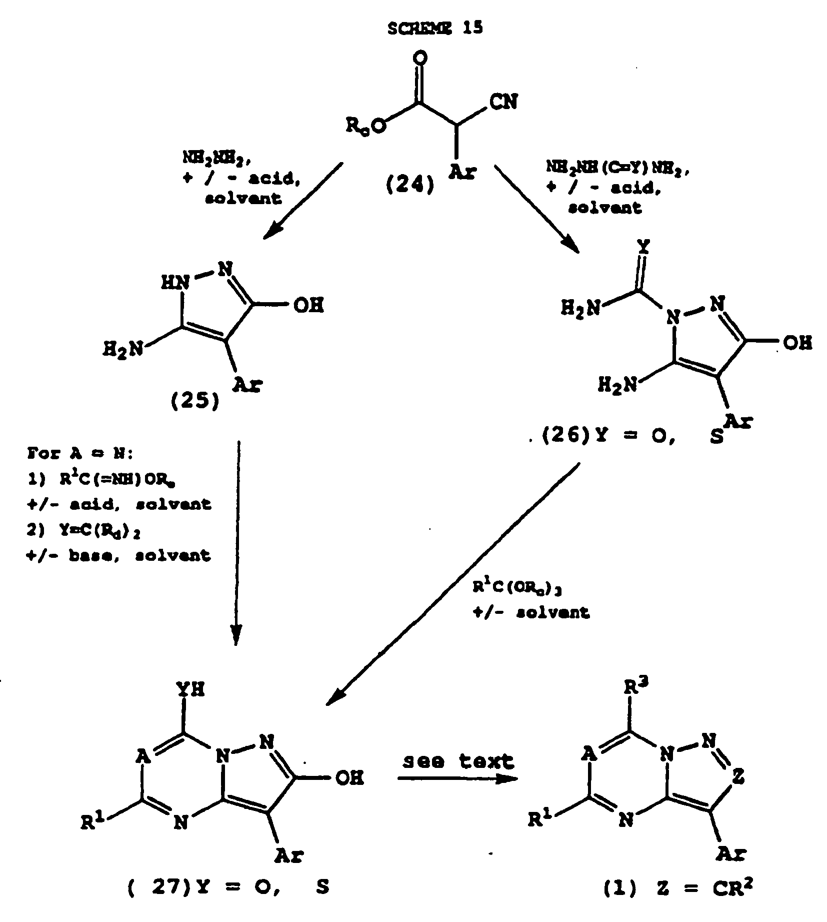 A compound of Formula (24) (R_{c} is a lower alkyl group and Ar is defined as above) may be reacted with hydrazine in the presence or absence of an inert solvent to afford an intermediate of Formula (25), where Ar is defined as above. The conditions employed are similar to those used for the preparation of intermediate of Formula (4) from compound of Formula (3) in Scheme 4. Compounds of Formula (25), where A is N, may be reacted with reagents of the formula R¹C(=NH)ORₑ, where R¹ is defined above and Rₑ is a lower alkyl group) in the presence or absence of an acid in an inert solvent, followed by reaction with a compound of formula YisC(R_{d})2 (where Y is O or S and R^{d} is halogen (preferably chlorine), alkoxy (1 to 4 carbons) or alkylthio (1 to 4 carbons)) in the presence or absence of a base in an inert solvent to give compounds of Formula (27) (where A is N and Y is 0, S). The conditions for these transformations are the same as those employed for the conversions of compound of Formula (4) to compound of Formula (7) in Scheme 4.

It will be recognized by those skilled in the art that various combinations of halogenating agents, sulfonylating agents, R³H or R²H may be used in different orders of reaction sequences in Scheme 15 to afford compounds of Formula (1). For example, in some cases, it may be desirable to react compounds with stoichiometric amounts of halogenating agents or sulfonylating agents, react with R²H (or R³H), then repeat the reaction with halogenating agents or sulfonylating agents and react with R³H (or R²H) to give compounds of Formula (1). The reaction conditions and reagents used for these conversions are similar to the ones employed for the conversion of intermediate compounds of Formulae (22) to (23) to (1) in Scheme 14 (for A is CR) or the conversion of intermediate compounds of Formulae (7) to (8) to (1) in Scheme 1 (where A is N).

Alternatively, compounds of Formula (27) (where Y is S) may be converted to compounds of Formula (1) in Scheme 15. Intermediate compounds of Formula (27) may be alkylated with a compound R^{f}X (where R^{f} is lower alkyl and X is halogen, alkanesulfonyloxy or haloalkanesulfonyloxy) in an inert solvent, (then optionally oxidized with an oxidizing agent in an inert solvent) and then reacted with R³H in the presence or absence of a base in an inert solvent to give a compound of Formula (1). The conditions and reagents employed are similar to those used in the conversion of intermediate compounds of Formulae (7) to (12) (or to (13)) to compounds of Formula (1) in Scheme 2.

Compounds of Formula (1) may be prepared from compounds of Formula (24), using an alternate route as depicted in Scheme 15. Compounds of Formula (24) may be converted to compounds of Formula (27) via reaction with compounds of formula NH₂NH(C=NH)NH₂ in the presence or absence of an acid in an inert solvent, followed by reaction with compounds R¹C(OR_{c})₃ (where R_{c} is lower alkyl and R¹ is defined as above), using the conditions employed for the conversion of compounds of Formulae (3) to (17) to (7) in Scheme 10.

### EXAMPLES

Analytical data were recorded for the compounds described below using the following general procedures. Proton NMR spectra were recorded on an IBM-Bruker FT-NMR (300 MHz); chemical shifts were recorded in ppm (δ) from an internal tetramethysilane standard in deuterochloroform or deuterodimethylsulfoxide as specified below. Mass spectra (MS) or high resolution mass spectra (HRMS) were recorded on a Finnegan MAT 8230 spectrometer (using chemi-ionization (CI) with NH₃ as the carrier gas or gas chromatography (GC) as specified below) or a Hewlett Packard 5988A model spectrometer. Melting points were recorded on a Buchi Model 510 melting point apparatus and are uncorrected. Boiling points are uncorrected. All pH determinations during workup were made with indicator paper.

Reagents were purchased from commercial sources and, where necessary, purified prior to use according to the general procedures outlined by D. Perrin and W.L.F. Armarego, Purification of Laboratory Chemicals, 3rd ed., (New York: Pergamon Press, 1988). Chromatography was performed on silica gel using the solvent systems indicated below. For mixed solvent systems, the volume ratios are given. Otherwise, parts and percentages are by weight.

The following examples are provided to describe the invention in further detail. These examples, which set forth the best mode presently contemplated for carrying out the invention, are intended to illustrate and not to limit the invention.

### EXAMPLE 1

### Preparation of 2,7-dimethyl-8-(2,9-dimethylphenyl)[1,5-a] -pyrazolo-[1,3,5]-triazin-4(3H)-one

### (Formula 7, where Y is O, R₁ is CH₃, Z is C-CH₃, Ar is 2,4-dimethylphenyl)

### A. 1-Cyano-1-(2,4-dimethylphenyl)propan-2-one

Sodium pellets (9.8g, 0.43 mol) were added portionwise to a solution of 2,4-dimethylphenylacetonitrile (48 g, 0.33 mol) in ethyl acetate (150 mL) at ambient temperature. The reaction mixture was heated to reflux temperature and stirred for 16 hours. The resulting suspension was cooled to room temperature and filtered. The collected precipitate was washed with copious amounts of ether and then air-dried. The solid was dissolved in water and a 1N HCl solution was added until the pH = 5-6. The mixture was extracted with ethyl acetate (3 X 200 mL); the combined organic layers were dried over MgSO₄ and filtered. Solvent was removed *in vacuo* to afford a white solid (45.7g, 74% yield): NMR (CDCl₃, 300 MHz):: CI-MS: 188 (M + H).

### B. 5-Amino-4-(2,4-dimethylphenyl)-3-methylpyrazole

A mixture of 1-cyano-1-(2,4-dimethylphenyl)propan-2-one (43.8g, 0.23 mol), hydrazine-hydrate (22 mL, 0.46 mol), glacial acetic acid (45 mL, 0.78 mol) and toluene (500 mL) were stirred at reflux temperature for 18 hours in an apparatus fitted with a Dean-Stark trap. The reaction mixture was cooled to ambient temperature and solvent was removed in vacuo. The residue was dissolved in 6N HCl and the resulting solution was extracted with ether three times. A concentrated ammonium hydroxide solution was added to the aqueous layer until pH = 11. The resulting semi-solution was extracted three times with ethyl acetate. The combined organic layers were dried over MgSO₄ and filtered. Solvent was removed *in vacuo* to give a pale brown viscous oil (34.6g, 75% yield): NMR (CDCl₃, 300 MHz): 7.10 (s, 1H), 7.05 (d, 2H, J=1), 2.37 (s, 3H), 2.10 (s, 3H); CI-MS: 202 (M + H).

### C. 5-Acetamidino-4-(2,4-dimethylphenyl)-3-methylpyrazole, acetic acid salt

Ethyl acetamidate hydrochloride (60g, 0.48 mol) was added quickly to a rapidly stirred mixture of potassium carbonate (69.5g, 0.50 mol), dichloromethane (120 mL) and water (350 mL). The layers were separated and the aqueous layer was extracted with dichloromethane (2 X 120 mL). The combined organic layers were dried over MgSO₄ and filtered. Solvent was removed by simple distillation and the pot residue, a clear pale yellow liquid, (35.0 g) was used without further purification.

Glacial aetic acid (9.7 mL, 0.17 mol) was added to a stirred mixture of 5-amino-4-(2,4-dimethylphenyl)-3-methylpyrazole (34g, 0.17 mol), ethyl acetamidate (22g, 0.25 mol) and acetonitrile (500 mL). The resulting reaction mixture was stirred at room temperature for 3 days; at the end of which time, it was concentrated in vacuo to about one-third of its original volume. The resulting suspension was filtered and the collected solid was washed with copious amounts of ether. The white solid was dried in vacuo (31.4g, 61% yield): NMR (DMSO-d₆,300 MHz): 7.00 (s, 1H), 6.90 (dd, 2H, J=7, 1), 2.28 (s, 3H), 2.08 (s, 3H), 2.00 (s, 3H), 1.90 (s, 3H), 1.81 (s, 3H); CI-MS: 243 (M + H).

### D. 2,7-dimethyl-8-(2,4 -dimethylphenyl) [1,5-a]-pyrazolo-[1,3,5]-triazin-4(3H)-one

Sodium pellets (23g, 1 mol) were added portionwise to ethanol (500 mL) with vigorous stirring. After all the sodium reacted, 5-acetamidino-4-(2,4-dimethylphenyl)-3-methylpyrazole, acetic acid salt (31.2g, 0.1 mol) and diethyl carbonate (97 mL, 0.8 mol) were added. The resulting reaction mixture was heated to reflux temperature and stirred for 18 hours. The mix was cooled to room temperature and solvent was removed in vacuo. The residue was dissolved in water and a 1N HCl solution was added slowly until pH = 5-6. The aqueous layer was extracted with ethyl acetate three times; the combined organic layers were dried over MgSO₄ and filtered. Solvent was removed *in vacuo* to give a pale tan solid (26g, 98% yield): NMR (CDCl₃,300 MHz): 7.15(s, 1H), 7.09 (s, 2H), 2.45 (s, 3H), 2.39 (s, 3H), 2.30 (s, 3H); CI-MS: 269 (M + H).

### EXAMPLE 2 (reference example)

### Preparation of 5-methyl-3-(2,4,6-trimethylphenyl)[1,5-a]-[1,2,3]-triazolo-[1,3,5]-triazin-7(6H)-one

### (Formula 7, where Y is O, R₁ is CH₃, Z is N, Ar is 2,4,6-trimethylphenyl)

### A. 1-Phenylmethyl-4-(2,4,6-trimethylphenyl)-5-aminotriazole

A mixture of 2,4,6-trimethylbenzyl cyanide (1.0g, 6.3 mmol), benzyl azide (0.92g, 6.9 mmol) and potassium t-butoxide (0.78g, 6.9 mmol) in tetrahydrofuran (10mL) was stirred at ambient temperature for 2.5 days. The resulting suspension was diluted with water and extracted three times with ethyl acetate. The combined organic layers were dried over MgSO₄ and filtered. Solvent was removed in vacuo to give a brown oil. Trituration with ether and filtration afforded a yellow solid (1.12g, 61% yield): NMR (CDCl₃, 300 MHz):7.60-7.30 (m, 5H), 7.30-7.20 (m, 2H), 5.50 (s, 2H), 3.18 (br s, 2H), 2.30 (s, 3H), 2.10 (s, 6H); CI-MS: 293 (M + H).

### B. 4- (2, 4, 6-Trimethylphenyl) -5-aminotriazole

Sodium (500 mg, 22 mmol) was added with stirring to a mixture of liquid ammonia (30 mL) and 1-phenylmethyl-4-(2,4,6-trimethylphenyl)-5-aminotriazole (1.1g, 3.8 mmol). The reaction mixture was stirred until a dark green color persisted. An ammonium chloride solution (mL) was added and the mixture was stirred while warming to ambient temperature over 16 hours. The residue was treated with a 1M HCl solution and filtered. The aqueous layer was basified with a concentrated ammonium hydroxide solution (pH = 9) and then extracted with ethyl acetate three times. The combined organic layers were dried over MgSO₄ and filtered. Solvent was removed in vacuo to give a yellow solid (520 mg), which was homogeneous by thin layer chromatography (ethyl acetate):
NMR (CDCl₃, 300 MHz): 6.97 (s, 2H), 3.68-3.50 (br.s, 2H), 2.32 (s, 3H), 2.10 (s, 6H); CI-MS: 203 (M + H).

### C. 4-(2,4,6-Trimethylphenyl)-5-acetamidinotriazole, acetic acid salt

A mixture of 4-(2,4,6-trimethylphenyl)-5-aminotriazole (400 mg, 1.98 mmol), ethyl acetamidate (261 mg, 3 mmol) and glacial acetic acid (0.1 mL, 1.98 mmol) in acetonitrile (6 mL) was stirred at ambient temperature for 4 hours. The resulting suspension was filtered and the collected solid was washed with copious amounts of ether. Drying *in vacuo* afforded a white solid (490 mg, 82% yield): NMR (DMSO-d₆, 300 MHz): 7.90-7.70 (br s, 0.5H), 7.50-7.20 (br. s, 0.5H), 6.90 (s, 2H), 6.90 (s, 2H), 3.50-3.10 (br s, 3H), 2.30-2.20 (br s, 3H), 2.05 (d, 1H, J = 7), 1.96 (s, 6H), 1.87 (s, 6H); CI-MS: 244 (M + H).

### D. 5-methyl-3-(2,4,6-trimethylphenyl){1,5-a]-[1,2,3]-triazolo-[1,3,5]-triazin-7(4H)-one

Sodium (368 mg, 16.2 mmol) was added with stirring to ethanol (10 mL) at room temperature. After the sodium had reacted, 4-(2,4,6-trimethylphenyl)-5-acetamidino-triazole, acetic acid salt (490 mg, 1.6 mmol) and diethyl carbonate (1.6 mL, 13 mmol) were added. The reaction mixture was stirred at reflux temperature for 5 hours, then cooled to room temperature. The reaction mixture was diluted with water; a 1N HCl solution was added until pH = 5-6 and three extractions with ethyl acetate were performed. The combined organic layers were dried over MgSO₄ and filtered. Solvent was removed *in vacuo* to give a yellow residue. Trituration with ether and filtration afforded a yellow solid (300 mg, 69% yield): NMR (CDCl₃,300 MHz): 6.98 (s, 2H), 2.55 (s, 3H), 2.35 (s, 3H), 2.10 (s, 6H); CI-MS: 270 (M + H).

### EXAMPLE 3 (Reference Example)

### Preparation of 4-(di(carbomethoxy)methyl)-2,7-dimethyl-8-(2,4-dimethylphenyl)[1,5-a]-pyrazolo-1,3,5-triazine

### (Formula 1, where R³ is CH(CHCO₂CH₃)₂, R₁ is CH₃, Z is C-CH₃, Ar is 2,4-dimethylphenyl)

### A. 4-chloro-2,7-dimethyl-8-(2,4-dichlorophenyl)[1,5-a]-pyrazolotriazine

A mixture of 2,7-dimethyl-8-(2,4-dimethylphenyl)[1,5-a] -pyrazolo-1,3,5-triazin-4-one (Example 1, 1.38g, 4.5 mmol), N,N-dimethylaniline (1 mL, 8 mmol) and phosphorus oxychloride (10 mL) was stirred at reflux temperature for 48 hours. The excess phosphorus oxychloride was removed *in vacuo.* The residue was poured onto ice-water, stirred briefly and extracted quickly with ethyl acetate three times. The combined organic layers were washed with ice water, then dried over MgSO₄ and filtered. Solvent was removed *in vacuo* to give a brown oil. Flash column chromatography (ethyl acetate:hexanes::1:4) gave one fraction (Rf = 0.5) Solvent was removed in vacuo to afford a yellow oil (1.0g, 68% yield): NMR (CDCl₃,300 MHz): 7.55 (d, 1H, J = 1), 7.38 (dd, 1M, J = 7, 1), 7.30 (d, 1H, J = 7),2.68 (s, 3H), 2.45 (s, 3H); CI-MS: 327 (M + H).

### B. 4-(di(carbomethoxy)methyl)-2,7-dimethyl-8-(2,4-dimethylphenyl)[1,5-a]-pyrazolo-1,3,5-triazine

Sodium hydride (60% in oil, 80 mg, 2 mmol) was washed with hexanes twice, decanted after each washing and taken up in anhydrous tetrahydrofuran (THF, 1 mL). A solution of diethyl malonate (0.32g, 2 mmol) in THF (2 mL) was added dropwise over 5 min, during which time vigorous gas evolution ensued. A solution of 4-chloro-2,7-dimethyl-8-(2,4-dichlorophenyl)[1,5-a]-pyrazolotriazine (0.5g, 1.75 mmol) in THF (2 mL) was added and the reaction mixture was then stirred under a nitrogen atmosphere for 48 hours. The resulting suspension was poured onto water and extracted three times with ethyl acetate. The combined organic layers were washed once with brine, dried over MgSO₄ and filtered. Solvent was removed *in vacuo* to give a brown oil. Column chromatography (ethyl acetate:hexanes::1:9) afforded, after removal of solvent *in vacuo,* a pale yellow solid (Rf = 0,2, 250 mg, 35% yield): mp 50-52°C; NMR (CDCl₃, 300 MHz): 12.35 (br.s, 1H, 7.15-7.00 (m, 3H), 4.40 (q, 2H, J = 7), 4.30 (q, 2H, J = 7), 2.4, 2.35, 2.3, 2.2, 2.1 (5 s, 12H), 1.4 (t, 3H, J = 7), 1.35-1.25 (m, 3H); CI-HRMS: Calcd: 411.2032, Found: 411.2023.

### EXAMPLE 6

### Preparation of 4-(1,3-dimethoxy-2-propylamino)-2,7-dimethyl-8-(2,4-dichlorophenyl) [1,5-a]-pyrazolo-1,3,5-triazine

### (Formula 1, where R³ is NHCH(CH₂OCH₃)₂, R₁ is CH₃, Z is C-CH₃, Ar is 2,4-dichlorophenyl)

### A. 4 -chloro-2,7-dimethyl-8-(2,4-dichlorophenyl) [1,5-a]-pyrazolotriazine

A mixture of 2,7-dimethyl-8-(2,4 dimethylphenyl) [1,5-a]-pyrazolo-1,3,5-triazin-4-one (Example 1, 1.38g, 4.5 mmol), N,N-dimethylaniline (1 mL, 8 mmol) and phosphorus oxychloride (10 mL) was stirred at reflux temperature for 48 hours. The excess phosphorus oxychloride was removed in vacuo. The residue was poured onto ice-water, stirred briefly and extracted quickly with ethyl acetate three times. The combined organic layers were washed with ice water, then dried over MgSO₄ and filtered. Solvent was removed *in vacuo* to give a brown oil. Flash column chromatography (ethyl acetate:hexanes: :1:4) gave one fraction (Rf = 0.5) Solvent was removed *in vacuo* to afford a yellow oil (1.0g, 68% yield): NMR (CDCl₃, 300 MHz) : 7.55 (d, 1H, J = 1), 7.38 (dd, 1H, J = 7,1), 7.30 (d, 1H, J = 7), 2.68 (s, 3H), 2.45 (s, 3H); CI-MS: 327 (M + H).

### B. 4-(1,3-dimethoxy-2-propylamino)-2,7-dimethyl-8-(2,4-dichlorophenyl)[1,5-a]-pyrazolo-1,3,5-triazine

A mixture of 4-chloro-2,7-dimethyl-8-(2,4-dichlorophenyl)[1,5-a]-pyrazolo-1,3,5-triazine (Part A, 570 mg, 1.74 mmol), 1,3-dimethoxypropyl-2-aminopropane (25mg, 2.08 mmol) and ethanol (10 mL) was stirred at ambient temperature for 18 hours. The reaction mixture was poured onto water (25 mL) and extracted three times with ethyl acetate. The combined organic layers were dried over MgSO₄ and filtered. Solvent was removed *in vacuo*. Column chromatography (CH₂Cl₂:CH₃OH::50:1) afforded one fraction. Removal of solvent *in vacuo* gave a solid (250 mg, 35% yield): mp 118-120°C; NMR (CDCl₃, 300 MHz) : 7.50 (s, 1H), 7.28 (dd, 2H, J = 8,1), 6.75 (d, 1H, J = 8), 4.70-4.58 (m, 1H), 3.70-3.55 (m, 4H), 3.43 (s, 6H), 2.50 (s, 3H), 2.35 (s, 3H) ; CI-HRMS : Calcd: 409.1072, Found: 409.1085; Analysis Calcd. for C₁₈H₂₁Cl₂N₅O₂ : C, 52.69, H, 5.17, N, 17.07, Cl, 17.28; Found: C, 52.82, H, 5.06, N, 16.77, Cl, 17.50.

Using the above procedures and modifications known to one skilled in the art of organic synthesis, the following additional examples of Tables 1-4 may be prepared.

The examples delineated in TABLE 1 may be prepared by the methods outlined in Examples 1, 2, 3 or 6. Commonly used abbreviations are: Ph is phenyl, Pr is propyl, Me is methyl, Et is ethyl, Bu is butyl, Ex is Example.

**TABLE 1**

| | | | | |
|---|---|---|---|---|
| 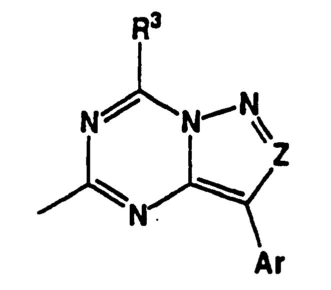 | | | | |

| Ex. | Z | B₃ | Ar | mp (°C) |
|---|---|---|---|---|
| 6^{a} | C-Me | NHCH(CH₂OMe)₂ | 2,4-Cl₂-Ph | 118-120 |
| 7^{b} | C-Me | NHCHPr₂ | 2,4-Cl₂-Ph | 114-116 |
| 8^{c} | C-Me | NEtBu | 2,4-Cl₂-Ph | oil |
| 9^{d} | C-Me | NPr(CH₂-c-C₃H₅) | 2,4-Cl₂-Ph | oil |
| 10^{e} | C-Me | N(CH₂CH₂OMe)₂ | 2,4-Cl₂-Ph | oil |
| 11^{f} | C-Me | NH-3-heptyl | 2,4-Cl₂-Ph | 90-92 |
| 12^{g} | C-Me | NHCH(Et)CH₂OMe | 2,4-Cl₂-Ph | 179-181 |
| 13^{h} | C-Me | NEt₂ | 2,4-Cl₂-Ph | 133-134 |
| 14ⁱ | C-Me | NHCH(CH₂OEt)₂ | 2,4-Cl₂-Ph | oil |
| 15^{j} | C-Me | NH-3-pentyl | 2,4-Cl₂-Ph | 139-140 |
| 16^{k} | C-Me | NMePh | 2,4-Cl₂-Ph | 60-62 |
| 17^{l} | C-Me | NPr₂ | 2,4-Cl₂-Ph | oil |
| 18^{m} | C-Me | NH-3-hexyl | 2,4-Cl₂-Ph | 130-132 |
| 19 | C-Me | morpholino | 2,4-Cl₂-Ph | |
| 20 | C-Me | N(CH₂Ph)CH₂CH₂OMe | 2,4-Cl₂-Ph | |
| 21 | C-Me | NHCH(CH₂Ph)CH₂OMe | 2,4-Cl₂-Ph | |
| 22 | C-Me | NH-4-tetrahydropyranyl | 2,4-Cl₂-Ph | |
| 23 | C-Me | NH-cyclopentyl | 2,4-Cl₂-Ph | |
| 24 | C-Me | 1,2,3,4-tetrahydro- | 2,4-Cl₂-Ph | |
| | | isoquinolinyl | | |
| 25 | C-Me | CH₂-(1,2,3,4-tetrahydroisoquinolinyl) | 2,4-Cl₂-Ph | |
| 49^{o} | C-Me | NHCH(CH₂OMe)₂ | 2,4,6-Me₃-Ph | 125-127 |
| 50 | C-Me | NHCHPr₂ | 2,4,6-Me₃-Ph | |
| 51 | C-Me | NEtBu | 2,4,6-Me₃-Ph | |
| 52 | C-Me | NPr(CH₂-c-C₃H₅) | 2,4,6-Me₃-Ph | |
| 53^{ae} | C-Me | N(CH₂CH₂OMe)₂ | 2,4,6-Me₃-Ph | 123-124 |
| 54 | C-Me | NH-3-heptyl | 2,4,6-Me₃-Ph | |
| 55^{ac} | C-Me | NRCH(Et)CH₂OMe | 2,4,6-Me₃-Ph | 145-146 |
| 56^{ah} | C-Me | NEt₂ | 2,4,6-Me₃-Ph | 88-90 |
| 57^{ai} | C-Me | NHCH(CH₂OEt)₂ | 2,4,6-Me₃-Ph | 132-134 |
| 58^{ad} | C-Me | NH-3-pentyl | 2,4,6-Me₃-Ph | 134-135 |
| 59 | C-Me | NMePh | 2,4,6-Me₃-Ph | |
| 60 | C-Me | NPr₂ | 2,4,6-Me₃-Ph | |
| 61 | C-Me | NH-3-hexyl | 2,4,6-Me₃-Ph | |
| 62 | C-Me | morpholino | 2,4,6-Me₃-Ph | |
| 63 | C-Me | N(CH₂Ph)CH₂CH₂OMe | 2,4,6-Me₃-Ph | |
| 64 | C-Me | NHCH(CH₂Ph)CH₂OMe | 2,4,6-Me₃-Ph | |
| 65 | C-Me | NH-4-tetrahydropyranyl | 2,4,6-Me₃-Ph | |
| 66 | C-Me | NH-cyclopentyl | 2,4,6-Me₃-Ph | |
| 67 | C-Me | 1,2,3,4-tetrahydroisoquinolinyl | 2,4,6-Me₃-Ph | |
| 92^{p} | C-Me | NHCH(CH₂OMe)₂ | 2,4-Me₂-Ph | 44-45 |
| 93^{q} | C-Me | N(CH₂CH₂OMe)₂ | 2,4-Me₂-Ph | oil |
| 94^{r} | C-Me | NHCH(Et)CH₂OMe | 2,4-Me₂-Ph | 102-104 |
| 95^{s} | C-Me | NH-3-pentyl | 2,4-Me₂-Ph | 102-104 |
| 96^{t} | C-Me | NEt₂ | 2,4-Me₂-Ph | oil |
| 97^{u} | C-Me | N(CH₂CN)₂ | 2,4-Me₂-Ph | 148-150 |
| 98^{v} | C-Me | NHCH(Me)CH₂OMe | 2,4-Me₂-Ph | 102-104 |
| 100^{x} | C-Me | NPr-c-C₃H₅ | 2,4-Me₂-Ph | oil |
| 101^{y} | C-Me | NHCH(Me)CH₂NMe₂ | 2,4-Me₂-Ph | 47-48 |
| 102^{z} | C-Me | N(c-C₃H₅)CH₂CH₂CN | 2,4-Me₂-Ph | 117-118 |
| 103^{aa} | C-Me | N(Pr)CH₂CH₂CN | 2,4-Me₂-Ph | oil |
| 104^{ab} | C-Me | N(Bu)CH₂CH₂CN | 2,4-Me₂-Ph | oil |
| 105 | C-Me | NHCHPr₂ | 2,4-Me₂-Ph | |
| 106 | C-Me | NEtBu | 2,4-Me₂-Ph | |
| 107 | C-Me | NPr(CH₂-c-C₃H₅) | 2,4-Me₂-Ph | |
| 108 | C-Me | NH-3-heptyl | 2,4-Me₂-Ph | |
| 109 | C-Me | NEt₂ | 2,4-Me₂-Ph | |
| 110 | C-Me | NHCH(CH₂OEt)₂ | 2,4-Me₂-Ph | |
| 111 | C-Me | NH-3-pentyl | 2,4-Me₂-Ph | |
| 112 | C-Me | NMePh | 2,4-Me₂-Ph | |
| 113 | C-Me | NPr₂ | 2,4-Me₂-Ph | |
| 114 | C-Me | NH-3-hexyl | 2,4-Me₂-Ph | |
| 115 | C-Me | morpholino | 2,4-Me₂-Ph | |
| 116 | C-Me | N(CH₂Ph)CH₂CH₂OMe | 2,4-Me₂-Ph | |
| 117 | C-Me | NHCH(CH₂Ph)CH₂OMe | 2,4-Me₂-Ph | |
| 118 | C-Me | NH-4-tetrahydropyranyl | 2,4-Me₂-Ph | |
| 119 | C-Me | NH-cyclopentyl | 2,4-Me₂-Ph | |
| 120 | C-Me | 1,2,3,4-tetrahydroisoquinolinyl | 2,4-Me₂-Ph | |
| 145^{bc} | C-Me | NHCH(CH₂OMe)₂ | 2-Me-4-MeO-Ph | 45-46 |
| 146^{bd} | C-Me | N(CH₂CH₂OMe)₂ | 2-Me-4-MeO-Ph | oil |
| 147^{be} | C-Me | NHCH(Et)CH₂OMe | 2-Me-4-MeO-Ph | 86-88 |
| 148^{bf} | C-Me | N(Pr)CH₂CH₂CN | 2-Me-4-MeO-Ph | oil |
| 150^{af} | C-Me | NHCH(CH₂OMe)₂ | 2-Br-4-MeO-Ph | 88-90 |
| 151^{al} | C-Me | N(CH₂CH₂OMe)₂ | 2-Br-4-MeO-Ph | oil |
| 152^{ag} | C-Me | NHCH(Et)CH₂OMe | 2-Br-4-MeO-Ph | 95-97 |
| 153 | C-Me | N(Pr)CH₂CH₂CN | 2-Br-4-MeO-Ph | |
| 155 | C-Me | NHCH(CH₂OMe)₂ | 2-Me-4-NMe₂-Ph | |
| 156 | C-Me | N(CH₂CH₂OMe)₂ | 2-Me-4-NMe₂-Ph | oil |
| 157 | C-Me | NHCH(Et)CH₂OMe | 2-Me-4-NMe₂-Ph | |
| 158 | C-Me | N(Pr)CH₂CH₂CN | 2-Me-4-NMe₂-Ph | |
| 160 | C-Me | NHCH(CH₂OMe)₂ | 2-Br-4-NMe₂-Ph | |
| 161 | C-Me | N(CH₂CH₂OMe)₂ | 2-Br-4-NMe₂-Ph | |
| 162 | C-Me | NHCH(Et)CH₂OMe | 2-Br-4-NMe₂-Ph | |
| 163 | C-Me | N(Pr)CH₂CH₂CN | 2-Br-4-NMe₂-Ph | |
| 165 | C-Me | NHCH(CH₂OMe)₂ | 2-Br-4-i-Pr-Ph | |
| 166 | C-Me | N(CH₂CH₂OMe)₂ | 2-Br-4-i-Pr-Ph | |
| 167 | C-Me | NHCH(Et)CH₂OMe | 2-Br-4-i-Pr-Ph | |
| 168 | C-Me | N(Pr)CH₂CH₂CN | 2-Br-4-i-Pr-Ph | |
| 170 | C-Me | NHCH(CH₂OMe)₂ | 2-Br-4-Me-Ph | |
| 171 | C-Me | N(CH₂CH₂OMe)₂ | 2-Br-4-Me-Ph | |
| 172 | C-Me | NHCH(Et)CH₂OMe | 2-Br-4-Me-Ph | |
| 173 | C-Me | N(Pr)CH₂CH₂CN | 2-Br-4-Me-Ph | |
| 175^{ar} | C-Me | NHCH(CH₂OMe)₂ | 2-Me-4-Br-Ph | 108-109 |
| 176 | C-Me | N(CH₂CH₂OMe)₂ | 2-Me-4-Br-Ph | |
| 177 | C-Me | NHCH(Et)CH₂OMe | 2-Me-4-Br-Ph | |
| 178 | C-Me | N(Pr)CH₂CH₂CN | 2-Me-4-Br-Ph | |
| 180 | C-Me | NHCH(CH₂OMe)₂ | 2-Cl-4,6-Me₂-Ph | |
| 181 | C-Me | N(CH₂CH₂OMe)₂ | 2-Cl-4,6-Me₂-Ph | |
| 182 | C-Me | NHCH(CH₂OMe)₂ | 4-Br-2,6-(Me)₂-Ph | |
| 183 | C-Me | N(CH₂CH₂OMe)₂ | 4-Br-2,6-(Me)₂-Ph | |
| 184 | C-Me | NHCH(CH₂OMe)₂ | 4-i-Pr-2-SMe-Ph | |
| 185 | C-Me | N(CH₂CH₂OMe)₂ | 4-i-Pr-2-SMe-Ph | |
| 186 | C-Me | NHCH(CH₂OMe)₂ | 2-Br-4-CF₃-Ph | |
| 187 | C-Me | N(CH₂CH₂OMe)₂ | 2-Br-4-CF₃-Ph | |
| 188 | C-Me | NHCH(CH₂OMe)₂ | 2-Br-4,6-(MeO)₂-Ph | |
| 189 | C-Me | N(CH₂CH₂OMe)₂ | 2-Br-4,6-(MeO)₂-Ph | |
| 190 | C-He | NHCH(CH₂OMe)₂ | 2-Cl-4,6-(MeO)₂-Ph | |
| 191 | C-Me | N(CH₂CH₂OMe)₂ | 2-Cl-4,6-(MeO)₂-Ph | |
| 192 | C-Me | NHCH(CH₂OMe)₂ | 2,6-(Me)₂-4-SMe-Ph | |
| 193 | C-Me | N(CH₂CH₂OMe)₂ | 2,6-(Me)₂-4-SMe-Ph | |
| 194 | C-Me | NHCH(CH₂OMe)₂ | 4-(COMe)-2-Br-Ph | |
| 195 | C-Me | N(CH₂CH₂OMe)₂ | 4-(COMe)-2-Br-Ph | |
| 196 | C-Me | NHCH(CH₂OMe)₂ | 2,4,6-Me₃-pyrid-3-yl | |
| 197 | C-Me | N(CH₂CH₂OMe)₂ | 2,4,6-Me₃-pyrid-3-yl | |
| 198 | C-Me | NHCH(CH₂OMe)² | 2,4-(Br)₂-Ph | |
| 199 | C-Me | N(CH₂CH₂OMe)₂ | 2,4-(Br)₂-Ph | |
| 200 | C-Me | NHCH(CH₂OMe)₂ | 4-i-Pr-2-SMe-Ph | |
| 201 | C-Me | N(CH₂CH₂OMe)₂ | 4-i-Pr-2-SMe-Ph | |
| 202 | C-Me | NHCH(CH₂OMe)₂ | 4-i-Pr-2-SO₂Me-Ph | |
| 203 | C-Me | N(CH₂CH₂OMe)₂ | 4-i-Pr-2-SO₂Me-Ph | |
| 204 | C-Me | NHCH(CH₂OMe)₂ | 2,6-(Me)₂-4-SMe-Ph | |
| 205 | C-Me | N(CH₂CH₂OMe)₂ | 2,6-(Me)₂-4-SMe-Ph | |
| 206 | C-Me | NHCH(CH₂OMe)₂ | 2,6-(Me)2-4-SO₂Me-Ph | |
| 207 | C-Me | N(CH₂CH₂OMe)₂ | 2,6-(Me)2-4-SO₂Me-Ph | |
| 208 | C-Me | NHCH(CH₂OMe)₂ | 2-I-4-i-Pr-Ph | |
| 209 | C-Me | N(CH₂CH₂OMe)₂ | 2-I-4-i-Pr-Ph | |
| 210 | C-Me | NHCH(CH₂OMe)₂ | 2-Br-4-N(Me)₂-6-MeO-Ph | |
| 211 | C-Me | N(CH₂CH₂OMe)₂ | 2-Br-4-N(Me)₂-6-MeO-Ph | |
| 212 | C-Me | NHCH(CH₂OMe)₂ | 2,4-[SMe]2-Ph | |
| 213 | C-Me | N(CH₂CH₂OMe)₂ | 2,4-[SMe]2-Ph | |
| 214 | C-Me | NHCH(CH₂OMe)₂ | 2,4-[SO₂Me]2-Ph | |
| 215 | C-Me | N(CH₂CH₂OMe)₂ | 2,4-[SO₂Me]2-Ph | |
| 216 | C-Me | NHCH(CH₂OMe)₂ | 4-i-Pr-2-SMe-Ph | |
| 217 | C-Me | N(CH₂CH₂OMe)₂ | 4-i-Pr-2-SMe-Ph | |
| 218 | C-Me | NHCH(CH₂OMe)₂ | 4-i-Pr-2-SO₂Me-Ph- | |
| 219 | C-Me | N(CH₂CH₂OMe)₂ | 4-i-Pr-2-SO₂Me-Ph | |
| 220 | C-Me | NHCH(CH₂OMe)₂ | 2-N(Me)₂-4-Me-Ph | |
| 221 | C-Me | N(CH₂CH₂OMe)₂ | 2-N(Me)₂-4-Me-Ph | |
| 222 | C-Me | NHCH(CH₂OMe)₂ | 2-MeS-4,6-(Me)₂-Ph | |
| 223 | C-Me | N(CH₂CH₂OMe)₂ | 2-MeS-4,6-(Me)₂-Ph | |
| 224 | C-Me | NHCH(CH₂OMe)₂ | 2-(CH₃CO)-4,6-(Me)₂-Ph | |
| 225 | C-Me | N(CH₂CH₂OMe)₂ | 2-(CH₃CO)-4,6-(Me)₂-Ph | |
| 226 | C-H | NHCH(CH₂OMe)₂ | 2,4-Me₂-Ph | |
| 227 | C-H | NHCH(CH₂OMe)₂ | 2,4-Me₂-Ph | |
| 228 | C-CF3 | N(CH₂CH₂OMe)₂ | 2,4-Me₂-Ph | |
| 229 | C-CF₃ | N(CH₂CH₂OMe)₂ | 2,4-Me₂-Ph | |
| 316^{an} | C-Me | NEt₂ | 2-Br-4-MeO-Ph | oil |
| 317^{am} | C-Me | NH-3-pentyl | 2-Br-4-MeO-Ph | oil |
| 318^{aj} | C-Me | NHCH(CH₂CH₂OMe)CH₂OMe | 2,4,6-Me₃-Ph | 101-103 |
| 319^{ao} | C-Me | NH(c-C₃H₅) | 2,4-Me₂-Ph | oil |
| 320^{ak} | C-Me | morpholino | 2,4,6-Me₃-Ph | 139-141 |
| 321^{ap} | C-Me | NHCH(CH₂OMe)₂ | 2-CN-4-Me-Ph | 152-153 |
| 322^{aq} | C-Me | N(c-C₃H₅)CH₂CH₂CN | 2,4,6-Me₃-Ph | 149-151 |
| 324^{as} | C-Me | NHCH(CH₂CH₂OMe)CH₂OMe | 2-Me-4-Br-Ph | 115-117 |
| 325^{at} | C-Me | NHCH(CH₂OMe)₂ | 2,5-Me₂-4-MeO-Ph | 55-57 |
| 326^{au} | C-Me | N(CH₂CH₂OMe)₂ | 2,5-Me₂-4-MeO-Ph | 72 |
| 327^{av} | C-Me | NH-3-pentyl | 2,5-Me₂-4-MeO-Ph | 45-47 |
| 328^{aw} | C-Me | NEt₂ | 2,5-Me₂-4-MeO-Ph | oil |
| 329^{ax} | C-Me | NHCH(CH₂OMe)₂ | 2-Cl-4-MePh | 80-81 |
| 330^{ay} | C-Me | NCH(Et)CH₂OMe | 2-Cl-4-MePh | 77-79 |
| 331^{az} | C-Me | N(CH₂CH₂OMe)₂ | 2-Cl-4-MePh | oil |
| 332^{ba} | C-Me | (S)-NHCH(CH₂CH₂OMe)CH₂OMe | 2-Cl-4-MePh | 139-140 |
| 333^{bb} | C-Me | N(c-C₃H₅)CH₂CH₂CN | 2,5-Me₂-4-MeOPh | 120-122 |
| 334^{bg} | C-Me | NEt₂ | 2-Me-4-MeOPh | oil |
| 336^{bi} | C-Me | (S)-NHCH(CH₂CH₂OMe)CH₂OMe | 2-Me-4-MeOPh | oil |
| 337^{bj} | C-Me | N(c-C₃H₅)CH₂CH₂CN | 2-Me-4-MeOPh | 129 |
| 338^{bk} | C-Me | NHCH(CH₂CH₂OEt)₂ | 2-Me-4-MeOPh | amorph. |
| 339 | C-Me | N(c-C₃H₅)CH₂CH₂CN | 2,4-Cl₂-Ph | 109-110 |
| 340 | C-Me | (S)-NHCH(CH₂CH₂OMe)CH₂OMe | 2,4-Cl₂-Ph | 93-94 |
| 341 | C-Me | NH-3-pentyl | 2-Me-4-BrPh | 118-119 |
| 342 | C-Me | N(CH₂CH₂OMe)₂ | 2-Me-4-BrPh | oil |
| 343 | C-Me | NHCH(CH₂-iPr)CH₂OMe | 2,4-Me₂-Ph | oil |
| 344 | C-Me | NHCH(Pr)CH₂OMe | 2,4-Me₂-Ph | 94-95 |
| 345 | C-Me | NHCH(Et)CH₂OEt | 2,4-Me₂-Ph | 76-77 |
| 346 | C-Me | NHCH(CH₂OMe)CH₂CH₂OMe | 2-Me-4-Me₂NPh | oil |
| 347 | C-Me | NEt₂ | 2-Me-4-ClPh | oil |
| 348 | C-Me | NH-3-pentyl | 2-Me-4-ClPh | 122-124 |
| 349 | C-Me | N(CH₂CH₂OMe)₂ | 2-Me-4-ClPh | oil |
| 350 | C-Me | NHCH(CH₂OMe)₂ | 2-Me-4-ClPh | 122-123 |
| 351 | C-Me | NEt₂ | 2-Me-4-ClPh | oil |
| 352 | C-Me | NEt₂ | 2-Cl-4-MePh | oil |
| 353 | C-Me | NH-3-pentyl | 2-Cl-4-MePh | 120-121 |
| 354 | C-Me | NHCH(CH₂OMe)₂ | 2-Cl-4-MeOPh | |
| 355^{bl} | C-Me | N(CH₂CH₂OMe)₂ | 2-Cl-4-MeOPh | oil |
| 356^{bm} | C-Me | NHCH(Et)CH₂OMe | 2-Cl-4-MeOPh | 108-110 |
| 357^{bn} | C-Me | N(c-Pr)CH₂CH₂CN | 2-Cl-4-MeOPh | 127-129 |
| 359^{bo} | C-Me | NEt₂ | 2-Cl-4-MeOPh | oil |
| 359^{bp} | C-Me | NH-3-pentyl | 2-Cl-4-MeOPh | 77-79 |
| 360 | C-Me | NHCH(Et)CH₂CH₂OMe | 2-Cl-4-MeOPh | |
| 361 | C-Me | NHCH(Me)CH₂CH₂OMe | 2-Cl-4-MeOPh | |
| 362 | C-Me | NHCH(Et)CH₂CH₂OMe | 2-Br-4-MeOPh | |
| 363 | C-Me | NHCH(Me)CH₂CH₂OMe | 2-Br-4-MeOPh | |
| 364 | C-Me | NHCH(Et)CH₂CH₂OMe | 2-Me-4-MeOPh | |
| 365 | C-Me | NHCH(Me)CH₂CH₂OMe | 2-Me-4-MeOPh | |
| 366 | C-Me | NHCH(CH₂OMe)₂ | 2-Cl-4,5-(MeO)₂Ph | |
| 367 | C-Me | N(CH₂CH₂OMe)₂ | 2-Cl-4,5-(MeO)₂Ph | |
| 368 | C-Me | NHCH(Et)CH₂OMe | 2-Cl-4,5-(MeO)₂Ph | |
| 369 | C-Me | N(c-Pr)CH₂CH₂CN | 2-Cl-4,5-(MeO)₂Ph | |
| 370 | C-Me | NEt₂ | 2-Cl-4,5-(MeO)₂Ph | |
| 371 | C-Me | NH-3-pentyl | 2-Cl-4,5-(MeO)₂Ph | |
| 372 | C-Me | NHCH(Et)CH₂CH₂OMe | 2-Cl-4,5-(MeO)₂Ph | |
| 373 | C-Me | NHCH(Me)CH₂CH₂OMe | 2-Cl-4,5-(MeO)₂Ph | |
| 374^{bq} | C-Me | NHCH(CH₂OMe)₂ | 2-Br-4,5-(MeO)₂Ph | 137-138 |
| 375 | C-Me | N(CH₂CH₂OMe)₂ | 2-Br-4,5-(MeO)₂Ph | |
| 376^{br} | C-Me | NHCH(Et)CH₂OMe | 2-Br-4,5-(MeO)₂Ph | 147-148 |
| 377 | C-Me | N(c-Pr)CH₂CH₂CN | 2-Br-4,5-(MeO)₂Ph | |
| 379^{bs} | C-Me | NEt₂ | 2-Br-4,5-(MeO)₂Ph | 52-58 |
| 379 | C-Me | NH-3-pentyl | 2-Br-4,5-(MeO)₂Ph | |
| 380 | C-Me | NHCH(Et)CH₂CH₂OMe | 2-Br-4,5-(MeO)₂Ph | |
| 381 | C-Me | NHCH(Me)CH₂CH₂OMe | 2-Br-4,5-(MeO)₂Ph | |
| 382 | C-Me | NHCH(CH₂OMe)₂ | 2-Cl-4,6-(MeO)₂Ph | |
| 383 | C-Me | N(CH₂CH₂OMe)₂ | 2-Cl-4,6-(MeO)₂Ph | |
| 384 | C-Me | NHCH(Et)CH₂OMe | 2-Cl-4,6-(MeO)₂Ph | |
| 385 | C-Me | N(c-Pr)CH₂CH₂CN | 2-Cl-4,6-(MeO)₂Ph | |
| 386 | C-Me | NEt₂ | 2-Cl-4,6-(MeO)₂Ph | |
| 387 | C-Me | NH-3-pentyl | 2-Cl-4,6-(MeO)₂Ph | |
| 388 | C-Me | NHCH(Et)CH₂CH₂OMe | 2-Cl-4,6-(MeO)₂Ph | |
| 389 | C-Me | NHCH(Me)CH₂CH₂OMe | 2-Cl-4,6-(MeO)₂Ph | |
| 390 | C-Me | NHCH(CH₂OMe)₂ | 2-Me-4,6-(MeO)₂Ph | |
| 391 | C-Me | N(CH₂CH₂OMe)₂ | 2-Me-4,6-(MeO)₂Ph | |
| 392 | C-Me | NHCH(Et)CH₂OMe | 2-Me-4,6-(MeO)₂Ph | |
| 393 | C-Me | N(c-Pr)CH₂CH₂CN | 2-Me-4,6-(MeO)₂Ph | |
| 395 | C-Me | NEt₂ | 2-Me-4,6-(MeO)₂Ph | |
| 396 | C-Me | NH-3-pentyl | 2-Me-4,6-(MeO)₂Ph | |
| 397 | C-Me | NHCH(Et)CH₂CH₂OMe | 2-Me-4,6-(MeO)₂Ph | |
| 398 | C-Me | NHCH(Me)CH₂CH₂OMe | 2-Me-4,6-(MeO)₂Ph | |
| 399 | C-Me | N(c-Pr)CH₂CH₂CN | 2-Br-4,6-(MeO)₂Ph | |
| 400 | C-Me | NEt₂ | 2-Br-4,6-(MeO)₂Ph | |
| 401 | C-Me | NH-3-pentyl | 2-Br-4,6-(MeO)₂Ph | |
| 402 | C-Me | NHCH(Et)CH₂CH₂OMe | 2-Br-4,6-(MeO)₂Ph | |
| 403 | C-Me | NHCH(Me)CH₂CH₂OMe | 2-Br-4,6-(MeO)₂Ph | |
| 404 | C-Me | NHCH(Et)CH₂CH₂OMe | 2-Me-4-MeOPh | |
| 405 | C-Me | NHCH(Me)CH₂CH₂OMe | 2-Me-4-MeOPh | |
| 406 | C-Me | NHCH(CH₂OMe)₂ | 2-Me0-4-MePh | |
| 407 | C-Me | N(CH₂CH₂OMe)₂ | 2-Me0-4-MePh | |
| 408 | C-Me | NHCH(Et)CH₂OMe | 2-Me0-4-MePh | |
| 409 | C-Me | N(c-Pr)CH₂CH₂CN | 2-Me0-4-MaPh | |
| 410 | C-Me | NEt₂ | 2-MeO-4-MePh | |
| 411 | C-Me | NH-3-pentyl | 2-Me0-4-MePh | |
| 412 | C-Me | NHCH(Et)CH₂CH₂OMe | 2-Me0-4-MePh | |
| 413 | C-Me | NHCH(Me)CH₂CH₂OMe | 2-Me0-4-MePh | |
| 414 | C-Me | NHCH(CH₂OMe)₂ | 2-Me0-4-MePh | |
| 415 | C-Me | N(CH₂CH₂OMe)₂ | 2-Me0-4-MePh | |
| 416 | C-Me | NHCH(Et)CH₂OMe | 2-Me0-4-MePh | |
| 417 | C-Me | N(c-Pr)CH₂CH₂CN | 2-Me0-4-MePh | |
| 418 | C-Me | NEt₂ | 2-Me0-4-MePh | |
| 419 | C-Me | NH-3-pentyl | 2-Me0-4-MePh | |
| 420 | C-Me | NHCH(Et)CH₂CH₂OMe | 2-Me0-4-MePh | |
| 421 | C-Me | NHCH(Me)CH₂CH₂OMe | 2-Me0-4-MePh | |
| 423^{bt} | C-Me | NHCH(CH₂OMe)₂ | 2-Me0-4-ClPh | oil |
| 424 | C-Me | N(CH₂CH₂OMe)₂ | 2-Me0-4-ClPh | |
| 425 | C-Me | NHCH(Et)CH₂OMe | 2-Me0-4-ClPh | |
| 426 | C-Me | N(c-Pr)CH₂CH₂CN | 2-Me0-4-ClPh | |
| 427 | C-Me | NEt₂ | 2-MeO-4-ClPh | |
| 428 | C-Me | NH-3-pentyl | 2-Me0-4-ClPh | |
| 429 | C-Me | NHCH(Et)CH₂CH₂OMe | 2-Me0-4-ClPh | |
| 430 | C-Me | NHCH(Me)CH₂CH₂OMe | 2-Me0-4-ClPh | |
| NOTES FOR TABLE 1: | | | | |
| a) Analysis Calcd: C, 52.69, H, 5.17, N, 17.07, Cl, 17.28; Found: C, 52.82, H, 5.06, N, 16.77, Cl, 17.50. | | | | |
| b) CI-HRMS: Calcd: 406.1565, Found: 405.1573 (M + H); Analysis Calcd: C: 59.11; H: 6.20; N: 17.23; Cl: 17.45; Found: C: 59.93; H: 6.34; N: 16.50; Cl: 16.95; NMR (CDCl₃, 300 MHz): 0.95 (t, J = 8, 4H), 1.30-1.40 (m, 4H), 1.50-1.75 (m, 4H), 2.35 (s, 3H), 2.48 (s, 3H), 4.30-4.45 (m, 1H), 6.15 (d, J = 8, 1H), 7.30 (s, 2H), 7.50 (s, 1H) | | | | |
| c) CI-HRMS: Calcd: 392.1409, Found: 392.1388 (M + H); NMR (CDCl₃, 300 MHz): 1.00 (t, J =8, 3H), 1.35 (t, J = 8, 3H), 1.41 (q, J = 8, 2H), 1.65-1.85 (m, 2H), 2.30 (s, 3H), 2.40 (s, 3H), 3.85-4.20 (m, 4H), 7.30 (s, 2H), 7.50 (s, 1H). | | | | |
| d) CI-HRMS: Calcd: 404.1409, Found: 404.1408 (M + H); NMR(CDCl₃, 300 MHz): 0.35-0.45 (m, 2H), 0.52-0.62 (m, 2H), 0.98 (t, J = 8, 3H), 1.70-1.90 (m, 2H), 2.30 (s, 3H), 2.40 (s, 3H), 3.85-4.02 (m, 2H), 4.02-4.20 (m, 2H), 7.30 (s, 2H), 7.50 (s, 1H). | | | | |
| e) CI-HRMS: Calcd: 424.1307, Found: 424.1307 (M + H): NMR (CDCl₃, 300 MHz): 2.28 (s, 3H), 2.40 (s, 3H), 3.40 (s, 6H), 3.75 (t, J = 8, 4H), 4.20-4.45 (m, 4H), 7.30 (s, 2H), 7.50 (s, 1H). | | | | |
| f) CI-HRMS: Calcd: 406.1565, Found: 406.1578 (M + H); NMR (CDCl₃, 300 MHz): 0.90 (t, J = 8, 3H), 1.00 (t, J = 8, 3H), 1.28-1.45 (m, 4H), 1.50-1.80 (m, 4H), 2.35 (s, 3H), 2.50 (s, 3H), 4.20-4.35 (m, 1H), 6.10-6.23 (m, 1H), 7.30 (s, 2H), 7.50 (s, 1H) . | | | | |
| g) CI-HRMS: Calcd: 394.1201, Found: 394.1209 (M + H); NMR (CDCl₃, 300 MHz): 1.02 (t, J = 8, 3H), 1.65-1.90 (m, 2H), 2.35 (s, 3H), 2.48 (s, 3H), 3.40 (s, 3H), 3.50-3.60 (m, 2H), 4.35-4.45 (brs, 1H), 6.50-6.60 (m, 1H), 7.30 (s, 2H), 7.50 (s, 1H). | | | | |
| h) CI-HRMS: Calcd: 364.1096, Found: 364.1093 (M + H); Analysis: Calcd: Cr 56.05; H: 5.27; N: 19.23; Cl: 19.46; Found: C: 55.96; H: 5.24; N: 18.93; Cl: 19.25; NMR (CDCl₃, 300 MHz): 1.35 (t, J = 8, 6H), 2.30 (3, 3H), 2.40 (s, 3H), 3.95-4.15 (m, 4H), 7.30 (s, 2H), 7.50 (d, J = 1, 1H). | | | | |
| i) CI-HRMS: Calcd: 438.1464, Found: 438.1454 (M + H); NMR (CDCl₃, 300 MHz): 1.22 (t, J = 8, 6H), 2.35 (s, 3H), 2.47 (s, 3H), 3.39 (q, J = 8, 4H), 3.65 (dd, J = 8, 1, 2H), 3.73 (dd, J = 8, 1, 2H), 4.55-4.65 (m, 1H), 6.75 (d, J = 8, 1H), 7.30 (d, J = 1, 2H), 7.50 (s, 1H). | | | | |
| j) CI-HRMS: Calcd: 378.1252, Found: 378.1249 (M + H); Analysis: Calcd: C: 57.15; H: 5.61; N: 18.51; Cl: 18.74; Found: C: 57.56; H: 5.65; N: 18.35; Cl: 18.45; NMR (CDCl₃, 300 MHz): 1.00 (t, J = 8, 6H), 1.55-1.70 (m, 2H), 1.70-1.85 (m, 2H), 2.35 (s, 3H), 2.50 (s, 3H), 4.15-4.25 (m, 1H), 6.18 (d, J = 8, 1H), 7.30 (s, 2H), 7.50 (s, 1H). | | | | |
| k) CI-HRMS: Calcd: 398.0939, Found: 398.0922 (M + H); Analysis: Calcd: C: 60.31; H: 4.30; N: 17.58; Cl: 17.80; Found: C: 60.29; H: 4.59; N: 17.09; Cl: 17.57; NMR (CDCl₃, 300 MHz): 2.05 (s, 3H), 2.50 (s, 3H), 3.78 (s, 3H), 7.20-7.45 (m, 7H), 7.50 (d, J = 1, 1H). | | | | |
| l) CI-HRMS: Calcd: 392.1409, Found: 392.1391 (M + H); NMR (CDCl₃, 300 MHz): 0.98 (t, J = 8, 6H), 1.70-1.85 (m, 4H), 2.30 (s, 3H), 2.40 (s, 3H), 3.80-4.10 (m, 4H), 7.30 (s, 2H), 7.50 (d, J = 1, 1H). | | | | |
| m) CI-HRMS: Calcd: 392.1409, Found: 392.1415 (M + H); Analysis: Calcd: C: 58.17; H: 5.92; N: 17.85; Cl: 18.07; Found: C: 58.41; H: 5.85: N: 18.10; Cl: 17.75; NMR (CDCl₃, 300 MHz): 0.90-1.05 (m, 6H), 1.35-1.55 (m, 2H), 1.55-1.85 (m, 4H), 2.35 (s, 3H), 2.48 (s, 3H), 4.20-4.35 (m, 1H), 6.15 (d, J = 8, 1H), 7.30 (s, 2H), 7.50 (d, J = 1, 1H). | | | | |
| o) CI-HRMS: Calcd: 383.2321, Found: 383.2309 (M + H); NMR (CDCl₃, 300 MHz): 2.00 (s, 6H), 2.20 (s, 3H), 2.30 (s, 3H), 2.45 (s, 3H), 3.45 (s, 6H), 3.61 (dd, J = 8, 8, 2H), 3.70 (dd, J = 8, 8, 2H), 4.60-4.70 (m, 1H), 6.70 (d, J = 8, 1H), 6.94 (s, 2H). | | | | |
| p) CI-HRMS: Calcd: 310.2243, Found: 370.2246 (M + H); Analysis: Calcd: C: 65.02; H: 7.38; N: 18.96; Found: C: 65.22; H: 7.39; N: 18.71; NMR (CDCl₃, 300 MHz): 2.18 (s, 3H), 2.30 (s, 3H), 2.45 (s, 3H), 3.45 (s, 6H), 3.60 (dd, J = 8, 8, 2H), 3.69 (dd, J = 8, 8, 2H), 4.60-4.70 (m, 1H), 6.70 (d, J = 8, 1H), 7.05 (d, J = 8, 1H), 7.07 (d, J = 8, 1H), 7.10 (s, 1H). | | | | |
| q) CI-HRMS: Calcd: 384.2400, Found: 384.2393 (M + H); NMR (CDCl₃, 300 MHz): 2.16 (s, 3H), 2.25 (s, 3H), 2.35 (s, 3H), 2.39 (s, 3H), 3.40 (s, 6H), 3.77 (t, J = 8, 4H), 4.20-4.45 (m, 4H), 7.02 (d, J = 8, 1H) 7.05 (s, 1H), 7.10 (d, J = 7, 1H). | | | | |
| r) CI-HRMS: Calcd: 354.2294, Found: 354.2271 (M + H); Analysis: Calcd: C: 67.96; H: 7.71; N: 19.81; Found: C: 67.56; H: 7.37; N: 19.60; NMR (CDCl₃, 300 MHz): 1.03 (t, J = 8, 3H), 1.65-1.88 (m, 2H), 2.17 (s, 3H), 2.30 (s, 3H), 2.35 (s, 3H), 2.45 (s, 3H), 3.40 (s, 3H), 3.50-3.62 (m, 2H), 4.30-4.45 (m, 1H), 6.51 (d, J = 8, 1H), 7.04 (d, J = 8, 1H), 7.10 (d, J =8, 1H), 7.12 (s, 1H). | | | | |
| s) CI-HRMS: Calcd: 338.2345, Found: 338.2332 (M + H); Analysis: Calcd: C: 71.18; H: 8.06; N: 20.75; Found: C: 71.43; H: 7.80; N: 20.70; NMR (CDCl₃, 300 MHz): 1.00 (t, J = 8, 6H), 1.55-1.70 (m, 2H), 1.70-1.85 (m, 2H), 2.19 (s, 3H), 2.30 (s, 3H), 2.35 (s, 3H), 2.46 (s, 3H), 4.15-4.26 (m, 1H), 6.17 (d, J = 8, 1H), 7.06 (d, J = 8, 1H), 7.10 (d, J = 1, 1H), 7.13 (s, 1H). | | | | |
| t) CI-HRMS: Calcd: 324.2188, Found: 324.2188 (M + H); NMR (CDCl₃, 300 MHz): 1.25 (t, J = 8, 6H), 2.16 (s, 3H), 2.28 (s, 3H), 2.35 (s, 3H), 2.40 (s, 3H), 3.95-4.20 (m, 4H), 7.05 (dd, J = 8, 1, 1H), 7.07 (s, 1H), 7.10 (d, J = 1, 1H) | | | | |
| u) CI-HRMS: Calcd: 346.1780, Found: 346.1785 (M + H); Analysis: Calcd: C: 66.07; H: 5.54; N: 28.39; Found: C: 66.07; H: 5.60; N: 27.81; NMR (CDCl₃, 300 MHz): 2.15 (s, 3H), 2.32 (s, 3H) 2.17 (s. 3H); 2.52 (s, 3H), 5.25-5.35 (m, 4H), 7.08 (s, 2H), 7.15 (s, 1H). | | | | |
| v) CI-HRMS: Calcd: 340.2137, Found: 340.2137 (M + H); Analysis: Calcd: C: 67.23; H: 7.42; N: 20.63; Found:C: 67.11; H: 7.39; N: 20.26; NMR (CDCl₃, 300 MHz): 1.40 (d, J = 8, 3H), 2.16 (s, 3H), 2.32 (s, 3H), 2.35 (s, 3H), 2.47 (s, 3H), 3.42 (s, 3H), 3.50-3.60 (m, 2H), 4.50-4.15 (m, 1H), 6.56 (d, J = 8, 1H), 7.00-7.15 (m, 3H). | | | | |
| x) CI-HRMS: Calcd: 364.2501, Found: 364.2501 (M + H); NMR (CDCl₃, 300 MHz): 0.35-0.43 (m, 2H), 0.50-0.60 (m, 2H), 0.98 (t, J = 8, 3H), 1.20-1.30 (m, 1H), 1.72-1.90 (m, 2H), 2.18 (s, 3H) 2.28 (s, 3H), 2.35 (s, 3H), 2.40 (s, 3H), 3.88-4.03 (m, 2H), 4.03-4.20 (m, 2H), 7.00-7.15 (m, 3H). | | | | |
| y) CL-HRMS: Calcd: 353.2454, Found: 353.2454 (M + H); Analysis: Calcd: C: 68.15; H: 8.02; N: 23.84; Found: C: 67.43; H: 7.81; N: 23.45; NMR (CDCl₃, 300 MHz) : 1.38 (d, J = 8, 3H), 2.18 (s, 3H), 2.30-2.40 (m, 12H), 2.47 93, 3H), 2.60-2.75 (m, 2H), 4.30-4.50 (m, 1H), 6.60-6.70 (m, 1H), 7.00-7.15 (m, 3H). | | | | |
| z) CI-HRMS: Calcd: 361.2140, Found: 361.2128 (M + H); NMR (CDCl₃, 300 MHz): 0.75-0.83 (m, 2H), 1.00-1.10 (m, 2H), 2.17 (s, 3H), 2.30 (s, 3H), 2.36 (s, 3H), 2.47 (s, 3H), 2.85 (t, J = 8, 2H), 3.30-3.40 (m, 1H), 4.40-4.55 (m, 2H), 7.00-7.18 (m, 3H). aa) CI-HRMS: Calcd: 363.2297, Found: 363.2311 (M + H); NMR (CDCl₃, 300 MHz): 1.01 (t, 3H, J=8), 1.75-1.90 (m, 2H), 2.15 (s, 3H), 2.19 (s, 3H), 2.35 (s, 3H), 2.40 (s, 3H), 2.40 (s, 3H), 2.98 (t, 2H, J = 8), 3.97-4.15 (m, 2H), 4.15-4.30. (m, 2H), 7.03(d, 1H, 1H), 7.08 (d, 1H, J = 8), 7.10 (s, 1H). | | | | |
| ab) CI-HRMS: Calcd: 363.2297, Found: 363.2295 (M + H); NMR (CDCl₃, 300 MHz): 1.01 (t, 3H, J = 8), 1.35-1.55 (m, 2H), 1.75-1.90 (m, 2H), 2.15 (s, 3H), 2.30 (s, 3H), 2.36 (s, 3H), 2.46 (s, 3H), 4.10-4.30 (m, 2H), 4.95-5.10 (br s, 2H), 7.05 (d, 1H, J = 8), 7.10 (d, 1H, J = 8), 7.15 (s, 1H). | | | | |
| ac) CI-HRMS: Calcd: 368.2450, Found: 368.2436; Analysis: Calcd: C, 68.62, H, 7.95, N, 19.06; Found: C, 68.73, H, 7.97, N, 19.09; NMR (CDCl₃, 300 MHz): 1.05 (t, J = 8, 3H), 1.70-1.90 (m, 2H), 2.01 (d, J = 3, 6H), 2.20 (s, 3H), 2.30 (s, 3H), 2.46, 2.465 (s, s, 3H), 3.42, 3.48 (s, s, 3H), 3.53-3.63 (m, 2H), 4.35-4.45 (m, 1H), 6.73 (d, J = 8, 1H), 6.97 (s, 2H). | | | | |
| (ad) CI- HRMS: Calcd: 352.2501, Found: 352.2500 (M + H): Analysis: Calcd: C: 71.76; H: 8.33; N: 19.92, Found: C: 71.55; H: 8.15; N: 19.28; NMR (CDCl₃, 300 MHz): 1.01(t, J = 8, 6H), 1.58 -1.70 (m, 2H), 1.70-1.85 (m, 2H), 2.02 (s, 6H), 2.19 (s, 3H), 2.45 (s, 3H), 4.12-4.28 (m, 1H), 6.18 (d, J = 8, 1H), 6.95 (s, 2H). | | | | |
| (ae) CI- HRMS: Calcd: 398.2556, Found: 398.2551 (M + H); Analysis: Calcd: C: 66.47; H: 7.86; N: 17.62, Found: C: 66.74; H: 7.79; N: 17.70; NMR (CDCl₃, 300 MHz): 2.00 (s, 6H), 2.12 (s, 3H), 2.30 (s, 3H), 2.37 (s, 3H), 3.40 (s, 6H), 3.78 (t, J = 8, 4H), 4.25-4.40 (m, 4H), 6.93 (s, 2H). | | | | |
| (af) CI-HRMS: Calcd: 450.1141, Found: 450.1133 (M + H); Analysis: Calcd: C: 50.67; H: 5.37; N: 15.55; Br: 17.74; Found: C: 52.36; H: 5.84; N: 14.90; Br: 17.44; NMR (CDCl₃, 300 MHz) : 2.32 (s, 3H), 2.57 (s, 3H), 3.42 (s, 6H), 3.60 (q, J = 8, 2H), 3.69 (q, J = 8, 2H), 3.82 (s, 3H), 4.60-4.70 (m, 1H), 6.73 (d, J = 8, 1H), 6.93 (dd, J = 8, 1, 1H), 7.22 (d, J = 8, 1H). | | | | |
| ag) CI-HRMS: Calcd: 434.1192, Found: 434.1169 (M + H); Analysis: Calcd: C: 52.54; H: 5.58; N: 16.12; Br: 18.40; Found: C: 52.57; H: 5.60; N: 15.98; Br: 18.22; NMR (CDCl₃, 300 MHz): 1.00-1.07 (m, 3H), 1.65-1.85 (m, 2H), 2.35 (s, 3H), 2.46, 2.47 (s, s, 3H), 3.40, 3.45 (s, s, 3H), 3.83 (s, 3H), 4.35-4.45 (m, 1H), 6.55 (d, J = 8, 1H), 6.92 (dd, J = 8, 1, 1H), 7.20-7.30 (m, 2H). | | | | |
| ah) CI-HRMS: Calcd: 337.2266, Found: 337.2251 (M + H); Analysis: Calcd: C: 70.18; H: 8.06; N: 20.75; Found: c: 70.69; H: 7.66; N: 20.34; NMR (CDCl₃, 300 MHz): 1.35 (t, J = 8, 6H), 2.01 (s, 6H), 2.15 (s, 3H), 2.30 (s, 3H), 2.38 (s, 3H), 4.07 (q, J = 8, 4H), 6.93 (s, 2H). | | | | |
| ai) CI-HRMS: Calcd: 412.2713, Found: 412.2687 (M + H); Analysis: Calcd: C: 67.13; H: 8.08; N: 17.02; Found : C: 67.22; H: 7.85; N: 17.13; NMR (CDCl₃, 300 MHz):1.24 (t, J = 8, 6H), 2.00 (s, 6H), 2.20 (s, 3H), 2.30 (s, 3H), 2.43 (s, 3H), 3.60 (q, J = 8, 4H), 3.66 (dd, J = 8, 3, 2H), 3.75 (dd, J = 8, 3, 2H), 4.55-4.65 (m, 1H), 6.75 (d, J = 8, 1H), 6.95 (s, 2H). | | | | |
| aj) CI-HRMS: Calcd: 398.2556, Found: 398.2545 (M + H); Analysis: Calcd: C: 66.47; H: 7.86; N: 17.62; Found: C: 66.87; H: 7.62; N: 17.75; NMR (CDCl₃, 300 MHz): 1.95-2.10 (m, 8H), 2.20 (s, 3H), 2.32 (s, 3H), 2.44 (s, 3H), 3.38 (s, 3H), 3.42 (s, 3H), 3.50-3.70 (m, 4H), 4.58-9.70 (m, 1H), 6.87 (d, J = 8, 1H), 6.95 (s, 2H). | | | | |
| ak) CI-HRMS: Calcd: 338.1981, Found: 338.1971 (M + H); Analysis: Calcd: C: 67.63; H: 6.87; N: 20.06; Found: C: 67.67; H: 6.82; N: 20.31; NMR (CDCl₃, 300 MHz): 2.15 (s, 3H), 2.29 (s, 3H), 2.35 (s, 3H), 2.43 (s, 3H), 3.90 (t, J = 8, 4H), 4.35-4.45 (m, 4H), 7.00-7.15 (m, 3H). | | | | |
| al) CI-HRMS: Calcd: 464.1297, Found: 464.1297 (M + H); NMR (CDCl₃, 300 MHz): 2.28 (s, 3H), 2.40 (s, 3H), 3.40 (s, 6H), 3.75 (t, J = 8, 4H), 3.83 (s, 3H), 4.20-4.50 (m, 4H), 6.93 (dd, J = 8, 1, 1H), 7.20 (s, 1H), 7.24 (d, J = 1, 1H). | | | | |
| am) CI-HRMS: Calcd: 418.1242, Found: 418.1223 (M + H); NMR (CDCl₃, 300 MHz): 1.00 (t, d, J = 8, 1, 6H), 1.55-1.75 (m, 4H), 2.34 (s, 3H), 2.49 (s, 3H), 2.84 (s, 3H), 4.15-4.27 (m, 1H), 6.19 (d, J = 8, 1H), 6.93 (dd, J = 8, 1, 1H), 7.21-7.30 (m, 2H). | | | | |
| an) CI-HRMS: Calcd: 404.1086, Found: 404.1079(M+ H); NMR (CDCl₃, 300 MHz): 1.35 (t, J = 8, 6H), 2.28 (s, 3H), 2.40 (s, 3H), 3.83 (s, 3H), 3.90-4.08 (m, 2H), 4.08-4.20 (m, 2H), 6.92 (dd, J = 8, 1, 1H), 7.20-7.25 (m, 2H). | | | | |
| ao) CI-HRMS: Calcd: 308.1875, Found: 308.1872 (M + H); NMR (CDCl₃, 300 MHz): 0.75-0.80 (m, 2H), 0.93-1.00 (m, 2H), 2.16 (s, 3H), 2.28 (s, 3H), 2.35 (s, 3H), 2.53 (s, 3H), 3.00-3.10 (m, 1H), 6.50-6.55 (m, 1H), 7.00-7.15 (m, 3H). | | | | |
| ap) CI-HRMS: Calcd: 397.1988, Found: 397.1984 (M + H); NMR (CDCl₃, 300 MHz): 2.43 (s, 3H), 2.50 (s, 3H), 3.43 (s, 3H), 3.61 (dd, J = 8, 8, 2H), 3.69 (dd, J = 8, 8, 2H), 3.88 (s, 3H), 4.58-4.70 (m, 1H), 6.75 (d, J - 8, 1H), 7.20 (dd, J = 8, 1, 1H), 7.25 (d, J = 1, 1H), 7.40 (s, 1H). | | | | |
| aq) CI-HRMS: Calcd: 375.2297, Found: 375.2286 (M + H); Analysis: Calcd: C: 70.56; H: 7.01; N: 22.44; Found: C: 70.49; H: 6.99; N: 22.45; NMR (CDCl₃, 300 MHz): 0.79-0.85 (m, 2H), 1.00-1.05 (m, 1H), 2.00 (s, 6H), 2.19 (s, 3H), 2.32 (s, 3H), 2.44 (s, 3H), 2.84 (t, J = 8, 2H), 3.30-3.40 (m, 1H), 4.50 (t, J = 8, 2H), 6.95 (s, 2H) | | | | |
| ar) CI-HRMS: Calcd: 434.1192, Found: 434.1189 (M + H) ; Analysis: Calcd: C: 52.54; H: 5.58; N: 16.12; Br: 18.40; Found: C: 52.75; H: 5.59; N: 16.09; Br: 18.67; NMR (CDCl₃, 300 MHz): 2.19 (s, 3H), 2.30 (s, 3H), 2.47 (s, 3H), 3.43 (s, 6H), 3.60 (dd, J = 8, 8, 2H), 3.70 (dd, J = 8,8, 2H), 4.58-4.70 (m, 1H), 6.71 (d, J = 8, 1H), 7.08 (d, J = 8, 1H), 7.37 (dd, J = 8, 1, 1H), 7.45 (d, J = 1, 1H) | | | | |
| as) CI-HRMS: Calcd: 448.1348, Found: 448.1332 (M + H); Analysis: Calcd: C: 53.58; H: 5.85; N: 16.62; Br: 17.82; Found: C: 53.68; H: 5.74; N: 15.52; Br: 13.03; NMR (CDCl₃, 300 MHz): 1.95-2.10 (m, 2H), 2.20 (s, 3H), 2.30 (s, 3H), 2.47 (s, 3H), 3.38 (s, 3H), 3.41 (s, 3H), 3.50-3.67 (m, 4H), 4.55-4.70 (m, 1H), 6.89 (d, J = 8, 1H), 7.05 (d, J = 8, 1H), 7.35 (dd, J = 8, 1, 1H), 7.47 (d, J = 1, 1H). | | | | |
| at) CI-HRMS: Calcd: 400.2349, Found: 400.2348 (M + H); Analysis: Calcd: C: C: 63.14; H: 7.32; N: 17.53; Found: C:63.40; H: 7.08; N: 17.14; NMR (CDCl₃, 300 MHz): 2.16 (s, 3H), 2.20 (s, 3H), 2.30 (s, 3H), 2.46 (s, 3H), 3.42 (s, 6H), 3.60 (q, J = 8, 2H), 3.70 (q, J = 8, 2H), 3.85 (s, 3H), 4.59-4.70 (m, 1H), 6.70 (d, J = 8, 1H), 6.76 (s, 1H), 6.96 (s, 1H). | | | | |
| au) CI-HRMS: Calcd: 414.2505, Found: 414.2493 (M + H); NMR (CDCl₃, 300 MHz): 2.15 (s, 3H), 2.19 (s, 3H), 2.25 (s, 3H), 2.40 (s, 3H), 3.40 (s, 6H), 3.76 (t, J = 8, 4H), 3.84 (s, 3H), 4.20-4.45 (m, 4H), 6.77 (s, 1H), 6.93 (s, 1H). | | | | |
| av) CI-HRMS: Calcd: 368.2450, Found: 368.2447 (M + H); NMR (CDCl₃, 300 MHz): 1.00 (t, J = 8, 6H), 1.55-1.85 (m, 4H), 2.19 (s, 3H), 2.20 (s, 3H), 2.30 (s, 3H), 2.47 (s, 3H), 3.88 (s, 3H), 4.10-4.30 (m, 1H), 6.15 (d, J = 8, 1H), 6.78 (s, 1H), 6.98 (s, 1H). | | | | |
| aw) CI-HRMS: Calcd: 353.2216, Found: 353.2197 (M + H) ; NMR (CDCl₃, 300 MHz): 1.35 (t, J = 8, 6H), 2.17 (s, 3H), 2.19 (s, 3H), 2.28 (s, 3H), 2.40 (s, 3H), 3.85 (s, 3H), 3.90-4.20 (m, 4H), 6.78 (s, 1H), 6.95 (s, 1H). | | | | |
| ax) CI-HRMS : Calcd : 390.1697, Found: 390.1688 (M + H) ; Analysis: Calcd: C: 58.53; H: 6.20; N: 17.96; Cl : 9.09; Found: C: 58.95; H: 6.28; N: 17.73; Cl: 9.15; NMR (CDCl₃, 300 MHz) : 2.35 (s, 3H), 2.37 (s, 3H), 2.48 (s, 3H), 3.42 (s, 6H), 3.60 (dd, J = 8, 8, 2H) 3.68 (dd, J = 8, 8, 2H), 4.59-4.72 (m, 1H), 6.72 (d, J = 8, 1H), 7.12 (d, J = 8, 1H), 7.23 (d, J = 8, 1H), 7.32 (s, 1H). | | | | |
| ay) CI-HRMS: Calcd: 374.1748, Found: 374.1735 (M + H); Analysis: Calcd: C: 61.04; H: 6.47; N: 18.73; Cl: 9.48; Found: C: 61..47; H: 6.54; N: 18.23; Cl: 9.61; NMR (CDCl₃, 300 MHz): 1.01 (t, J = 8, 3H), 1.62-1.88 (m, 4H), 2.35 (s, 3H), 2.37 (s, 3H), 2.48 (d, J = 1, 3H), 3.40, 3.45 (s, s, 3H), 3.50-3.64 (m, 2H), 4.38-4.47 (m, 1H), 6.53 (d, J = 8, 1H), 7.12 (d, J = 8, 1H), 7.07 (d, J = 8, 1H), 7.12 (s, 1H). | | | | |
| az) CI-HRMS: Calcd: 404.1853, Found: 404.1839 (M + H); NMR (CDCl₃, 300 MHz): 2.29 (s, 3H), 2.38 (s, 3H), 2.40 (s, 3H), 3.40 (s, 6H), 3.76 (t, J = 8, 4H), 4.20-4.45 (m, 4H), 7.11 (d, J = 8, 1H), 7.22 (d, J = 8, 1H), 7.31 (s, 1H). | | | | |
| ba) CI-HRMS: Calcd: 404.1853, Found: 404.1859 (M + H); Analysis: C: 59.47; H: 6.50; N: 17.34; Cl: 8.79; Found: C: 59.73; H: 6.46; N: 17.10; Cl: 8.73; NMR (CDCl₃; 300 MHz): 1.95-2.08 (m, 2H), 2.35 (s, 3H), 2.38 (s, 3H), 2.46 (s, 3H), 3.38 (s, 3H), 3.41 (s, 3H), 3.50-3.65 (m, 4H), 4.56-4.70 (m, 1H), 6.85 (d, J = 8, 1H), 7.12 (d, J = 8, 1H), 7.45 (d, J = 8, 1H), 7.32 (s, 1H). | | | | |
| bb) CI-HRMS: Calcd: 391.2246, Found: 391.2258 (M + H); Analysis: C: 67.67; H: 6.71; N: 21.52; Found: C: 67.93; H: 6.70; N: 21.48; NMR (CDCl₃, 300 MHz): 0.76-0.84 (m, 2H), 0.84-0.91 (m, 2H), 1.00-1.08 (m, 2H), 2.15 (s, 3H), 2.20 (s, 3H), 2.29 (s, 3H), 2.45 (s, 3H), 2.85 (t, J = 8, 2H), 3.28-3.30 (m, 1H), 3.85 (s, 3H), 6.78 (s, 1H), 6.95 (s, 1H). | | | | |
| bc) CI-HRMS: Calcd: 386.2192, Found: 386.2181 (M + H); Analysis: C: 62.32; H: 7.06; N: 18.17; Found: C: 62.48; H: 6.83; N: 18.15; NMR (CDCl₃, 300 MHz): 7.1 (d, 1H, J = 8), 6.9 (d, 1H, J = 1), 6.8 (dd, 1H, J = 8,1), 6.7 (br.d, 1H, J = 8), 4.7-4.6 (m, 1H), 3.85 (s, 3H), 3.70-3.55 (m, 4H), 3.45 (s, 6H), 2.5 (s, 3H), 2.3 (s, 3H), 2.15 (s, 3H). | | | | |
| bd) CI-HRMS: Calcd: 400.2349, Found: 400.2336 (M + H) ; NMR (CDCl₃, 300 MHz): 7.1 (d, 1H, J = 7), 6.85 (d, 1H, J = 1), 6.75 (dd, 1H, J = 7,1), 4.45-4.25 (br.s, 4H), 3.75 (t, 4H, J = 7), 3.4 (s, 6H), 2.4 (s, 3H), 2.25 (s, 3H), 2.15 (s, 3H). | | | | |
| be) CI-HRMS: Calcd: 370.2243, Found: 370.2247 (M + H); Analysis: C: 65.02; H: 7.38; N: 18.96; Found: C: 65.28; H: 7.27; N: 18.71; NMR (CDCl₃, 300 MHz) : 7.1 (d, 1H, J = 8), 6.85 (d, 1H, J = 1), 6.8 (dd, 1H, J = 8,1), 6.5 (br. d, 1H, J = 1), 4.5-4.3 (m, 1H), 3.85 (s, 3H), 3.65-3.5 (m, 2H), 3.4 (s, 2H), 2.5 (s, 3H), 2.3 (s, 3H), 2.2 (s, 3H), 1.9-1.7 (m, 2H), 1.05 (t, 3H, J = 7). | | | | |
| bf) CI-HRMS: Calcd: 379.2246, Found: 379.2248 (M + H); NMR (CDCl₃, 300 MHz): 7.1 (d, 1H, J = 8), 6.85 (d, 1H, J = 1), 6.8 (dd, 1H, J = 8,1), 4.3-4.0 (m, 4H), 3.85 (s, 3H), 3.0 (t, 2H, J = 7), 2.45 (s, 3H), 2.3 (s, 3H), 2.2 (s, 3H), 1.9-1.8 (m, 2H), 1.0 (t, 3H, J = 7). | | | | |
| bg) CI-HRMS: Calcd: 340.2137, Found: 340.2122 (M + H); NMR (CDCl₃, 300 MHz): 7.1 (d, 1H, J = 8), 6.85 (d, 1H, J = 1), 6.75 (dd, 1H, J = 8,1), 4.2-4.0 (br.m, 4H), 3.85 (s, 3H, 2.4 (s, 3H), 2.3 (s, 3H), 2.2 (s, 3H), 1.35 (t, 6H, J = 7). | | | | |
| bi) CI-HRMS: Calcd: 400.2349, Found: 400.2346 (M + H); NMR (CDCl₃, 300 MHz): 7.1 (d, 1H, J = 7), 6.9-6.75 (m, 3H), 4.7-4.55 (m, 1H), 3.8 (s, 3H), 3,7-3.5 (m, 4H), 3.45 (s, 3H), 3.35 (s, 3H), 2.5 (s, 3H), 2.3 (s, 3H), 2.2 (s, 3H), 2.1-1.95 (m, 2H). | | | | |
| bj) CI-HRMS: Calcd: 377.2090, Found: 377.2092 (M + H); Analysis: C: 67.00; H: 6.44; N: 22.32; Found: C: 67.35; H: 6.44; N: 22.23; NMR (CDCl₃, 300 MHz): 7.1 (d, 1H, J = 8), 6.9 (d, 1H, J = 1), 6.8 (dd, 1H, J = 8,1), 4.55-4.4 (m, 2H), 3.85 (s, 3H), 3.4-3.3 (m, 1H), 2.85 (t, 2H, J = 7), 2.5 (s, 3H), 2.3 (s, 3H), 2.2 (s, 3H), 1.1-1.0 (m, 2H), 0.85-0.75 (m, 2H). | | | | |
| bk) CI-HRMS: Calcd: 413.2427, Found: 413.2416 (M + H); NMR (CDCl₃, 300Hz): 7.1 (d, 1H, J = 8), 6.85 (d, 1H, J = 1), 6.75 (dd, 1H, J = 8,1), 4.6 (m, 1H), 3.85 (s, 3H), 3.75-3.6(m, 4H), 3.6 (q, 4H, J = 7), 2.5 (s, 3H), 2.3 s, 3H), 2.2 (s, 3H), 1.25 (t, 6H, J = 7). | | | | |
| bl) CI-HRMS: Calcd: 420.1802, Found: 420.1825(M + H); | | | | |
| bm) CI-HRMS: Calcd: 390.1697, Found: 390.1707(M + H); | | | | |
| bn) CI-HRMS: Calcd: 397.1465, Found: 397.1462(M + H); | | | | |
| bo) CI-HRMS: Calcd: 360.1.513, Found: 360.1514(M + H); | | | | |
| bp) CI-HRMS: Calcd: 374.1748, Found: 374.1737(M + H); | | | | |
| bq) CI-HRMS: Calcd: 479.1155, Found: 479.1154(M + H); | | | | |
| br) CI-HRMS: Calcd: 463.1219, Found: 463.1211 (M + H); Analysis Calcd: C: 51.96, H: 5.23, N, 15.15, Br: 17.28; Found: C: 52.29, H: 5.62, N: 14.79, Br: 17.47 | | | | |
| bs) CI-HRMS: Calcd: 433.1113, Found: 433.1114(M, ⁷⁹Br); | | | | |
| bt) NH₃-CI MS: Calcd: 406, Found: 406 (M + H)+; NMR (CDCl₃, 300 MHz) : δ 7.28 (d, J = 10Hz, 1H), 7.03 (d, J=8Hz, 1H), 6.96 (s, 1H), 6.7 (d, J=9, 1H), 4.63 (m, 1H), 3.79 (s, 3H), 3.6 (m, 4H), 3.42 (s, 6H), 2.47 (s, 3H), 2.32 (s, 3H). | | | | |

### EXAMPLE 431 (Reference example)

### Preparation of 2,4,7-dimethyl-8-(4-methoxy-2-methylphenyl)[1,5-a]-pyrazolo-1,3,5-triazine

### (Formula 1, where R³ is CH₃, R₁ is CH₃, Z is C-CH₃, Ar is 2,4-dimethylphenyl)

5-Acetamidino-4-(4-methoxy-2-methylphenyl)-3-methylpyrazole, acetic acid salt (602 mg, 2 mmol) was mixed with a saturated NaHCO₃ solution (10 mL). The aqueous mixture was extracted with EtOAc three times. The combined organic layers were dried over MgSO₄, filtered and concentrated in vacuo. The residue was taken up in toluene (10 mL) and trimethyl orthoacetate (0.36 g, 3 mmol) was added to the suspension. The reaction mixture was heated to reflux temperature under a nitrogen atmosphere and stirred for 16 hours. After being cooled to ambient temperature, the reaction mixture was concentrated in vacuo to give an oily solid. Column chromatography (CHCl₃:MeOH::9:1) afforded, after removal of solvent in vacuo, a yellow viscous oil (Rf = 0.6, 210 mg, 37% yield): NMR (CDCl₃, 300 MHz): 7.15 (d, 1H, J = 8), 6.9 (d, 1H, J = 1), 6.85 (dd, 1H, J = 8,1), 3.85 (s, 3H), 2.95 (s, 3H), 2.65 (s, 3H), 2.4 (s, 3H), 2.15 (s, 3H); CI-HRMS: Calcd: 283.1559, Found: 283.1554 (M + H).

### EXAMPLE 432 (Reference example)

### 7-hydroxy-5-methyl-3-(2-chloro-4-methylphenyl)pyrazolo[1,5-a]pyrimidine

### (Formula 1 where A is CH, R1 is Me, R3 is OH, Z is C-Me, Ar is 2-chloro-4-methylphenyl)

5-Amino-4-(2-chloro-4-methylphenyl)-3-methylpyrazole (1.86 g, 8.4 mmol) was dissolved in glacial acetic acid (30 mL) with stirring. Ethyl acetoacetate (1.18 mL, 9.2 mmol) was then added dropwise to the resulting solution. The reaction mixture was then heated to reflux temperature and stirred for 16 hours, then cooled to room temperature. Ether (100 mL) was added and the resulting precipitate was collected by filtration. Drying in vacuo afforded a white solid (1.0 g, 42% yield): NMR (CDCl₃, 300Hz): 8.70 (br.s 1H), 7.29 (s, 1H), 7.21-7.09 (m, 2H), 5.62 (s, 1H), 2.35 (s, 6H), 2.29 (s, 3H); CI-MS: 288 (M+H).

### EXAMPLE 433 (Reference example)

### 7-chloro-5-methyl-3-(2-chloro-4-methylphenyl)pyrazolo[1,5-a]pyrimidine

### (Formula 1 where A is CH, R1 is Me, R3 is Cl, Z is C-Me, Ar is 2-chloro-4-methylphenyl)

A mixture of 7-hydroxy-5-methyl-3-(2-chloro-4-methylphenyl)-pyrazolo[1,5-a]pyrimidine (1.0 g, 3.5 mmol), phosphorus oxychloride (2.7 g, 1.64 mL, 17.4 mmol), N,N-diethylaniline (0.63 g, 0.7 mL, 4.2 mmol) and toluene (20 mL) was stirred at reflux temperature for 3 hours, then it was cooled to ambient temperature. The volatiles were removed in vacuo. Flash chromatography (EtOAc:hexane::1:2) on the residue gave 7-chloro-5-methyl-3-(2-chloro-4-methylphenyl)-pyrazolo[1,5-a]pyrimidine (900 mg, 84% yield) as a yellow oil: NMR (CDCl₃, 300Hz): 7.35 (s, 1H), 7.28-7.26 (m, 1H), 71.6 (d, 1H, J = 7), 6.80 (s, 1H), 2.55 (s, 3H), 2.45 (s, 3H), 2.40 (s, 3H); CI- MS: 306 (M+H).

### EXAMPLE 434 (Reference example)

### 7-(pentyl-3-amino)-5-methyl-3-(2-chloro-4-methylphenyl)pyrazolo[1,5-a]pyrimidine

### (Formula 1 where A is CH, R1 is Me, R3 is pentyl-3-amino, Z is C-Me, Ar is 2-chloro-4-methylphenyl)

A solution of 3-pentylamine (394mg, 6.5 mmol) and 7-chloro-5-methyl-3-(2-chloro-4-methylphenyl)pyrazolo[1,5-a]pyrimidine (200 mg, 0.65 mmol) in dimethylsulfoxide (DMSO, 10 mL) was stirred at 150°C for 2 hours; then it was cooled to ambient temperature. The reaction mixture was then poured onto water (100 mL) and mixed. Three extractions with dichloromethane, washing the combined organic layers with brine, drying over MgSO₄, filtration and removal of solvent in vacuo produced a yellow solid. Flash chromatography (EtOAc:hexanes::1:4) afforded a white solid (140 mg, 60% yield): mp 139-141°C; NMR (CDCl₃, 300Hz):7.32 (s, 1H), 7.27 (d, 1H, J = 8), 7.12 (d, 1H, J = 7), 6.02 (d, 1H, J = 9), 5.78 (s, 1H), 3.50-3.39 (m, 1H), 2.45 (s, 3H), 2.36 (s, 6H), 1.82-1.60 (m, 4H), 1.01 (t, 6H, J = 8); Analysis Calcd for C₂OH₂₅ClN₄: C, 67.31, H, 7.06, N, 15.70, Cl: 9.93; Found: C, 67.32, H, 6.95, N, 15.50, Cl, 9.93.
The examples delineated in TABLE 2 may be prepared by the methods outlined in Examples 1A, 1B, 432, 433, 434. Commonly used abbreviations are: Ph is phenyl, Pr is propyl, Me is methyl, Et is ethyl, Bu is butyl, Ex is Example, EtOAc is ethyl acetate.

**TABLE 2**

| | | | | |
|---|---|---|---|---|
| 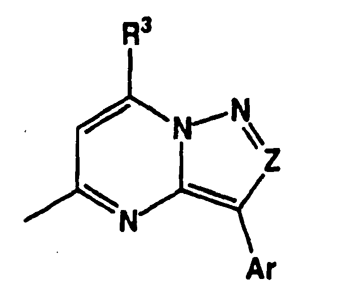 | | | | |

| Ex | Z | R₃ | Ar | mp (°C) |
|---|---|---|---|---|
| 435^{b} | C-Me | N(CH₂CH₂OMe)₂ | 2,4-Cl₂-Ph | 71-73 |
| 436^{c} | C-Me | N(Bu)Et | 2,4-Cl₂-Ph | 86-87 |
| 437^{d} | C-Me | NHCH(Et)CH₂OMe | 2,4-Cl₂-Ph | 110-111 |
| 438^{e} | C-Me | N(Pr)CH₂CH₂CN | 2,4-Cl₂-Ph | 83-85 |
| 439^{f} | C-Me | NH-3-pentyl | 2,4-Cl₂-Ph | 175-176 |
| 440^{g} | C-Me | NHCH(CH₂OMe)₂ | 2,4-Cl₂-Ph | 107 |
| 441h | C-Me | NHCH(Et)₂ | 2,4-Me₂-Ph | oil |
| 442ⁱ | C-Me | NHCH(CH₂OMe)₂ | 2,4-Me₂-Ph | 103-105 |
| 443^{j} | C-Me | N(CH₂CH₂OMe)₂ | 2,4-Me₂-Ph | 87-89 |
| 444^{k} | C-Me | N(c-Pr)CH₂CH₂CN | 2,4-Me₂-Ph | 133(dec) |
| 445^{l} | C-Me | N(CH₂CH₂OMe)₂ | 2-Cl,4-MePh | 77-78 |
| 446^{m} | C-Me | NHCH(CH₂OMe)₂ | 2-Cl,4-MePh | 131-133 |
| 447ⁿ | C-Me | NHCH(Et)₂ | 2-Cl,4-MePh | 139-141 |
| 448^{o} | C-Me | NEt₂ | 2,4-Me₂-Ph | 92-94 |
| 449^{p} | C-Me | N(Pr)CH₂CH₂CN | 2,4-Me₂-Ph | 143-144 |
| 450^{q} | C-Me | N(Bu)CH₂CH₂CN | 2,4-Me₂-Ph | 115-117 |
| 451^{r} | C-Me | NHCH(Et)CH₂OMe | 2,4-Me₂-Ph | oil |
| 452^{s} | C-Me | NHCH(Et)₂ | 2-Me,4-MeOPh | 104-106 |
| 453^{t} | C-Me | NHCH(CH₂OMe)₂ | 2-Me,4-MeOPh | 115-116 |
| 954^{u} | C-Me | N(CH₂CH₂OMe)₂ | 2-Me,4-MeOPh | oil |
| 455^{v} | C-Me | (5)-NHCH(CH₂CH₂OMe)-(CH₂Ome) | 2-Me,4-MeOPh | oil |
| 456^{w} | C-Me | (S)-NHCH(CH₂CH₂OMe)-(CH₂OMe) | 2,4-Me₂-Ph | oil |
| 457^{x} | C-Me | N(CH₂CH₂OMe)₂ | 2-Me,4-ClPh | oil |
| 458^{y} | C-Me | NHEt | 2,4-Me₂-Ph | oil |
| 459^{z} | C-Me | NHCH(Et)₂ | 2-Me,4-ClPh | 94-96 |
| 460^{aa} | C-Me | NHCH(CH₂OMe)₂ | 2-Me,4-ClPh | 113-114 |
| 461^{ab} | C-Me | N(Ac)Et | 2,4-Me₂-Ph | oil |
| 462^{ac} | C-Me | (S)-NHCH(CH₂CH₂OMe)-(CH₂OMe) | 2-Me,4-ClPh | oil |
| 463^{ad} | C-Me | N(Pr)CH₂CH₂CN | 2-Me,4-MeOPh | 116-119 |
| 464^{ae} | C-Me | NEt₂ | 2-Me,4-MeOPh | 97-99 |
| 465^{af} | C-Me | (S)-NHCH(CH₂CH₂OMe)-(CH₂OMe) | 2-Cl,4-MePh | 101-103 |
| 466^{ag} | C-Me | NEt₂ | 2-Cl,4-MePh | 129-130 |
| 467^{ah} | C-Me | N(c-Pr)CH₂CH₂CN | 2-Me,4-MeOPh | 177-178 |
| 468^{ai} | C-Me | N(c-Pr)CH₂CH₂CN | 2-Cl,4-MePh | 162-163 |
| 469^{aj} | C-Me | NHCH(Et)CH₂OMe | 2-Me,4-MeOPh | oil |
| 470^{ak} | C-Me | NHCH(Et)CH₂OMe | 2-Cl,4-MePh | 111-113 |
| 471 | C-Me | NHCH(CH₂OMe)₂ | 2-Cl-4-MeOPh | |
| 472 | C-Me | N(CH₂CH₂OMe)₂ | 2-Cl-4-MeOPh | |
| 473 | C-Me | NHCH(Et)CH₂OMe | 2-Cl-4-MeOPh | |
| 474 | C-Me | N(c-Pr)CH₂CH₂CN | 2-Cl-4-MeOPh | |
| 475 | C-Me | NEt₂ | 2-Cl-4-MeOPh | |
| 476 | C-Me | NH-3-pentyl | 2-Cl-4-MeOPh | |
| 477 | C-Me | NHCH(Et)CH₂CH₂OMe | 2-Cl-4-MeOPh | |
| 478 | C-Me | NHCH(Me)CH₂CH₂OMe | 2-Cl-4-MeOPh | |
| 479 | C-Me | NHCH(Et)CH₂CH₂OMe | 2-Br-4-MeOPh | |
| 480 | C-Me | NHCH(Me)CH₂CH₂OMe | 2-Br-4-MeOPh | |
| 481 | C-Me | NHCH(Et)CH₂CH₂OMe | 2-Me-4-MeOPh | |
| 482 | C-Me | NHCH(Me)CH₂CH₂OMe | 2-me-4-MeOPh | |
| 483 | C-Me | NHCH(CH₂OMe)₂ | 2-Cl-4,5-(MeO)₂Ph | |
| 484 | C-Me | N(CH₂CH₂OMe)₂ | 2-Cl-4,5-(MeO)₂Ph | |
| 485 | C-Me | NHCH(Et)CH₂OMe | 2-Cl-4,5-(MeO)₂Ph | |
| 486 | C-Me | N(c-Pr)CH₂CH₂CN | 2-Cl-4,5-(MeO)₂Ph | |
| 487 | C-Me | NEt₂ | 2-Cl-4,5-(MeO)₂Ph | 99-101 |
| 488 | C-Me | NH-3-pentyl | 2-Cl-4,5-(MeO)₂Ph | 169-170 |
| 489 | C-Me | NHCH(Et)CH₂CH₂OMe | 2-Cl-4,5-(MeO)₂Ph | |
| 490 | C-Me | NHCH(Me)CH₂CH₂OMe | 2-Cl-4,5-(MeO)₂Ph | |
| 491 | C-Me | NHCH(CH₂OMe)₂ | 2-Br-4,5-(MeO)₂Ph | 90-93 |
| 492 | C-Me | N(CH₂CH₂OMe)₂ | 2-Br-4,5-(MeO)₂Ph | 110 |
| 493 | C-Me | NHCH(Et)CH₂OMe | 2-Br-4,5-(MeO)₂Ph | |
| 494 | C-Me | N(c-Pr)CH₂CH₂CN | 2-Br-4,5-(MeO)₂Ph | |
| 495 | C-Me | NEt₂ | 2-Br-4,5-(MeO)₂Ph | |
| 496 | C-Me | NH-3-pentyl | 2-Br-4,5-(MeO)₂Ph | |
| 497 | C-Me | NHCH(Et)CH₂CH₂OMe | 2-Br-4,5-(MeO)₂Ph | |
| 498 | C-Me | NHCH(Me)CH₂CH₂OMe | 2-Br-4,5-(MeO)₂Ph | |
| 499 | C-Me | NHCH(CH₂OMe)₂ | 2-Cl-4,6-(MeO)₂Ph | |
| 500 | C-Me | N(CH₂CH₂OMe)₂ | 2-Cl-4,6-(MeO)₂Ph | |
| 501 | C-Me | NHCH(Et)CH₂OMe | 2-Cl-4,6-(MeO)₂Ph | |
| 502 | C-Me | N(c-Pr)CH₂CH₂CN | 2-Cl-4,6-(MeO)₂Ph | |
| 503 | C-Me | NEt₂ | 2-Cl-4,6-(MeO)₂Ph | |
| 504 | C-Me | NH-3-pentyl | 2-Cl-4,6-(MeO)₂Ph | |
| 505 | C-Me | NHCH(Et)CH₂CH₂OMe | 2-Cl-4,6-(MeO)₂Ph | |
| 506 | C-Me | NHCH(Me)CH₂CH₂OMe | 2-Cl-4,6-(MeO)₂Ph | |
| 507 | C-Me | NHCH(CH₂OMe)₂ | 2-Me-4,6-(MeO)₂Ph | |
| 508 | C-Me | N(CH₂CH₂OMe)₂ | 2-Me-4,6-(MeO)₂Ph | |
| 509 | C-Me | NHCH(Et)CH₂OMe | 2-Me-4,6-(MeO)₂Ph | |
| 510 | C-Me | N(c-Pr)CH₂CH₂CN | 2-Me-4,6-(MeO)₂Ph | |
| 511 | C-Me | NEt₂ | 2-Me-4,6-(MeO)₂Ph | |
| 512 | C-Me | NH-3-pentyl | 2-Me-4,6-(MeO)₂Ph | |
| 513 | C-Me | NHCH(Et)CH₂CH₂OMe | 2-Me-4,6-(MeO)₂Ph | |
| 514 | C-Me | NHCH(Me)CH₂CH₂OMe | 2-Me-4,6-(MeO)₂Ph | |
| 515 | C-Me | N(c-Pr)CH₂CH₂CN | 2-Br-4,6-(MeO)₂Ph | |
| 516 | C-Me | NEt₂ | 2-Br-4,6-(MeO)₂Ph | |
| 517 | C-Me | NH-3-pentyl | 2-Br-4,6-(MeO)₂Ph | |
| 518 | C-Me | NHCH(Et)CH₂CH₂OMe | 2-Br-4,6-(MeO)₂Ph | |
| 519 | C-Me | NHCH(Me)CH₂CH₂OMe | 2-Br-4,6-(MeO)₂Ph | |
| 520 | C-Me | NHCH(Et)CH₂CH₂OMe | 2-Me-4-MeOPh | |
| 521 | C-Me | NHCH(Me)CH₂CH₂OMe | 2-Me-4-MeOPh | |
| 522 | C-Me | NHCH(CH₂OMe)₂ | 2-MeO-4-MePh | |
| 523 | C-Me | N(CH₂CH₂OMe)₂ | 2-MeO-4-MePh | |
| 524 | C-Me | NHCH(Et)CH₂OMe | 2-MeO-4-MePh | |
| 525 | C-Me | N(c-Pr)CH₂CH₂CN | 2-MeO-4-MePh | |
| 526 | C-Me | NEt₂ | 2-Me0-4-MePh | |
| 527 | C-Me | NH-3-pentyl | 2-Me0-4-MePh | |
| 528 | C-Me | NHCH(Et)CH₂CH₂OMe | 2-Me0-4-MePh | |
| 529 | C-Me | NHCH(Me)CH₂CH₂OMe | 2-Me0-4-MePh | |
| 530 | C-Me | NHCH(CH₂OMe)₂ | 2-Me0-4-MePh | |
| 531 | C-Me | N(CH₂CH₂OMe)₂ | 2-Me0-4-MePh | |
| 532 | C-Me | NHCH(Et)CH₂OMe | 2-Me0-4-MePh | |
| 533 | C-Me | N(c-Pr)CH₂CH₂CN | 2-Me0-4-MePh | |
| 534 | C-Me | NEt₂ | 2-Me0-4-MePh | |
| 535 | C-Me | NH-3-pentyl | 2-Me0-4-MePh | |
| 536 | C-Me | NHCH(Et)CH₂CH₂OMe | 2-Me0-4-MePh | |
| 537 | C-Me | NHCH(Me)CH₂CH₂OMe | 2-Me0-4-MePh | |
| 538 | C-Me | NHCH(CH₂OMe)₂ | 2-Me0-4-ClPh | |
| 539 | C-Me | N(CH₂CH₂OMe)₂ | 2-Me0-4-ClPh | |
| 540 | C-Me | NHCH(Et)CH₂OMe | 2-Me0-4-ClPh | |
| 541 | C-Me | N(c-Pr)CH₂CH₂CN | 2-Me0-4-ClPh | |
| 542 | C-Me | NEt₂ | 2-Me0-4-ClPh | |
| 543 | C-Me | NH-3-pentyl | 2-me0-4-ClPh | |
| 544 | C-Me | NHCH(Et)CH₂CH₂OMe | 2-Me0-4-ClPh | |
| 545 | C-Me | NHCH(Me)CH₂CH₂OMe | 2-Me0-4-ClPh | |
| NOTES FOR TABLE 2: | | | | |
| b) CI-HRMS: Calcd: 423.1355; Found: 423.1337 (M + H). | | | | |
| c) Analysis: Calcd: C, 61.38, H, 6.18, N, 14.32: Found: C, 61.54, H, 6.12, N, 14.37. | | | | |
| d) Analysis: Calcd: C: 58.02, H, 5.65, N, 14.24; Found: C, 58.11, H, 5.52, N, 14.26. | | | | |
| e) Analysis: Calcd: C, 59.71, H, 5.26, N, 14.85; Found: C, 59.94, H, 5.09, N, 17.23. | | | | |
| f) Analysis: Calcd: C, 60.48, H, 5.89, N, 14.85, Found: C, 60.62, H, 5.88, N, 14.82. | | | | |
| h) CI-HRMS: Calcd: 337.2388; Found: 337.2392 (M + H). | | | | |
| i) Analysis: Calcd: C, 68.45, H, 7.669, N, 15.21, Found: C, 68.35, H, 7.49 N, 14.91. | | | | |
| j) Analysis: Calcd: C, 69.08, H, 7.915, N, 14.65, Found: C, 68.85, H, 7.83, N, 14.54. | | | | |
| k) Analysis: Calcd: C, 73.51, H, 7.01, N, 19.48, Found: C, 71.57, H, 7.15, N, 19.12. | | | | |
| l) CI-HRMS: Calcd: 403.1899; Found: 403.1901 (M + H). | | | | |
| m) Analysis: Calcd: C, 61.77, H, 6.49, N, 14.41, Cl. 9.13; Found: C, 61.90, H, 6.66, N, 13.62, Cl, 9.25. | | | | |
| n) Analysis: Calcd: C, 67.31, H, 7.06, N, 15.70, Cl. 9.93; Found: C, 67.32, H, 6.95, N, 15.50, Cl, 9.93. | | | | |
| o) Analysis: Calcd: C, 74.50, H, 8.14, N, 17.38, Found: C, 74.43, H, 7.59, N, 17.16. | | | | |
| p) Analysis: Calcd: C, 73.10, H, 7.54, N, 19.37, Found: C, 73.18, H, 7.59, N, 18.81. | | | | |
| q) Analysis: Calcd: C, 73.57, H, 7.78, N, 18.65, Found: C, 73.55, H, 7.79, N, 18.64. | | | | |
| r) CI-HRMS: Calcd: 353.2333; Found: 353.2341 (M + H). | | | | |
| s) Analysis: Calcd: C, 71.56, H, 8.02, N, 15.90, Found: C, 71.45, H, 7.99, N, 15.88. | | | | |
| t) Analysis: Calcd: C, 65.60, H, 7.34, N, 14.57, Found: C, 65.42, H, 7.24, N, 14.37. | | | | |
| u) CI-HRMS: Calcd: 399.2398; Found: 399.2396 (M + H). | | | | |
| v) CI-HRMS: Calcd: 399.2398; Found: 399.2396 (M + H). | | | | |
| w) CI-HRMS: Calcd: 383.2450; Found: 383.2447 (M + H). | | | | |
| x) CI-HRMS: Calcd: 403.1887; Found: 403.1901 (M + H). | | | | |
| y) CI-HRMS: Calcd: 295.1919; Found.: 295.1923 (M + H). | | | | |
| z) Analysis: Calcd: C, 67.31, H, 7.06, N, 15.70, Found: C, 67.12, H, 6.86, N, 15.53. | | | | |
| aa) Analysis: Calcd: C, 61.77, H, 6.49, N, 14.41, Cl, 9.13; Found: C, 62.06, H, 6.37, N, 14.25, Cl, 9.12. | | | | |
| ab) CI-HRMS: Calcd: 337.2017; Found: 337.2028 (M + H). | | | | |
| ac) CI-HRMS: Calcd: 403.1893; Found: 403.1901 (M + H). | | | | |
| ad) Analysis: Calcd: C, 70.00, H, 7.22, N, 18.55, Found: C, 70.05, H, 7.22, N, 18.36. | | | | |
| ae) Analysis: Calcd: C, 70.98, H, 7.74, N, 16.55, Found: C, 71.15, H,7.46, N, 16.56. | | | | |
| ag) Analysis: Calcd: C, 66.59, H, 6.76, N, 16.34, Found: C, 66.69, H,6.82, N, 16.20. | | | | |
| ah) Analysis: Calcd: C, 70.38, H, 6.71, N, 18.65, Found: C, 70.35, H, 6.82, N, 18.83. | | | | |
| ai) Analysis: Calcd: C, 66.39, H, 5.85, N, 18.44, Cl, 9.33; Found: C, 66.29, H, 5.51, N, 18.36, Cl, 9.31. | | | | |
| aj) CI-HRMS: Calcd: 369.2278; Found: 369.2291 (M + H). | | | | |
| ak) Analysis: Calcd: C, 64.42, H, 6.77, N, 15.02, Found: C, 64.59, H, 6.51, N, 14.81. | | | | |

The examples delineated in TABLE 3 may be prepared by the methods outlined in Examples 1, 2, 3 or 6. Commonly used abbreviations are: Ph is phenyl, Pr is propyl, Me is methyl, Et is ethyl, Bu is butyl, Ex is Example.

**TABLE 3**

| | | | | |
|---|---|---|---|---|
| 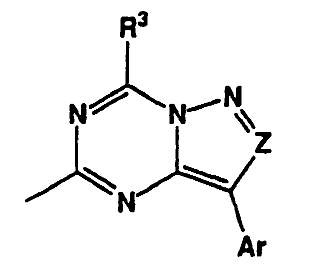 | | | | |

| Ex. | Z | R₃ | Ar | mp(°C) |
|---|---|---|---|---|
| 546^{a} | C-Me | NHCH(Et)₂ | 2-Me-4-Me₂N-Ph | 164-166 |
| 547^{b} | c-Me | S-NHCH(CH₂CH₂OMe) -CH₂OMe | 2,4-Me2-Ph | oil |
| 548^{c} | C-Me | S-NHCH(CH₂CH₂OMe) -CH₂OMe | 2-Me-4-Cl-Ph | oil |
| 549^{d} | C-We | N(c-Pr)CH₂CH₂CN | 2-Me-4-Cl-Ph | 115-116 |
| 550^{e} | C-Me | NHCH(Et)CH₂CN | 2-Me-4-Cl-Ph | 131-132 |
| 551^{f} | C-Me | N(Et)₂ | 2,3-Me₂-4-OMe-Ph | oil |
| 552^{g} | C-Me | N(CH₂CH₂OMe)CH₂CH₂OH | 2.4-Cl₂-Ph | oil |
| 553^{h} | C-Me | N(CH₂CH₂OMe)₂ | 2,3-Me₂-4-OMe-Ph | oil |
| 554ⁱ | C-Me | NHCH(Et)₂ | 2,3-Me₂-4-OMePh | 123-124 |
| 555^{j} | C-Me | N(CH₂-c-Pr)Pr | 2-Me-4-Cl-Ph | oil |
| 556^{k} | C-Me | N(c-Pr)CH₂CH₂CN | 2,3-Me₂-4-OMePh | 158-160 |
| 557 | C-Me | N(c-Pr)Et | 2-Cl-4-OMePh | |
| 558 | C-Me | N(c-Pr)Me | 2-Cl-4-OMePh | |
| 559 | C-Me | N(c-Pr)Pr | 2-Cl-4-OMePh | |
| 560 | C-Me | N(c-Pr)Bu | 2-Cl-4-OMePh | |
| 561^{l} | C-Me | N(Et)₂ | 2-Cl-4-CN-Ph | 115-117 |
| 562 | C-Me | N(c-Pr)₂ | 2-Cl-4-OMe | 127-129 |
| 563^{m} | C-Me | NHCH(CH₂OH)₂ | 2,4-Cl₂-Ph | 128-129 |
| 564 | C-Me | N(c-Pr)Et | 2-Br-4,5-(MeO)2Ph | |
| 565 | C-Me | N(c-Pr)Me | 2-Br-4,5-(MeO)2Ph | |
| 566 | C-Me | NH-c-Pr | 2-Me-4-MeOPh | 126-128 |
| 567 | C-Me | NHCH(Et)CH2OH | 2-Me-4-MeOPh | 60-62 |
| 568 | C-Me | NMe₂ | 2-Br-4,5-(MeO)2Ph | |
| 569 | C-Me | NHCH(Et)₂ | 2-Me-4-MeOPh | 103-105 |
| 570 | C-Me | N(c-Pr)Et | 2-Me-4-MeOPh | 173-174 |
| 571 | C-Me | NH-2-pentyl | 2,4-Cl₂-Ph | 118-120 |
| 572 | C-Me | NHCH(Et)CH2CN | 2,4-Cl₂-Ph | 141-142 |
| 573 | C-Me | NHCH(Pr)CH2OMe | 2,4-Cl₂-Ph | 87-88 |
| 574 | C-Me | NHCH(CH2-iPr)CH2OMe | 2,4-Cl₂-Ph | amorphous |
| 575 | C-Me | NH-2-butyl | 2,4-Me₂-Ph | oil |
| 576 | C-Me | NH-2-pentyl | 2,4-Me₂-Ph | oil |
| 577 | C-Me | NH-2-hexyl | 2,4-Me₂-Ph | oil |
| 578 | C-Me | NHCH(i-Pr)Me | 2,4-Me₂-Ph | oil |
| 579 | C-Me | NHCH(Me)CH2-iPr | 2,4-Me₂-Ph | oil |
| 580 | C-Me | NHCH(Me)-c-C6H11 | 2,4-Me₂-Ph | oil |
| 581 | C-Me | NH-2-indanyl | 2,4-Me₂-Ph | oil |
| 582 | C-Me | NH-1-indanyl | 2,4-Me₂-Ph | oil |
| 583 | C-Me | NHCH(Me)Ph | 2,4-Me₂-Ph | oil |
| 584 | C-Me | NHCH(Me)CH₂-(4-ClPh) | 2,4-Me₂-Ph | oil |
| 585 | C-Me | NHCH(Me)CH₂COCH₃ | 2,4-Me₂-Ph | oil |
| 586 | c-Me | NHCH(Ph)CH₂Ph | 2,4-Me₂-Ph | oil |
| 587 | C-Me | NHCH(Me)(CH₂)3NEt₂ | 2,4-Me₂-Ph | oil |
| 588 | C-Me | NH-(2-Ph-c-C₃H₄) | 2,4-Me₂-Ph | oil |
| 589 | C-Me | NHCH(Et)CH₂CN | 2,4-Me₂-Ph | 119-120 |
| 590 | C-Me | NH-3-hexyl | 2,4-Me₂-Ph | oil |
| 591ⁿ | C-Me | NEt₂ | 2-MeO-4-ClPh | oil |
| 592^{o} | C-Me | NHCH(Et)₂ | 2-MeO-4-ClPh | oil |
| 593^{p} | C-Me | NHCH(Et)CH₂OMe | 2-MeO-4-ClPh | oil |
| 594 | C-Me | NMe₂ | 2-MeO-4-ClPh | oil |
| 595^{q} | C-Me | NHCH(Et)₂ | 2-OMe-4-MePh | oil |
| 596^{r} | C-Me | NEt₂ | 2-OMe-4-MePh | oil |
| 597^{s} | C-c-Pr | NHCH(CH₂OMe)₂ | 2,4-Cl₂-Ph | oil |
| 598 | C-Me | N(c-Pr)Et | 2,4-Me₂-Ph | |
| 599 | C-Me | N(c-Pr)Et | 2,4-Cl₂-Ph | |
| 600 | C-Me | N(c-Pr)Et | 2,4,6-Me₃-Ph | |
| 601 | C-Me | N(c-Pr)Et | 2-Me-4-Cl-Ph | |
| 602 | C-Me | M(c-Pr)Et | 2-Cl-4-Me-Ph | |
| 603 | C-Me | NHCH(c-Pr)₂ | 2,4-Cl₂-Ph | |
| 604 | C-Me | NHCH(c-Pr)₂ | 2,4-Me₂-Ph | |
| 605 | C-Me | NHCH(c-Pr)₂ | 2-Me-4-Cl-Ph | |
| 606 | C-Me | NHCH(c-Pr)₂ | 2-Cl-4-Me-Ph | |
| 607 | C-Me | NHCH(c-Pr)₂ | 2-Me-4-OMe-Ph | |
| 608 | C-Me | NHCH(c-Pr)₂ | 2-Cl-4-OMe-Ph | |
| 609 | C-He | NHCH(CH₂OMe)₂ | 2-Cl-5-F-OMePh | |
| 610 | C-Me | NEt₂ | 2-Cl-5-F-OMePh | |
| 611 | C-Me | N(c-Pr)CH₂CH₂CN | 2-Cl-5-F-OMePh | |
| 612 | C-Me | NHCH(Et)₂ | 2-Cl-5-F-OMePh | |
| 613 | C-Me | N(CH₂CH₂OMe)₂ | 2-Cl-5-F-OMePh | |
| 614 | C-Me | NEt₂ | 2,6-Me₂-pyrid-3-yl | |
| 615 | C-Me | N(c-Pr)CH₂CH₂CN | 2,6-Me₂-pyrid-3-yl | |
| 616 | C-Me | NHCH(Et)₂ | 2,6-Me₂-pyrid-3-yl | |
| 617 | C-Me | N(CH₂CH₂OMe)₂ | 2,6-Me₂-pyrid-3-yl | |
| 618 | C-OH | NHCH(CH₂OMe)₂ | 2,4-Me₂-Ph | |
| 619 | C-OH | NEt₂ | 2,4-Me₂-Ph | |
| 620 | C-OH | N(c-Pr)CH₂CH₂CN | 2,4-Me₂-Ph | |
| 621 | C-OH | NHCH(Et)₂ | 2,4-Me₂-Ph | |
| 623 | C-OH | N(CH₂CH₂OMe)₂ | 2,4-Me₂-Ph | |
| 624 | C-NEt₂ | NHCH(CH₂OMe)₂ | 2,4-Me₂-Ph | |
| 625 | C-NEt₂ | NEt₂ | 2,4-Me₂-Ph | |
| 626 | C-NEt₂ | N(c-Pr)CH₂CH₂CN | 2,4-Me₂-Ph | |
| 627 | C-NEt₂ | NHCH(Et)₂ | 2,4-Me₂-Ph | |
| 628 | C-NEt₂ | N(CH₂CH₂OMe)₂ | 2,4-Me₂-Ph | |
| 629 | C-Me | NHCH(Et)₂ | 2-Me-4-CN-Ph | |
| 630 | C-Me | N(CH₂CH₂OMe)₂ | 2-Me-4-CN-Ph | |
| Notes for Table 3: | | | | |
| a) CI-HRMS: Calcd:367-2610, Found: 367.2607 (M + H); | | | | |
| b) CI-HRMS: Calcd:384.2400, Found: 384.2393 (M + H); | | | | |
| c) CI-HRMS : Calcd:404.1853, Found: 404.1844 (M + H); | | | | |
| d) CI-HRMS: Calcd:381.1594, Found: 381.1596 (M + H); Analysis: Calcd: C: 63.07, H, 5.57, N, 22.07, Cl, 9.32; Found: C: 63.40, H, 5.55, N, 21.96, Cl: 9.15 | | | | |
| e) CI-HRMS: Calcd:369.1594, Found: 369.1576 (M + H); | | | | |
| f) CI-HRMS: Calcd:354.2216, Found: 354.2211 (M + H); | | | | |
| g) CI-HRMS: Calcd:410.1072, Found: 410.1075 (M + H); | | | | |
| h) CI-HRMS: Calcd:414.2427, Found: 414.2427(M + H); | | | | |
| i) CI-HRMS: Calcd:368.2372, Found: 368.2372(M + H); | | | | |
| j) CI-HRMS: Calcd:384.1955, Found: 384.1947(M + H); | | | | |
| k) CI-HRMS: Calcd:391.2168, Found: 391.2160(M + H); | | | | |
| l) CI-HRMS: Calcd:335.1984, Found: 335.1961(M + H); | | | | |
| m) CI-HRMS: Calcd:382.0759, Found: 382.0765(M + H); | | | | |
| n) NH₃-CI MS: Calcd: 360, Found: 360 (M + H)+ | | | | |
| o) NH₃-CI MS: Calcd: 374, Found: 374 (M + H)+; NMR (CDCl₃, 300 MHz) : δ 7.29 (d, J=8.4Hz, 1H), 7.04 (dd, J=1.8,8Hz, 1H), 6.96 (d, J=1.8Hz, 1H), 6.15 (d, J=10, 1H), 4.19 (m, 1H), 3.81 (s, 3H), 2.47 (s, 3H), 2.32 (s, 3H), 1.65 (m, 4H), 0.99 (t, J=7.32Hz, 6H) | | | | |
| p) NH₃-CI MS: Calcd: 390, Found: 390 (M + H)+; NMR (CDCl₃, 300 MHz) : δ 7.28 (d, J=8Hz, 1H), 7.03 (d, J=8Hz, 1H), 6.96 (s, 1H), 6.52 (d, J=9Hz, 1H), 4.36 (m, 1H), 3.8 (s, 3H), 3.55 (m, 2H), 3.39 (s, 3H), 2.47 (s, 3H), 2.32 (s, 3H), 1.76 (m, 2H), 1.01 (t, J=7.32Hz, 3H). | | | | |
| q) CI-HRMS: Calcd: 354.2294, Found: 354.2279 (M + H)+ | | | | |
| r) CI-HRMS: Calcd: 340.2137, Found: 340.2138 (M + H)+ | | | | |
| s) CI-HRMS: Calcd: 436.1307, Found: 436.1296 (M + H)+ | | | | |

### Utility

### CRF-R1 Receptor Binding Assay for the Evaluation of Biological Activity

The following is a description of the isolation of cell membranes containing cloned human CRF-R1 receptors for use in the standard binding assay as well as a description of the assay itself.

Messenger RNA was isolated from human hippocampus. The mRNA was reverse transcribed using oligo (dt) 12-18 and the coding region was amplified by PCR from start to stop codons The resulting PCR fragment was cloned into the EcoRV site of pGEMV, from whence the insert was reclaimed using XhoI + XbaI and cloned into the XhoI + XbaI sites of vector pm3ar (which contains a CMV promoter, the SV40 't' splice and early poly A signals, an Epstein-Barr viral origin of replication, and a hygromycin selectable marker). The resulting expression vector, called phchCRFR was transfected in 293EBNA cells and cells retaining the episome were selected in the presence of 400 µM hygromycin. Cells surviving 4 weeks of selection in hygromycin were pooled, adapted to growth in suspension and used to generate membranes for the binding assay described below. Individual aliquots containing approximately 1 x 10⁸ of the suspended cells were then centrifuged to form a pellet and frozen.

For the binding assay a frozen pellet described above containing 293EBNA cells transfected with hCRFR1 receptors is homogenized in 10 ml of ice cold tissue buffer (50 mM HEPES buffer pH 7.0, containing 10 mM MgCl₂, 2 mM EGTA, 1 µg/l aprotinin, 1 µg/ml leupeptin and 1 µg/ml pepstatin). The homogenate is centrifuged at 40,000 x g for 12 min and the resulting pellet rehomogenized in 10 ml of tissue buffer. After another centrifugation at 40,000 x g for 12 min, the pellet is resuspended to a protein concentration of 360 µg/ml to be used in the assay.

Binding assays are performed in 96 well plates; each well having a 300 µl capacity. To each well is added 50 µl of test drug dilutions (final concentration of drugs range from 10-¹⁰ - 10-⁵ M), 100 µl of ¹²⁵I-ovine-CRF (¹²⁵I-o-CRF) (final concentration 150 pM) and 150 µl of the cell homogenate described above. Plates are then allowed to incubate at room temperature for 2 hours before filtering the incubate over GF/F filters (presoaked with 0.3% polyethyleneimine) using an appropriate cell harvester. Filters are rinsed 2 times with ice cold assay buffer before removing individual filters and assessing them for radioactivity on a gamma counter.

Curves of the inhibition of ¹²⁵I-o-CRF binding to cell membranes at various dilutions of test drug are analyzed by the iterative curve fitting program LIGAND [P.J. Munson and D. Rodbard, *Anal. Biochem.* 107:220 (**1980**), which provides Ki values for inhibition which are then used to assess biological activity.

A compound is considered to be active if it has a Kᵢ value of less than about 10000 nM for the inhibition of CRF.

### Inhibition of CRF-Stimulated Adenylate Cyclase Activity

Inhibition of CRF-stimulated.adenylate cyclase activity can be performed as described by G. Battaglia et al. *Synapse* 1:572 (**1987**). Briefly, assays are carried out at 37° C for 10 min in 200 ml of buffer containing 100 mM Tris-HCl (pH 7.4 at 37° C), 10 mM MgCl₂, 0.4 mM EGTA, 0.1% BSA, 1 mM isobutylmethylxanthine (IBMX), 250 units/ml phosphocreatine kinase, 5 mM creatine phosphate, 100 mM guanosine 5'-triphosphate, 100 nM oCRF, antagonist peptides (concentration range 10⁻⁹ to 10^{-6m}) and 0.8 mg original wet weight tissue (approximately 40-60 mg protein). Reactions are initiated by the addition of 1 mM ATP/³²P]ATP (approximately 2-4 mCi/tube) and terminated by the addition of 100 ml of 50 mM Tris-HCL, 45 mM ATP and 2% sodium dodecyl sulfate. In order to monitor the recovery of cAMP, 1 µl of [³H]cAMP (approximately 40,000 dpm) is added to each tube prior to separation. The separation of [³²P]cAMP from [³²P]ATP is performed by sequential elution over Dowex and alumina columns.

### In vivo Biological Assay

The *in vivo* activity of the compounds of the present invention can be assessed using any one of the biological assays available and accepted within the art. Illustrative of these tests include the Acoustic Startle Assay, the Stair Climbing Test, and the Chronic Administration Assay. These and other models useful for the testing of compounds of the present invention have been outlined in C.W. Berridge and A.J. Dunn *Brain Research Reviews* 15:71 (**1990**). Compounds may be tested in any species of rodent or small mammal.

Compounds of this invention have utility in the treatment of inbalances associated with abnormal levels of corticotropin releasing factor in patients suffering from depression, affective disorders, and/or anxiety.

Compounds of this invention can be administered to treat these abnormalities by means that produce contact of the active agent with the agent's site of action in the body of a mammal. The compounds can be administered by any conventional means available for use in conjunction with pharmaceuticals either as individual therapeutic agent or in combination of therapeutic agents. They can be administered alone, but will generally be administered with a pharmaceutical carrier selected on the basis of the chosen route of administration and standard pharmaceutical practice.

The dosage administered will vary depending on the use and known factors such as pharmacodynamic character of the particular agent, and its mode and route of administration; the recipient's age, weight, and health; nature and extent of symptoms; kind of concurrent treatment; frequency of treatment; and desired effect. For use in the treatment of said diseases or conditions, the compounds of this invention can be orally administered daily at a dosage of the active ingredient of 0.002 to 200 mg/kg of body weight. Ordinarily, a dose of 0.01 to 10 mg/kg in divided doses one to four times a day, or in sustained release formulation will be effective in obtaining the desired pharmacological effect.

Dosage forms (compositions) suitable for administration contain from about 1 mg to about 100 mg of active ingredient per unit. In these pharmaceutical compositions, the active ingredient will ordinarily be present in an amount of about 0.5 to 95% by weight based on the total weight of the composition.

The active ingredient can be administered orally is solid dosage forms, such as capsules, tablets and powders; or in liquid forms such as elixirs, syrups, and/or suspensions. The compounds of this invention can also be administered parenterally in sterile liquid dose formulations.

Gelatin capsules can be used to contain the active ingredient and a suitable carrier such as but not limited to lactose, starch, magnesium stearate, steric acid, or cellulose derivatives. Similar diluents can be used to make compressed tablets. Both tablets and capsules can be manufactured as sustained release products to provide for continuous release of medication over a period of time. Compressed tablets can be sugar-coated or film-coated to mask any unpleasant taste, or used to protect the active ingredients from the atmosphere, or to allow selective disintegration of the tablet in the gastrointestinal tract.

Liquid dose forms for oral administration can contain coloring or flavoring agents to increase patient acceptance.

In general, water, pharmaceutically acceptable oils, saline, aqueous dextrose (glucose), and related sugar solutions and glycols, such as propylene glycol or polyethylene glycol, are suitable carriers for parenteral solutions. Solutions for parenteral administration preferably contain a water soluble salt of the active ingredient, suitable stabilizing agents, and if necessary, butter substances. Antioxidizing agents, such as sodium bisulfite, sodium sulfite, or ascorbic acid, either alone or in combination, are suitable stabilizing agents. Also used are citric acid and its salts, and EDTA. In addition, parenteral solutions can contain preservatives such as benzalkonium chloride, methyl- or propyl-paraben, and chlorobutanol.

Suitable pharmaceutical carriers are described in "Remington's Pharmaceutical Sciences", A. Osol, a standard reference in the field.

Useful pharmaceutical dosage-forms for administration of the compounds of this invention can be illustrated as follows:

### Capsules

A large number of units capsules are prepared by filling standard two-piece hard gelatin capsules each with 100 mg of powdered active ingredient, 150 mg lactose, 50 mg cellulose, and 6 mg magnesium stearate.

### Soft Gelatin Capsules

A mixture of active ingredient in a digestible oil such as soybean, cottonseed oil, or olive oil is prepared and injected by means of a positive displacement was pumped into gelatin to form soft gelatin capsules containing 100 mg of the active-ingredient. The capsules were washed and dried.

### Tablets

A large number of tablets are prepared by conventional procedures so that the dosage unit was 100 mg active ingredient, 0.2 mg of colloidal silicon dioxide, 5 mg of magnesium stearate, 275 mg of microcrystalline cellulose, 11 mg of starch, and 98.8 mg lactose. Appropriate coatings may be applied to increase palatability or delayed adsorption.

The compounds of this invention may also be used as reagents or standards in the biochemical study of neurological function, dysfunction, and disease.

Although the present invention has been described and exemplified in terms of certain preferred embodiments, other embodiments will be apparent to those skilled in the art. The invention is, therefore, not limited to the particular embodiments described and exemplified, but is capable of modification or variation without departing from the spirit of the invention, the full scope of which is delineated by the appended claims.

## Claims

1. [1,5-a]-Pyrazolo-1,3,5-triazines of the Formula (1) 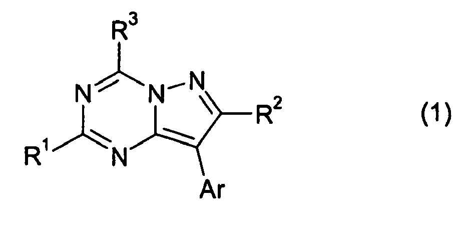 wherein
Ar is phenyl, pyridyl or 2,3-dihydrobenzofuranyl, each (Ar) optionally substituted with 1 to 4 R⁴ substituents;
R¹ is independently selected at each occurrence from H, C₁-C₄ alkyl, C₂-C₄ alkenyl, C₂-C₄ alkynyl, halo, CN, C₁-C₄ haloalkyl, C₁-C₁₂ hydroxyalkyl, C₂-C₁₂ alkoxyalkyl, C₂-C₁₀ cyanoalkyl, C₃-C₆ cycloalkyl, C₄-C₁₀ cycloalkylalkyl, NR⁹R¹⁰, C₁-C₄ alkyl-NR⁹R¹⁰, NR⁹COR¹⁰, OR¹¹, SH or S(O)ₙR¹²;
R² is selected from H, C₁-C₄ alkyl, C₂-C₄ alkenyl, C₂-C₄ alkynyl, C₃-C₆ cycloalkyl, C₄-C₁₀ cycloalkylalkyl, C₁-C₄ hydroxyalkyl, halo, CN, -NR⁶R⁷, NR⁹COR¹⁰, -NR⁶S(O)ₙR⁷, S(O)ₙNR⁶R⁷, C₁-C₄ haloalkyl, -OR⁷, SH or -S(O)ₙR¹²;
R³ is NR^{6a}R^{7a};
R⁴ is independently selected at each occurrence from: C₁-C₁₀ alkyl, C₂-C₁₀ alkenyl, C₂-C₁₀ alkynyl, C₃-C₆ cycloalkyl, C₄-C₁₂ cycloalkylalkyl, NO₂, halo, CN, C₁-C₄ haloalkyl, NR⁶R⁷, NR⁸COR⁷, NR⁸CO₂R⁷, COR⁷, OR⁷, CONR⁶R⁷, CO(NOR⁹)R⁷, CO₂R⁷, or S(O)ₙR⁷, where each such C₁-C₁₀ alkyl, C₂-C₁₀ alkenyl, C₂-C₁₀ alkynyl, C₃-C₆ cycloalkyl and C₄-C₁₂ cycloalkylalkyl are optionally substituted with 1 to 3 substituents independently selected at each occurrence from C₁-C₄ alkyl, NO₂, halo, CN, NR⁶R⁷, NR⁸COR⁷, NR⁸CO₂R⁷, COR⁷ OR⁷, CONR⁶R⁷, CO₂R⁷, CO(NOR⁹)R⁷, or S(O)ₙR⁷;
R⁶ and R⁷, R^{6a} and R^{7a} are independently selected at each occurrence from:
• H,
• C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)_{R}¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
• aryl, aryl(C₁-C₄ alkyl), heteroaryl, heteroaryl(C₁-C₄ alkyl), heterocyclyl or heterocyclyl(C₁-C₄ alkyl);
alternatively, NR⁶R⁷ and NR^{6a}R^{7a} are independently piperidine, pyrrolidine, piperazine, N-methylpiperazine, morpholine or thiomorpholine, each optionally substituted with 1 to 3 C₁-C₄ alkyl groups;
R⁸ is independently selected at each occurrence from H or C₁-C₄ alkyl;
R⁹ and R¹⁰ are independently selected at each occurrence from H, C₁-C₄ alkyl, or C₃-C₆ cycloalkyl;
R¹¹ is selected from H, C₁-C₄ alkyl, C₁-C₄ haloalkyl, or C₃-C₆ cycloalkyl;
R¹² is C₁-C₄ alkyl or C₁-C₄ haloalkyl;
R¹³ is selected from C₁-C₄ alkyl, C₁-C₄ haloalkyl, C₂-C₈ alkoxyalkyl, C₃-C₆ cycloalkyl, C₄-C₁₂ cycloalkylalkyl, aryl, aryl(C₁-C₄ alkyl)-, heteroaryl or heteroaryl(C₁-C₄ alkyl)-;
R¹⁴ is selected from C₁-C₁₀ alkyl,C₃-C₁₀ alkenyl, C₃-C₁₀ alkynyl, C₃-C₈ cycloalkyl, or C₄-C₁₂ cycloalkylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, CONR¹⁶R¹⁵, and C₁-C₆ alkylthio, C₁-C₆ alkylsulfinyl and C₁-C₆ alkylsulfonyl;
R¹⁵ and R¹⁶ are independently selected at each occurrence from H, C₁-C₆ alkyl, C₃-C₁₀ cycloalkyl, C₄-C₁₆ cycloalkylalkyl, except that for S(O)ₙR¹⁵, R¹⁵ cannot be H;
aryl is phenyl or naphthyl, each optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, and CONR¹⁶R¹⁵;
heteroaryl is pyridyl, pyrimidinyl, triazinyl, furanyl, pyranyl, quinolinyl, isoquinolinyl, thienyl, imidazolyl, thiazolyl, indolyl, pyrrolyl, oxazolyl, benzofuranyl, benzothienyl, benzothiazolyl, isoxazolyl, pyrazolyl, 2,3 dihydrobenzothienyl or 2,3-dihydrobenzofuranyl, each being optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, -COR¹⁵, CO₂R¹⁵, CO(O)R¹⁵, NR⁸COR¹⁵, N(COR¹⁵)₂, NR⁸CONR¹⁶R¹⁵, NR⁸CO₂R¹⁵, NR¹⁶R¹⁵ and CONR¹⁶R¹⁵;
heterocyclyl is saturated or partially saturated heteroaryl, optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁵R¹⁶, and CONR¹⁶R¹⁵;
n is independently at each occurrence 0, 1 or 2,
with the proviso that:
(1) when R¹ is methyl or ethyl, R² is H, and R³ is NH(n-C₄H₉) or N(C₂H₅)₂, then Ar is not unsubstituted phenyl or m-methylphenyl; and
(2) when R² is H, and Ar is pyridyl, and R³ is NR^{6a}R^{7a}, then R^{6a} and R^{7a} are not H or alkyl,
and geometric isomers thereof, stereoisomeric forms thereof, or mixtures of stereoisomeric forms thereof, and pharmaceutically acceptable salt or pro-drug forms thereof.

2. The [1,5-a]-pyrazolo-1,3,5-triazines according to claim 1, wherein
Ar is phenyl, pyridyl or 2,3-dihydrobenzofuranyl, each (Ar) substituted with 1 to 4 R⁴ substituents,
and geometric isomers thereof, stereoisomeric forms thereof, or mixtures of stereoisomeric forms thereof, and pharmaceutically acceptable salt or pro-drug forms thereof.

3. The [1,5-a]-pyrazolo-1,3,5-triazines according to claim 1 or 2, wherein
Ar is phenyl or pyridyl, each (Ar) optionally substituted with 1 to 4 R⁴ substituents,
and geometric isomers thereof, stereoisomeric forms thereof, or mixtures of stereoisomeric forms thereof, and pharmaceutically acceptable salt or pro-drug forms thereof.

4. The [1,5-a]-pyrazolo-1,3,5-triazines according to any one of claims 1 to 3, wherein
R¹ and R² are independently selected from H, C₁₋₄ alkyl, C₃-C₆ cycloalkyl, C₄-C₁₀ cycloalkylalkyl,
and geometric isomers thereof, stereoisomeric forms thereof, or mixtures of stereoisomeric forms thereof, and pharmaceutically acceptable salt or pro-drug forms thereof.

5. The [1,5-a]-pyrazolo-1,3,5-triazines according to claim 4, wherein
R¹ and R² are independently selected from H or C₁₋₄ alkyl,
and geometric isomers thereof, stereoisomeric forms thereof, or mixtures of stereoisomeric forms thereof, and pharmaceutically acceptable salt or pro-drug forms thereof.

6. The [1,5-a]-pyrazolo-1,3,5-triazines according to claim 4, wherein
R¹ and R² are independently selected from C₁₋₄ alkyl,
and geometric isomers thereof, stereoisomeric forms thereof, or mixtures of stereoisomeric forms thereof, and pharmaceutically acceptable salt or pro-drug forms thereof.

7. The [1,5-a]-pyrazolo-1,3,5-triazines according to claim 4, wherein
R¹ is methyl,
and geometric isomers thereof, stereoisomeric forms thereof, or mixtures of stereoisomeric forms thereof, and pharmaceutically acceptable salt or pro-drug forms thereof.

8. The [1,5-a]-pyrazolo-1,3,5-triazines according to any one of claims 1 to 7, wherein
R^{6a} is independently selected from:
• H,
• C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₈ alkyl, C₃-C₆ cycloalkyl, halo, C₁₋C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
• aryl, aryl(C₁-C₄ alkyl)-, heteroaryl, heteroaryl(C₁-C₄ alkyl)-, heterocyclyl or heterocyclyl(C₁-C₄ alkyl)-; and
R^{7a} is independently selected at each occurrence from:
• H,
• C₅-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
• aryl, aryl(C₁-C₄ alkyl)-, heteroaryl, heteroaryl(C₁-C₄ alkyl)-, heterocyclyl or heterocyclyl(C₁-C₄ alkyl)-;
alternatively, NR⁶R⁷ and NR^{6a}R^{7a} are independently piperidine, pyrrolidine, piperazine, N-methylpiperazine, morpholine or thiomorpholine, each optionally substituted with 1 to 3 C₁-C₄ alkyl groups,
and geometric isomers thereof, stereoisomeric forms thereof, or mixtures of stereoisomeric forms thereof, and pharmaceutically acceptable salt or pro-drug forms thereof.

9. The [1,5-a]-pyrazolo-1,3,5-triazines according to any one of claims 1 to 7, wherein
R^{6a} is selected from:
• H,
• C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
• aryl, aryl(C₁-C₄ alkyl)-, heteroaryl, heteroaryl(C₁-C₄ alkyl)-, heterocyclyl or heterocyclyl(C₁-C₄ alkyl)-;
R^{7a} is selected from:
• C₁-C₄ alkyl and each such C₁-C₄ alkyl is substituted with 1-3 substituents independently selected at each occurrence from C₁-C₆ alkyl, C₃-C₆ cycloalkyl, halo,C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵,CONR¹⁶R¹⁵, aryl, heteroaryl or heterocyclyl,
and geometric isomers thereof, stereoisomeric forms thereof, or mixtures of stereoisomeric forms thereof, and pharmaceutically acceptable salt or pro-drug forms thereof.

10. The [1,5-a]-pyrazolo-1,3,5-triazines according to any one of claims 1 to 7, wherein
R^{6a} is H,
R^{7a} is selected from:
• C₁-C₄ alkyl and each such C₁-C₄ alkyl is substituted with 1-3 substituents independently selected at each occurrence from C₁-C₆ alkyl, C₃-C₆ cycloalkyl, halo,C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵,CONR¹⁶R¹⁵, aryl, heteroaryl or heterocyclyl,
and geometric isomers thereof, stereoisomeric forms thereof, or mixtures of stereoisomeric forms thereof, and pharmaceutically acceptable salt or pro-drug forms thereof.

11. The [1,5-a]-pyrazolo-1,3,5-triazines according to any one of claims 1 to 7, wherein
R^{6a} and R^{7a} are independently H or C₁-C₁₀ alkyl, each such C₁-C₁₀ alkyl optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁶COR¹⁵, N(COR¹⁵)₂, R⁸CONR¹⁶R¹⁵, NR⁸CO₂R¹³ NR¹⁶R¹⁵, CONR¹⁶R¹⁵, aryl, heteroaryl or heterocyclyl,
and geometric isomers thereof, stereoisomeric forms thereof, or mixtures of stereoisomeric forms thereof, and pharmaceutically acceptable salt or pro-drug forms thereof.

12. The [1,5-a]-pyrazolo-1,3,5-triazines according to any one of claims 1 to 7, wherein
R^{6a} and R^{7a} are independently H or C₁-C₁₀ alkyl,
and geometric isomers thereof, stereoisomeric forms thereof, or mixtures of stereoisomeric forms thereof, and pharmaceutically acceptable salt or pro-drug forms thereof.

13. The [1,5-a]-pyrazolo-1,3,5-triazines according to claim 1 of the formula (50) 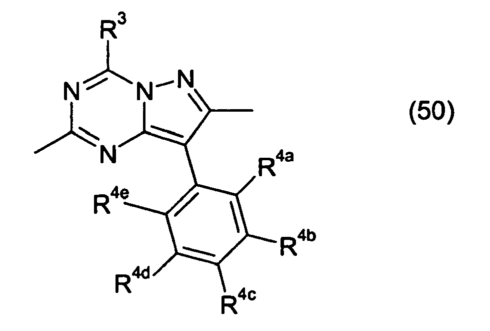 wherein the compound is selected from the group consisting of:
• a compound of Formula (50) wherein R³ is -NHCH(n-Pr)₂, R^{4a} is Cl, R^{4b} is H, R^{4c} is Cl, R^{4d} is H and R^{4e} is H;
• a compound of Formula (50) wherein R³ is -N(Et)(n-Bu), R^{4a} is Cl, R^{4b} is H, R^{4c} is Cl, R^{4d} is H and R^{4e} is H;
• a compound of Formula (50) wherein R³ is -N(CH₂CH₂OMe)₂, R^{4a} is Cl, R^{4b} is H, R^{4c} is Cl, R^{4d} is H and R^{4e} is H;
• a compound of Formula (50) wherein R³ is -NHCH(Et)(n-Bu), R^{4a} is Cl, R^{4b} is H, R^{4c} is Cl, R^{4d} is H and R^{4e} is H;
• a compound of Formula (50) wherein R³ is -NHCH(Et)(CH₂OMe), R^{4a} is Cl, R^{4b} is H, R^{4c} is Cl, R^{4d} is H and R^{4e} is H;
• a compound of Formula (50) wherein R³ is -NHCH(CH₂OMe)₂, R^{4a} is Cl, R^{4b} is H, R^{4c} is Cl, R^{4d} is H and R^{4e} is H;
• a compound of Formula (50) wherein R³ is -N(Et)₂, R^{4a} is Cl, R^{4b} is H, R^{4c} is Cl, R^{4d} is H and R^{4e} is H;
• a compound of Formula (50) wherein R³ is -NHCH(CH₂OEt)₂, R^{4a} is Cl, R^{4b} is H, R^{4c} is Cl, R^{4d} is H and R^{4e} is H;
• a compound of Formula (50) wherein R³ is -NHCH(Et)₂, R^{4a} is Cl, R^{4b} is H, R^{4c} is Cl, R^{4d} is H and R^{4e} is H;
• a compound of Formula (50) wherein R³ is -N(Me)(Ph), R⁴a is Cl, R^{4b} is H, R^{4c} is Cl, R^{4d} is H and R^{4e} is H;
• a compound of Formula (50) wherein R³ is -N(n-Pr)₂, R^{4a} is Cl, R^{4b} is H, R^{4c} is Cl, R^{4d} is H and R^{4e} is H;
• a compound of Formula (50) wherein R³ is -NHCH(Et)(n-Pr), R^{4a} is Cl, R^{4b} is H, R^{4c} is Cl, R^{4d} is H and R^{4e} is H;
• a compound of Formula (50) wherein R³ is -NHCH(CH₂OMe)₂, R^{4a} is Me, R^{4b} is H, R^{4c} is Me, R^{4d} is H and R^{4e} is Me;
• a compound of Formula (50) wherein R³ is -NHCH(CH₂OMe)₂, R^{4a} is Me, R^{4b} is H, R^{4c} is Me, R^{4d} is H and R^{4e} is H;
• a compound of Formula (50) wherein R³ is -N(CH₂CH₂OMe)₂, R^{4a} is Me, R^{4b} is H, R^{4c} is Me, R^{4d} is H and R^{4e} is H;
• a compound of Formula (50) wherein R³ is -NHCH(Et)(CH₂OMe), R^{4a} is Me, R^{4b} is H, R^{4c} is Me, R^{4d} is H and R^{4e} is H;
• a compound of Formula (50) wherein R³ is -NHCH(Et)₂, R^{4a} is Me, R^{4b} is H, R^{4c} is Me, R^{4d} is H and R^{4e} is H;
• a compound of Formula (50) wherein R₃ is -N(Et)₂, R^{4a} is Me, R^{4b} is H, R^{4c} is Me, R^{4d} is H and R^{4e} is H;
• a compound of Formula (50) wherein R³ is -N(CH₂CN)₂, R^{4a} is Me, R^{4b} is H, R^{4c} is Me, R^{4d} is H and R^{4e} is H;
• a compound of Formula (50) wherein R³ is -NHCH(Me)(CH₂OMe), R^{4a} is Me, R^{4b} is H, R^{4c} is Me, R^{4d} is H and R^{4e} is H;
• a compound of Formula (50) wherein R³ is -N(n-Pr)(CH₂c-Pr), R^{4a} is Me, R^{4b} is H, R^{4c} is Me, R^{4d} is H and R^{4e} is H;
• a compound of Formula (50) wherein R³ is -NHCH(Me)(CH₂N(Me)₂), R^{4a} is Me, R^{4b} is H, R^{4c} is Me, R^{4d} is H and R^{4e} is H;
• a compound of Formula (50) wherein R³ is -N(c-Pr)(CH₂CH₂CN), R^{4a} is Me, R^{4b} is H, R^{4c} is Me, R^{4d} is H and R^{4e} is H;
• a compound of Formula (50) wherein R³ is -N(n-Pr)(CH₂CH₂CN), R^{4a} is Me, R^{4b} is H, R^{4c} is Me, R^{4d} is H and R^{4e} is H;
• a compound of Formula (50) wherein R³ is -N(n-Bu)(CH₂CN), R^{4a} is Me, R^{4b} is H, R^{4c} is Me, R^{4d} is H and R^{4e} is H;
• a compound of Formula (50) wherein R³ is -NHCH(Et)(CH₂OMe), R^{4a} is Me, R^{4b} is H, R^{4c} is Me, R^{4d} is H and R^{4e} is Me;
• a compound of Formula (50) wherein R³ is -NHCH(Et)₂, R^{4a} is Me, R^{4b} is H, R^{4c} is Me, R^{4d} is H and R^{4e} is Me;
• a compound of Formula (50) wherein R³ is -N(CH₂CH₂OMe)₂, R^{4a} is Me, R^{4b} is H, R^{4c} is Me, R^{4d} is H and R^{4e} is Me;
• a compound of Formula (50) wherein R³ is -NHCH(CH₂OMe)₂, R^{4a} is Br, R^{4b} is H, R^{4c} is OMe, R^{4d} is H and R^{4e} is H;
• a compound of Formula (50) wherein R³ is -NHCH(Et)(CH₂OMe), R^{4a} is Br, R^{4b} is H, R^{4c} is OMe, R^{4d} is H and R^{4e} is H;
• a compound of Formula (50) wherein R³ is -N(Et)₂, R^{4a} is Me, R^{4b} is H, R^{4c} is Me, R^{4d} is H and R^{4e} is Me;
• a compound of Formula (50) wherein R³ is -NHCH(CH₂OEt)₂, R^{4a} is Me, R^{4b} is H, R^{4c} is Me, R^{4d} is H and R^{4e} is Me;
• a compound of Formula (50) wherein R³ is -NHCH(CH₂CH₂OMe)(CH₂OMe)₂, R^{4a} is Me, R^{4b} is H, R^{4c} is Me, R^{4d} is H and R^{4e} is Me;
• a compound of Formula (50) wherein R³ is morpholino, R^{4a} is Me, R^{4b} is H, R^{4c} is Me, R^{4d} is H and R^{4e} is H;
• a compound of Formula (50) wherein R³ is -N(CH₂CH₂OMe)₂, R^{4a} is Br, R^{4b} is H, R^{4c} is OMe, R^{4d} is H and R^{4e} is H;
• a compound of Formula (50) wherein R³ is -NHCH(Et)₂, R^{4a} is Br, R⁴b is H, R^{4c} is OMe, R^{4d} is H and R^{4e} is H;
• a compound of Formula (50) wherein R³ is -N(Et)₂, R^{4a} is Br, R^{4b} is H, R^{4c} is OMe, R^{4d} is H and R^{4e} is H;
• a compound of Formula (50) wherein R³ is -NH(c-Pr), R⁴a is Me, R^{4b} is H, R^{4c} is Me, R^{4d} is H and R^{4e} is H;
• a compound of Formula (50) wherein R³ is -NHCH(CH₂OMe)₂, R^{4a} is CN, R^{4b} is H, R^{4c} is OMe, R^{4d} is H and R^{4e} is H;
• a compound of Formula (50) wherein R³ is -N(c-Pr)(CH₂CH₂CN), R^{4a} is Me, R^{4b} is H, R^{4c} is Me, R^{4d} is H and R^{4e} is Me;
• a compound of Formula (50) wherein R³ is -NCH(CH₂OMe)₂, R^{4a} is Me, R^{4b} is H, R^{4c} is Br, R^{4d} is H and R^{4e} is H;
• a compound of Formula (50) wherein R³ is -NHCH(CH₂OMe)(CH₂CH₂OMe). R^{4a} is Me, R^{4b} is H, R^{4c} is Br, R^{4d} is H and R^{4e} is H;
• a compound of Formula (50) wherein R³ is -NHCH(CH₂OMe)₂, R^{4a} is Me, R^{4b} is H, R^{4c} is OMe, R^{4d} is Me and R^{4e} is H;
• a compound of Formula (50) wherein R³ is -N(CH₂CH₂OMe)₂, R^{4a} is Me, R^{4b} is H, R^{4c} is OMe, R^{4d} is Me and R^{4e} is H;
• a compound of Formula (50) wherein R³ is -NHCH(Et)₂, R^{4a} is Me, R^{4b} is H, R^{4c} is OMe, R^{4d} is Me and R^{4e} is H;
• a compound of Formula (50) wherein R³ is -N(Et)₂, R^{4a} is Me, R^{4b} is H, R^{4c} is OMe, R^{4d} is Me and R^{4e} is H;
• a compound of Formula (50) wherein R³ is -NHCH(CH₂OMe)₂, R^{4a} is Cl, R^{4b} is H, R^{4c} is Me, R^{4d} is H and R^{4e} is H;
• a compound of Formula (50) wherein R is -NHCH(Et)(CH₂OMe), R^{4a} is Cl, R^{4b} is H, R^{4c} is Me, R^{4d} is H and R^{4e} is H;
• a compound of Formula (50) wherein R³ is -N(CH₂CH₂OMe)₂, R^{4a} is Cl, R^{4b} is H, R^{4c} is Me, R^{4d} is H and R^{4e} is H;
• a compound of Formula (50) wherein R³ is -NHCH(CH₂OMe)(CH₂CH₂OMe). R^{4a} is Cl, R^{4b} is H, R^{4c} is Me, R^{4d} is H and R^{4e} is H;
• a compound of Formula (50) wherein R³ is -N(c-Pr)(CH₂CH₂CN), R^{4a} is Me, R^{4b} is H, R^{4c} is OMe, R^{4d} is Me and R^{4e} is H;
• a compound of Formula (50) wherein R³ is -N(c-Pr)(CH₂CH₂CN), R^{4a} is Cl, R^{4b} is H, R^{4c} is Cl, R^{4d} is H and R^{4e} is H;
• a compound of Formula (50) wherein R³ is (S)-NHCH(CH₂OMe)(CH₂CH₂OMe), R^{4a} is Cl, R^{4b} is H, R^{4c} is Cl, R^{4d} is Hand R^{4e} is H;
• a compound of Formula (50) wherein R³ is -NHCH(CH₂OMe)(CH₂CH₂OMe), R^{4a} is Cl, R^{4b} is H, R^{4c} is Cl, R^{4d} is H and R^{4e} is H;
• a compound of Formula (50) wherein R³ is -NHCH(Et)₂, R^{4a} is Me, R^{4b} is H, R^{4c} is Br, R^{4d} is H and R^{4e} is H;
• a compound of Formula (50) wherein R³ is -N(CH₂CH₂OMe)₂, R^{4a} is Me, R^{4b} is H, R^{4c} is Br, R^{4d} is H and R^{4e} is H;
• a compound of Formula (50) wherein R³ is -NH(CH₂OMe)(CH₂-i-Pr), R^{4a} is Me, R^{4b} is H, R^{4c} is Me, R^{4d} is H and R^{4e} is H;
• a compound of Formula (50) wherein R³ is -N(CH₂CH₂OMe)₂, R^{4a} is Me, R^{4b} is H, R^{4c} is H, R^{4d} is H and R^{4e} is H;
• a compound of Formula (50) wherein R³ is -N(CH₂CH₂OMe)₂, R^{4a} is Me, R^{4b} is H, R^{4c} is NMe₂, R^{4d} is H and R^{4e} is H;
• a compound of Formula (50) wherein R³ is -NHCH(CH₂OMe)(n-Pr), R^{4a} is Me, R^{4b} is H, R^{4c} is Me, R^{4d} is H and R^{4e} is H;
• a compound of Formula (50) wherein R³ is -NHCH(CH₂OEt)(Et), R^{4a} is Me, R^{4b} is H, R^{4c} is Me, R^{4d} is H and R^{4e} is H;
• a compound of Formula (50) wherein R³ is -NHCH(CH₂OMe)(CH₂CH₂OMe), R^{4a} is Me, R^{4b} is H, R^{4c} is NMe₂, R^{4d} is H and R^{4e} is H;
• a compound of Formula (50) wherein R³ is -N(Et)₂, R^{4a} is Me, R^{4b} is H, R^{4c} is Cl, R^{4d} is H and R^{4e} is H;
• a compound of Formula (50) wherein R³ is -NHCH(Et)₂, R^{4a} is Me, R^{4b} is H, R^{4c} is Cl, R^{4d} is H and R^{4e} is H;
• a compound of Formula (50) wherein R³ is -N(CH₂CH₂OMe)₂, R^{4a} is Me, R^{4b} is H, R^{4c} is Cl, R^{4d} is H and R^{4e} is H;
• a compound of Formula (50) wherein R³ is -NHCH(CH₂OMe)₂, R^{4a} is Me, R^{4b} is H, R^{4c} is Cl, R^{4d} is H and R^{4e} is H;
• a compound of Formula (50) wherein R³ is -N(Et)₂, R^{4a} is Me, R^{4b} is H, R^{4c} is • R^{4d} is H and R^{4e} is H;
• a compound of Formula (50) wherein R³ is -N(Et)₂, R^{4a} is Cl, R^{4b} is H, R^{4c} is Me, R^{4d} is H and R^{4e} is H;
• a compound of Formula (50) wherein R³ is -NHCH(Et)₂, R^{4a} is Cl, R^{4b} is H, R^{4c} is Me, R^{4d} is H and R^{4e} is H;
• a compound of Formula (50) wherein R³ is -NHCH(Et)₂, R^{4a} is Me, R^{4b} is H, R^{4c} is NMe₂, R^{4d} is H and R^{4e} is H;
• a compound of Formula (50) wherein R³ is (S)-NHCH(CH₂OMe)(CH₂CH₂OMe), R^{4a} is Me, R^{4b} is H, R^{4c} is Me, R^{4d} is H and R^{4e} is H;
• a compound of Formula (50) wherein R³ is -NHCH(CH₂OMe)(CH₂CH₂OMe), R^{4a} is Me, R^{4b} is H, R^{4c} is Me, R^{4d} is H and R^{4e} is H;
• a compound of Formula (50) wherein R³ is (S)-NHCH(CH₂OMe)(CH₂CH₂OMe), R^{4a} is Me, R^{4b} is H, R^{4c} is Cl, R^{4d} is H and R^{4e} is H;
• a compound of Formula (50) wherein R³ is -NHCH(CH₂OMe)(CH₂CH₂OMe), R^{4a} is Me, R^{4b} is H, R^{4c} is Cl, R^{4d} is H and R^{4e} is H;
• a compound of Formula (50) wherein R³ is -N(c-Pr)(CH₂CH₂CN), R^{4a} is Me, R^{4b} is H, R^{4c} is Cl, R^{4d} is H and R^{4e} is H;
• a compound of Formula (50) wherein R³ is -NH(Et)(CH₂CN), R^{4a} is Me, R^{4b} is H, R^{4c} is Cl, R^{4d} is H and R^{4e} is H;
• a compound of Formula (50) wherein R³ is -N(Et)₂, R^{4a} is Me, R^{4b} is Me, R^{4c} is OMe, R^{4d} is H and R^{4e} is H;
• a compound of Formula (50) wherein R³ is -N(CH₂CH₂OMe)(CH₂CH₂OH), R^{4a} is Cl, R^{4b} is H, R^{4c} is Cl, R^{4d} is H and R^{4e} is H;
• a compound of Formula (50) wherein R³ is -N(CH₂CH₂OMe)₂, R^{4a} is Me, R^{4b} is Me, R^{4c} is OMe, R^{4d} is H and R^{4e} is H;
• a compound of Formula (50) wherein R³ is -NHCH(Et)₂, R^{4a} is Me, R^{4b} is Me, R^{4c} is OMe, R^{4d} is H and R^{4e} is H;
• a compound of Formula (50) wherein R³ is -N(CH₂c-Pr)(n-Pr), R^{4a} is Me, R^{4b} is H, R^{4c} is Cl, R^{4d} is H and R^{4e} is H;
• a compound of Formula (50) wherein R³ is -N(c-Pr)(CH₂CH₂CN), R^{4a} is Me, R^{4b} is Me, R^{4c} is OMe, R^{4d} is H and R^{4e} is H;
• a compound of Formula (50) wherein R³ is -NHCH(Et)₂, R^{4a} is Cl, R^{4b} is H, R^{4c} is OMe, R^{4d} is H and R^{4e} is H;
• a compound of Formula (50) wherein R³ is -N(Et)₂, R^{4a} is Cl, R^{4b} is H, R^{4c} is OMe, R^{4d} is H and R^{4e} is H;
• a compound of Formula (50) wherein R³ is -N(CH₂CH₂OMe)₂, R^{4a} is Cl, R^{4b} is H, R^{4c} is OMe, R^{4d} is H and R^{4e} is H;
• a compound of Formula (50) wherein R³ is -NHCH(Et)(CH₂OMe), R^{4a} is Cl, R^{4b} is H, R^{4c} is OMe, R^{4d} is H and R^{4e} is H;
• a compound of Formula (50) wherein R³ is -N(Et)₂, R^{4a} is Cl, R^{4b} is H, R^{4c} is CN, R^{4d} is H and R^{4e} is H;
• a compound of Formula (50) wherein R³ is -N(c-Pr)(CH₂CH₂CN), R^{4a} is Cl, R^{4b} is H, R^{4c} is OMe, R^{4d} is H and R^{4e} is H;
• a compound of Formula (50) wherein R³ is -NHCH(CH₂OH)₂, R^{4a} is Cl, R^{4b} is H, R^{4c} is Cl, R^{4d} is H and R^{4e} is H; and
• a compound of Formula (50) wherein R³ is -N(CH₂CH₂OMe)₂, R^{4a} is Me, R^{4b} is H, R^{4c} is OMe, R^{4d} is H and R^{4e} is H,
and geometric isomers thereof, stereoisomeric forms thereof, or mixtures of stereoisomeric forms thereof, and pharmaceutically acceptable salt or pro-drug forms thereof.

14. The [1,5-a]-pyrazolo-1,3,5-triazines according to claim 1, wherein
Ar is 2-chloro-5-fluoro-methoxyphenyl;
R¹ and R² are methyl; and
R³ is NHCH(Et)₂,
and geometric isomers thereof, stereoisomeric forms thereof, or mixtures of stereoisomeric forms thereof, and pharmaceutically acceptable salt or pro-drug forms thereof.

15. [1,5-a]-Pyrazolo-1,3,5-triazines of the Formula (1) 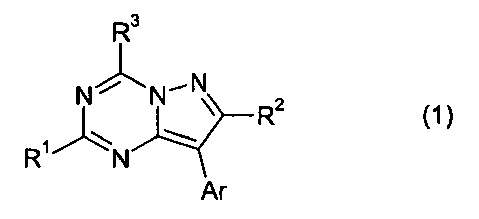 wherein
Ar is phenyl, pyridyl or 2,3-dihydrobenzofuranyl, each (Ar) optionally substituted with 1 to 4 R⁴ substituents;
R¹ and R² are independently selected from C₁-C₄ alkyl;
R³ is NR^{6a}R^{7a};
R⁴ is independently selected at each occurrence from: C₁-C₁₀ alkyl, C₂-C₁₀ alkenyl, C₂-C₁₀ alkynyl, C₃-C₆ cycloalkyl, C₄-C₁₂ cycloalkylalkyl, NO₂, halo, CN, C₁-C₄ haloalkyl, NR⁶R⁷, NR⁸COR⁷, NR⁸CO₂R⁷, COR⁷, OR⁷, CONR⁶R⁷, CO(NOR⁹)R⁷, CO₂R⁷, or S(O)ₙR⁷, where each such C₁-C₁₀ alkyl, C₂-C₁₀ alkenyl, C₂-C₁₀ alkynyl, C₃-C₆ cycloalkyl and C₄-C₁₂ cycloalkylalkyl are optionally substituted with 1 to 3 substituents independently selected at each occurrence from C₁-C₄ alkyl, NO₂, halo, CN, NR⁶R⁷, NR⁸COR⁷, NR⁸CO₂R⁷, COR⁷ OR⁷, CONR⁶R⁷, CO₂R⁷, CO(NOR⁹)R⁷, or S(O)ₙR⁷;
R⁶ and R⁷, R^{6a} and R^{7a} are independently selected at each occurrence from:
• H,
• C₁-C₁₀ alkyl, C₃-C₁₀ alkenyl, C₃-C₁₀ alkynyl, C₁-C₁₀ haloalkyl with 1-10 halogens, C₂-C₈ alkoxyalkyl, C₃-C₆ cycloalkyl, C₄-C₁₂ cycloalkylalkyl, C₅-C₁₀ cycloalkenyl, or C₆-C₁₄ cycloalkenylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryl, heteroaryl or heterocyclyl,
• aryl, aryl(C₁-C₄ alkyl), heteroaryl, heteroaryl(C₁-C₄ alkyl), heterocyclyl or heterocyclyl(C₁-C₄ alkyl);
alternatively, NR⁶R⁷ and NR^{6a}R^{7a} are independently piperidine, pyrrolidine, piperazine, N-methylpiperazine, morpholine or thiomorpholine, each optionally substituted with 1 to 3 C₁-C₄ alkyl groups;
R⁸ is independently selected at each occurrence from H or C₁-C₄ alkyl;
R⁹ and R¹⁰ are independently selected at each occurrence from H, C₁-C₄ alkyl, or C₃-C₆ cycloalkyl;
R¹¹ is selected from H, C₁-C₄ alkyl, C₁-C₄ haloalkyl, or C₃-C₆ cycloalkyl;
R¹² is C₁-C₄ alkyl or C₁-C₄ haloalkyl;
R¹³ is selected from C₁-C₄ alkyl, C₁-C₄ haloalkyl, C₂-C₈ alkoxyalkyl, C₃-C₆ cycloalkyl, C₄-C₁₂ cycloalkylalkyl, aryl, aryl(C₁-C₄ alkyl)-, heteroaryl or heteroaryl(C₁-C₄ alkyl)-;
R¹⁴ is selected from C₁-C₁₀ alkyl,C₃-C₁₀ alkenyl, C₃-C₁₀ alkynyl, C₃-C₈ cycloalkyl, or C₄-C₁₂ cycloalkylalkyl, each optionally substituted with 1 to 3 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, CONR¹⁶R¹⁵, and C₁-C₆ alkylthio, C₁-C₆ alkylsulfinyl and C₁-C₆ alkylsulfonyl;
R¹⁵ and R¹⁶ are independently selected at each occurrence from H, C₁-C₆ alkyl, C₃-C₁₀ cycloalkyl, C₄-C₁₆ cycloalkylalkyl, except that for S(O)ₙR¹⁵, R¹⁵ cannot be H;
aryl is phenyl or naphthyl, each optionally substituted with 1 to 5 substituents independently selected at each occurrence from C₁-C₆ alkyl, C₃-C₆ cycloalkyl, halo, C₁-C₄haloalkyl, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, and CONR¹⁶R¹⁵;
heteroaryl is pyridyl, pyrimidinyl, triazinyl, furanyl, pyranyl, quinolinyl, isoquinolinyl, thienyl, imidazolyl, thiazolyl, indolyl, pyrrolyl, oxazolyl, benzofuranyl, benzothienyl, benzothiazolyl, isoxazolyl, pyrazolyl, 2,3 dihydrobenzothienyl or 2,3-dihydrobenzofuranyl, each being optionally substituted with 1 to 5 substituents independently selected at each occurrence from C₁-C₆ alkyl, C₃-C₆ cycloalkyl, halo, C₁-C₄ haloalkyl, cyano, OR¹⁵, SH, S(O)ₙR¹⁵, -COR¹⁵, CO₂R¹⁵, CO(O)R¹⁵, NR⁸COR¹⁵, N(COR¹⁵)₂, NR⁸CONR¹⁶R¹⁵, NR⁸CO₂R¹⁵, NR¹⁶R¹⁵ and CONR¹⁶R¹⁵;
heterocyclyl is saturated or partially saturated heteroaryl, optionally substituted with 1 to 5 substituents independently selected at each occurrence from C₁-C₆ alkyl, C₃-C₆ cycloalkyl, halo, C₁-C₄haloalkyl, cyano, OR¹⁵, SH, S(O)ₙR¹⁵, COR¹⁵, CO₂R¹⁵, OC(O)R¹⁵ , NR⁸COR¹⁵, N(COR¹⁵)₂, NR⁸CONR¹⁶R¹⁵, NR⁸CO₂R¹⁵, NR¹⁵R¹⁶, and CONR¹⁶R¹⁵;
n is independently at each occurrence 0, 1 or 2,
and geometric isomers thereof, stereoisomeric forms thereof, or mixtures of stereoisomeric forms thereof, and pharmaceutically acceptable salt or pro-drug forms thereof.

16. The [1,5-a]-pyrazolo-1,3,5-triazines according to claim 15, wherein
Ar is phenyl, pyridyl or 2,3-dihydrobenzofuranyl, each (Ar) substituted with 1 to 4 R⁴ substituents,
and geometric isomers thereof, stereoisomeric forms thereof, or mixtures of stereoisomeric forms thereof, and pharmaceutically acceptable salt or pro-drug forms thereof.

17. The [1,5-a]-pyrazolo-1,3,5-triazines according to claim 15 or 16 wherein
Ar is phenyl or pyridyl, each (Ar) optionally substituted with 1 to 4 R⁴ substituents,
and geometric isomers thereof, stereoisomeric forms thereof, or mixtures of stereoisomeric forms thereof, and pharmaceutically acceptable salt or pro-drug forms thereof.

18. The [1,5-a]-pyrazolo-1,3,5-triazines according to any one of claims 15 to 17, wherein
R^{6a} and R^{7a} are independently H or C₁-C₁₀ alkyl,
and geometric isomers thereof, stereoisomeric forms thereof, or mixtures of stereoisomeric forms thereof, and pharmaceutically acceptable salt or pro-drug forms thereof.

19. Pharmaceutical composition, comprising a [1,5-a]-pyrazolo-1,3,5-triazine of the Formula (1) as claimed in any one of claims 1 to 18 or a geometric isomer thereof, stereoisomeric form thereof, or mixture of stereoisomeric forms thereof, or a pharmaceutically acceptable salt or pro-drug form thereof.

20. The use of a [1,5-a]-pyrazolo-1,3,5-triazine of the Formula (1) as claimed in any one of claims 1 to 18 or a geometric isomer thereof, stereoisomeric form thereof, or mixture of stereoisomeric forms thereof, or a pharmaceutically acceptable salt or prodrug form thereof, in the manufacture of a medicament for treating affective disorder, anxiety, depression, headache, irritable bowel syndrome, post-traumatic stress disorder, supranuclear palsy, immune suppression, Alzheimer's disease, gastrointestinal diseases, anorexia nervosa or other feeding disorder, drug addiction, drug or alcohol withdrawal symptoms, inflammatory diseases, cardiovascular or heart-related diseases, fertility problems, human immunodeficiency virus infections, hemorrhagic stress, obesity, infertility, head and spinal cord traumas, epilepsy, stroke, ulcers, amyotrophic lateral sclerosis, or hypoglycemia

21. The use according to claim 20, for treating affective disorder, anxiety, depression, irritable bowel syndrome, post-traumatic stress disorder, supranuclear palsy, immune suppression, Alzheimer's disease, gastrointestinal diseases, anorexia nervosa or other feeding disorder, drug addiction, drug or alcohol withdrawal symptoms, inflammatory diseases, or fertility problems.

22. The use according to claim 21, for treating anxiety.

## Patentansprüche

1. [1,5-a]-Pyrazolo-1,3,5-triazine der Formel (1) 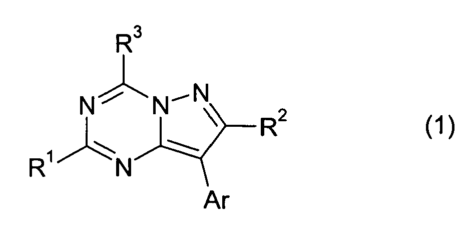 worin
Ar für Phenyl, Pyridyl oder 2,3-Dihydrobenzofuranyl steht, wobei jedes (Ar) wahlweise mit 1 bis 4 R⁴-Substituenten substituiert ist;
R¹ jeweils unabhängig ausgewählt ist unter H, C₁-C₄-Alkyl, C₂-C₄-Alkenyl, C₂-C₄-Alkinyl, Halo, CN, C₁-C₄-Haloalkyl, C₁-C₁₂-Hydroxyalkyl, C₂-C₁₂-Alkoxyalkyl, C₂-C₁₀-Cyanoalkyl, C₃-C₆-Cycloalkyl, C₄-C₁₀-Cycloalkylalkyl, NR⁹R¹⁰, C₁-C₄-Alkyl-NR⁹R¹⁰, NR⁹COR¹⁰, OR¹¹, SH oder S(O)ₙR¹²;
R² ausgewählt ist unter H, C₁-C₄-Alkyl, C₂-C₄-Alkenyl, C₂-C₄-Alkinyl, C₃-C₆-Cycloalkyl, C₄-C₁₀-Cycloalkylalkyl, C₁-C₄-Hydroxyalkyl, Halo, CN, -NR⁶R⁷, NR⁹COR¹⁰, -NR⁶S(O)ₙR⁷, S(O)ₙNR⁶R⁷, C₁-C₄-Haloalkyl, -OR⁷, SH oder -S(O)ₙR¹²;
R³ NR^{6a}R^{7a} ist;
R⁴ jeweils unabhängig ausgewählt ist unter C₁-C₁₀-Alkyl, C₂-C₁₀-Alkenyl, C₂-C₁₀-Alkinyl, C₃-C₆-Cycloalkyl, C₄-C₁₂-Cycloalkylalkyl, NO₂, Halo, CN, C₁-C₄-Haloalkyl, NR⁶R⁷, NR⁸COR⁷, NR⁸CO₂R⁷, COR⁷, OR⁷, CONR⁶R⁷, CO(NOR⁹)R⁷, CO₂R⁷ oder S(O)ₙR⁷, ein jedes solches C₁-C₁₀-Alkyl, C₂-C₁₀-Alkenyl, C₂-C₁₀-Alkinyl, C₃-C₆-Cycloalkyl und C₄-C₁₂-Cycloalkylalkyl wahlweise substituiert mit 1 bis 3 Substituenten, die jeweils unabhänig ausgewählt sind unter C₁-C₄-Alkyl, NO₂, Halo, CN, NR⁶R⁷, NR⁸COR⁷, NR⁸CO₂R⁷, COR⁷ OR⁷, CONR⁶R⁷, CO₂R⁷, CO(NOR⁹)R⁷ oder S(O)ₙR⁷;
R⁶ und R⁷, R^{6a} und R^{7a} jeweils unabhängig ausgewählt sind unter:
• H,
• C₁-C₁₀-Alkyl, C₃-C₁₀-Alkenyl, C₃-C₁₀-Alkinyl, C₁-C₁₀-Haloalkyl mit 1-10 Halogenen, C₂-C₈-Alkoxyalkyl, C₃-C₆-Cycloalkyl, C₄-C₁₂-Cycloalkylalkyl, C₅-C₁₀-Cycloalkenyl oder C₆-C₁₄-Cycloalkenylalkyl, jedes wahlweise substituiert mit 1 bis 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, Aryl, Heteroaryl oder Heterocyclyl,
• Aryl, Aryl(C₁-C₄-Alkyl), Heteroaryl, Heteroaryl(C₁-C₄-Alkyl), Heterocyclyl oder Heterocyclyl(C₁-C₄-Alkyl);
alternativerweise NR⁶R⁷ und NR^{6a}R^{7a} unabhängig für Piperidin, Pyrrolidin, Piperazin, N-Methylpiperazin, Morpholin oder Thiomorpholin stehen, jedes wahlweise mit 1 bis 3 C₁-C₄-Alkylgruppen substitutiert;
R⁸ jeweils unabhängig ausgewählt ist unter H oder C₁-C₄-Alkyl;
R⁹ und R¹⁰ jeweils unabhängig ausgewählt sind unter H, C₁-C₄-Alkyl, oder C₃-C₆-Cycloalkyl;
R¹¹ ausgewählt ist unter H, C₁-C₄-Alkyl, C₁-C₄-Haloalkyl, oder C₃-C₆-Cycloalkyl;
R¹² für C₁-C₄-Alkyl oder C₁-C₄-Haloalkyl steht;
R¹³ ausgewählt ist unter C₁-C₄-Alkyl, C₁-C₄-Haloalkyl, C₂-C₈-Alkoxyalkyl, C₃-C₆-Cycloalkyl, C₄-C₁₂-Cycloalkylalkyl, Aryl, Aryl(C₁-C₄-Alkyl)-, Heteroaryl oder Heteroaryl(C₁-C₄-Alkyl)-;
R¹⁴ ausgewählt ist unter C₁-C₁₀-Akyl, C₃-C₁₀-Alkenyl, C₃-C₁₀-Alkinyl, C₃-C₈-Cycloalkyl, oder C₄-C₁₂-Cycloalkylalkyl, jedes wahlweise substituiert mit 1 bis 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, CONR¹⁶R¹⁵, und C₁-C₆-Alkylthio, C₁-C₆-Alkylsulfinyl und C₁-C₆-Alkylsulfonyl;
R¹⁵ und R¹⁶ jeweils unabhängig ausgewählt sind unter H, C₁-C₆-Alkyl, C₃-C₁₀-Cycloalkyl, C₄-C₁₆-Cycloalkylalkyl, mit der Maßgabe, dass R¹⁵ in S(O)ₙR¹⁵ nicht H sein kann;
wobei Aryl Phenyl oder Naphthyl ist, wobei jedes wahlweise substituiert ist mit 1 bis 5 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵ und CONR¹⁶R¹⁵_{;}
Heteroaryl Pyridyl, Pyrimidinyl, Triazinyl, Furanyl, Pyranyl, Chinolinyl, Isochinolinyl, Thienyl, Imidazolyl, Thiazolyl, Indolyl, Pyrrolyl, Oxazolyl, Benzofuranyl, Benzothienyl, Benzothiazolyl, Isoxazolyl, Pyrazolyl, 2,3-Dihydrobenzothienyl oder 2,3-Dihydrobenzofuranyl ist, wobei jedes wahlweise substituiert ist mit 1 bis 5 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, -COR¹⁵, CO₂R¹⁵, CO(O)R¹⁵, NR⁸COR¹⁵, N(COR¹⁵)₂, NR⁸CONR¹⁶R¹⁵, NR⁸CO₂R¹⁵, NR¹⁶R¹⁵ und CONR¹⁶R¹⁵;
Heterocyclyl gesättigtes oder partiell gesättigtes Heteroaryl ist, wahlweise substituiert mit 1 bis 5 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁵R¹⁶ und CONR¹⁶R¹⁵ ;
n jeweils unabhängig 0, 1 oder 2 ist,
mit der Maßgabe, dass:
(1) Ar nicht unsubstituiertes Phenyl oder m-Methylphenyl ist, falls R¹ Methyl oder Ethyl ist, R² H ist und R³ NH(n-C₄H₉) oder N(C₂H₅)₂ ist; und
(2) R^{6a} und R^{7a} nicht H oder Alkyl sind, falls R² H ist und Ar Pyridyl ist und R³ NR^{6a}R^{7a} ist,
und geometrische Isomere davon, stereoisomere Formen davon, oder Gemische stereoisomerer Formen davon, und pharmazeutisch akzeptable Salze oder Propharmaka davon.

2. [1,5-a]-Pyrazolo-1,3,5-triazine nach Anspruch 1, wobei
Ar Phenyl, Pyridyl oder 2,3-Dihydrobenzofuranyl ist, wobei jedes (Ar) mit 1 bis 4 R⁴-Substituenten substituiert ist,
und geometrische Isomere davon, stereoisomere Formen davon, oder Gemische stereoisomerer Formen davon, und pharmazeutisch akzeptable Salze oder Propharmaka davon.

3. [1,5-a]-Pyrazolo-1,3,5-triazine nach Anspruch 1 oder 2, wobei
Ar Phenyl oder Pyridyl ist, wobei jedes (Ar) wahlweise mit 1 bis 4 R⁴-Substituenten substituiert ist,
und geometrische Isomere davon, stereoisomere Formen davon, oder Gemische stereoisomerer Formen davon, und pharmazeutisch akzeptable Salze oder Propharmaka davon.

4. [1,5-a]-Pyrazolo-1,3,5-triazine nach einem der Ansprüche 1 bis 3, wobei
R¹ und R² unabhängig ausgewählt sind unter H, C₁₋₄-Alkyl, C₃-C₆-Cycloalkyl, C₄-C₁₀-Cycloalkylalkyl,
und geometrische Isomere davon, stereoisomere Formen davon, oder Gemische stereoisomerer Formen davon, und pharmazeutisch akzeptable Salze oder Propharmaka davon.

5. [1,5-a]-Pyrazolo-1,3,5-triazine nach Anspruch 4, wobei
R¹ und R² unabhängig ausgewählt sind unter H oder C₁₋₄-Alkyl,
und geometrische Isomere davon, stereoisomere Formen davon, oder Gemische stereoisomerer Formen davon, und pharmazeutisch akzeptable Salze oder Propharmaka davon.

6. [1,5-a]-Pyrazolo-1,3,5-triazine nach Anspruch 4, wobei
R¹ und R² unabhängig ausgewählt sind unter C₁₋₄-Alkyl,
und geometrische Isomere davon, stereoisomere Formen davon, oder Gemische stereoisomerer Formen davon, und pharmazeutisch akzeptable Salze oder Propharmaka davon.

7. [1,5-a]-Pyrazolo-1,3,5-triazine nach Anspruch 4, wobei
R¹ Methyl ist,
und geometrische Isomere davon, stereoisomere Formen davon, oder Gemische stereoisomerer Formen davon, und pharmazeutisch akzeptable Salze oder Propharmaka davon.

8. [1,5-a]-Pyrazolo-1,3,5-triazine nach einem der Ansprüche 1 bis 7, wobei
R^{6a} unabhängig ausgewählt ist unter:
• H,
• C₁-C₁₀-Alkyl, C₃-C₁₀-Alkenyl, C₃-C₁₀-Alkinyl, C₁-C₁₀-Haloalkyl mit 1-10 Halogenen, C₂-C₈-Alkoxyalkyl, C₃-C₆-Cycloalkyl, C₄-C₁₂-Cycloalkylalkyl, C₅-C₁₀-Cycloalkenyl oder C₆-C₁₄-Cycloalkenylalkyl, jedes wahlweise substituiert ist mit 1 bis 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, Aryl, Heteroaryl oder Heterocyclyl,
• Aryl, Aryl(C₁-C₄-Alkyl)-, Heteroaryl, Heteroaryl(C₁-C₄-Alkyl)-, Heterocyclyl oder Heterocyclyl(C₁-C₄-Alkyl)-; und
R^{7a} unabhängig ausgewählt ist unter
• H,
• C₅-C₁₀-Alkyl, C₃-C₁₀-Alkenyl, C₃-C₁₀-Alkinyl, C₁-C₁₀-Haloalkyl mit 1-10 Halogenen, C₂-C₈-Alkoxyalkyl, C₃-C₆-Cycloalkyl, C₄-C₁₂-Cycloalkylalkyl, C₅-C₁₀-Cycloalkenyl, oder C₆-C₁₄-Cycloalkenylalkyl, jedes wahlweise substituiert mit 1 bis 3 Substituenten, die jeweils unabhängig ausgewählt sind unter C₁-C₆-Alkyl, C₃-C₆-Cycloalkyl, Halo, C₁-C₄ Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, Aryl, Heteroaryl oder Heterocyclyl,
• Aryl, Aryl(C₁-C₄-Alkyl)-, Heteroaryl, Heteroaryl(C₁-C₄-Alkyl)-, Heterocyclyl oder Heterocyclyl(C₁-C₄-Alkyl)-;
alternativerweise NR⁶R⁷ und NR^{6a}R^{7a} unabhängig Piperidin, Pyrrolidin, Piperazin, N-Methylpiperazin, Morpholin oder Thiomorpholin sind, jedes wahlweise substituiert mit 1 bis 3 C₁-C₄-Alkylgruppen,
und geometrische Isomere davon, stereoisomere Formen davon, oder Gemische stereoisomerer Formen davon, und pharmazeutisch akzeptable Salze oder Propharmaka davon.

9. [1,5-a]-Pyrazolo-1,3,5-triazine nach einem der Ansprüche 1 bis 7, wobei
R^{6a} ausgewählt ist unter:
• H,
• C₁-C₁₀-Alkyl, C₃-C₁₀-Alkenyl, C₃-C₁₀Alkinyl, C₁-C₁₀-Haloalkyl mit 1-10 Halogenen, C₂-C₈-Alkoxyalkyl, C₃-C₆-Cycloalkyl, C₄-C₁₂-Cycloalkylalkyl, C₅-C₁₀-Cycloalkenyl, oder C₆-C₁₄-Cycloalkenylalkyl, jedes wahlweise substituiert mit 1 to 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, Aryl, Heteroaryl oder Heterocyclyl,
• Aryl, Aryl(C₁-C₄-Alkyl)-, Heteroaryl, Heteroaryl(C₁-C₄-Alkyl)-, Heterocyclyl oder Heterocyclyl(C₁-C₄-Alkyl)-;
R^{7a} ausgewählt ist unter:
• C₁-C₄-Alkyl und ein jedes solches C₁-C₄-Alkyl substituiert ist mit 1-3 Substituenten, die jeweils unabhängig ausgewählt sind unter C₁-C₆-Alkyl, C₃-C₆-Cycloalkyl, Halo,C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵,CONR¹⁶R¹⁵, Aryl, Heteroaryl oder Heterocyclyl,
und geometrische Isomere davon, stereoisomere Formen davon, oder Gemische stereoisomerer Formen davon, und pharmazeutisch akzeptable Salze oder Propharmaka davon.

10. [1,5-a]-Pyrazolo-1,3,5-triazine nach einem der Ansprüche 1 bis 7, wobei
R^{6a} H ist,
R^{7a} ausgewählt ist unter:
• C₁-C₄-Alkyl und ein jedes solches C₁-C₄-Alkyl substituiert ist mit 1-3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, Aryl, Heteroaryl oder Heterocyclyl,
und geometrische Isomere davon, stereoisomere Formen davon, oder Gemische stereoisomerer Formen davon, und pharmazeutisch akzeptable Salze oder Propharmaka davon.

11. [1,5-a]-Pyrazolo-1,3,5-triazine nach einem der Ansprüche 1 bis 7, wobei
R^{6a} und R^{7a} unabhängig H oder C₁-C₁₀-Alkyl sind, wobei ein jedes solches C₁-C₁₀-Alkyl wahlweise substituiert ist mit 1 to 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, R⁸CONR¹⁶R¹⁵, NR⁸CO₂R¹³ NR¹⁶R¹⁵, CONR¹⁶R¹⁵, Aryl, Heteroaryl oder Heterocyclyl,
und geometrische Isomere davon, stereoisomere Formen davon, oder Gemische stereoisomerer Formen davon, und pharmazeutisch akzeptable Salze oder Propharmaka davon.

12. [1,5-a]-Pyrazolo-1,3,5-triazine nach einem der Ansprüche 1 bis 7, wobei
R^{6a} und R^{7a} unabhängig H oder C₁-C₁₀-Alkyl sind,
und geometrische Isomere davon, stereoisomere Formen davon, oder Gemische stereoisomerer Formen davon, und pharmazeutisch akzeptable Salze oder Propharmaka davon.

13. [1,5-a]-Pyrazolo-1,3,5-triazine nach Anspruch 1 der Formel (50) 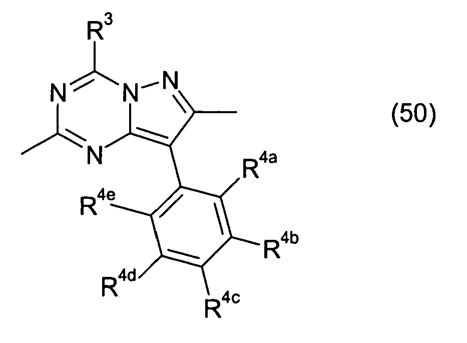 worin die Verbindung ausgewählt ist unter:
• einer Verbindung der Formel (50), worin R³ -NHCH(n-Pr)₂ ist, R^{4a} Cl ist, R^{4b} H ist, R^{4c} Cl ist, R^{4d} H ist und R^{4e} H ist;
• einer Verbindung der Formel (50), worin R³ -N(Et)(n-Bu) ist, R^{4a} Cl ist, R^{4b} H ist, R^{4c} Cl ist, R^{4d} H ist und R^{4e} H ist;
• einer Verbindung der Formel (50), worin R³ -N(CH₂CH₂OMe)₂ ist, R^{4a} Cl ist, R^{4b} H ist, R^{4c} Cl ist, R^{4d} H ist and R^{4e} H ist;
• einer Verbindung der Formel (50), worin R³ -NHCH(Et)(n-Bu) ist, R^{4a} Cl ist, R^{4b} H ist, R^{4c} Cl ist, R^{4d} H ist und R^{4e} H ist;
• einer Verbindung der Formel (50), worin R³ -NHCH(Et)(CH₂OMe) ist, R^{4a} Cl ist, R^{4b} H ist, R^{4c} Cl ist, R^{4d} H ist und R^{4e} H ist;
• einer Verbindung der Formel (50), worin R³ -NHCH(CH₂OMe)₂ ist, R^{4a} Cl ist, R^{4b} H ist, R^{4c} Cl ist, R^{4d} H ist und R^{4e} H ist;
• einer Verbindung der Formel (50), worin R³ -N(Et)₂ ist, R^{4a} Cl ist, R^{4b} H ist, R^{4c} Cl ist, R^{4d} H ist und R^{4e} H ist;
• einer Verbindung der Formel (50), worin R³ -NHCH(CH₂OEt)₂ ist, R^{4a} Cl ist, R^{4b} H ist, R^{4c} Cl ist, R^{4d} H und R^{4e} H ist;
• einer Verbindung der Formel (50), worin R³ -NHCH(Et)₂ ist, R^{4a} Cl ist, R^{4b} H ist, R^{4c} Cl ist, R^{4d} H ist und R^{4e} H ist;
• einer Verbindung der Formel (50), worin R³ -N(Me)(Ph) ist, R⁴a Cl ist, R^{4b} H ist, R^{4c} Cl ist, R^{4d} H ist und R^{4e} H ist;
• einer Verbindung der Formel (50), worin R³ -N(n-Pr)₂ ist, R^{4a} Cl ist, R^{4b} H ist, R^{4c} Cl ist, R^{4d} H ist und R^{4e} H ist;
• einer Verbindung der Formel (50), worin R³ -NHCH(Et)(n-Pr) ist, R^{4a} Cl ist, R^{4b} H ist, R^{4c} Cl ist, R^{4d} H ist und R^{4e} H ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} Me ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)(CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R₃ -N(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂CN)₂ ist, R^{4a} Me ist, R^{4b} H ist, R^{4c} Me ist, R^{4d} H ist und R^{4e} H ist;
• einer Verbindung der Formel (50), worin R³ -NHCH(Me)(CH₂OMe) ist, R^{4a} Me ist, R^{4b} H ist, R^{4c} Me ist, R^{4d} H ist und R^{4e} H ist;
• einer Verbindung der Formel (50), worin R³ -N(n-Pr)(CH₂c-Pr) ist, R^{4a} Me ist, R^{4b} H ist, R^{4c} Me ist, R^{4d} H ist und R^{4e} H ist;
• einer Verbindung der Formel (50), worin R³ -NHCH(Me)(CH₂N(Me)₂) ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -N(n-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -N(n-Bu)(CH₂CN), R^{4a} Me ist, R^{4b} H ist, R^{4c} Me ist, R^{4d} H ist and R^{4e} H ist;
• einer Verbindung der Formel (50), worin R³ -NHCH(Et)(CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)(CH₂OMe) ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OEt)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂CH₂OMe)(CH₂OMe)₂ ist, R^{4a} Me ist, R^{4b} H ist, R^{4c} Me ist, R^{4d} H ist und R^{4e} H ist;
• einer Verbindung der Formel (50), worin R³ Morpholino ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -NH(c-Pr) ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -NCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)(CH₂OMe) ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ (S)-NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NH(CH₂OMe)(CH₂-i-Pr) ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(n-Pr) ist, R^{4a} Me ist, R^{4b} H ist, R^{4c} Me ist, R^{4d} H ist und R^{4e} H ist;
• einer Verbindung der Formel (50), worin R³ -NHCH(CH₂OEt)(Et) ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)₂ ist, R^{4a} Me ist, R^{4b} H ist, R^{4c} Me ist, R^{4d} H ist und R^{4e} H ist;
• einer Verbindung der Formel (50), worin R³ -N(Et)₂, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ (S)-NHCH(CH₂OMe) (CH₂CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ (S)-NHCH(CH₂OMe) (CH₂CH₂OMe) ist, R^{4a} Me ist, R^{4b} H ist, R^{4c} Me ist, R^{4d} H ist und R^{4e} H ist;
• einer Verbindung der Formel (50), worin R³ -NHCH(CH₂OMe)(CH₂CH₂OMe) ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -NH(Et)(CH₂CN) ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)(CH₂CH₂OH) ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂c-Pr)(n-Pr) ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -NHCH(Et)₂ ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -N(CH₂CH₂OMe)₂ ist, R^{4a} Me ist, R^{4b} H ist, R^{4c} Me ist, R^{4d} H ist und R^{4e} H ist;
• einer Verbindung der Formel (50), worin R³ -NHCH(Et)(CH₂OMe) ist, R^{4a} Me ist, R^{4b} H ist, R^{4c} Me ist, R^{4d} H ist und R^{4e} H ist;
• einer Verbindung der Formel (50), worin R³ -N(Et)₂ ist, R^{4a} Me ist, R^{4b} H ist, R^{4c} Me ist, R^{4d} H ist und R^{4e} H ist;
• einer Verbindung der Formel (50), worin R³ -N(c-Pr)(CH₂CH₂CN) ist, R^{4a} Me ist, R^{4b} H ist, R^{4c} Me ist, R^{4d} H ist und R^{4e} H ist;
• einer Verbindung der Formel (50), worin R³ -NHCH(CH₂OH)₂ ist, R^{4a} Me ist, R^{4b} H ist, R^{4c} Me ist, R^{4d} H ist und R^{4e} H ist;
• einer Verbindung der Formel (50), worin R³- N(CH₂CH₂OMe)₂ ist, R^{4a} Me ist, R^{4b} H ist, R^{4c} Me ist, R^{4d} H ist und R^{4e} H ist;
und geometrische Isomere davon, stereoisomere Formen davon, oder Gemische stereoisomerer Formen davon, und pharmazeutisch akzeptable Salze oder Propharmaka davon.

14. [1,5-a]-Pyrazolo-1,3,5-triazine nach Anspruch 1, wobei
Ar 2-Chlor-5-fluor-methoxyphenyl ist;
R¹ und R² Methyl sind; und
R³ NHCH(Et)₂ ist,
und geometrische Isomere davon, stereoisomere Formen davon, oder Gemische stereoisomerer Formen davon, und pharmazeutisch akzeptable Salze oder Propharmaka davon.

15. [1,5-a]-Pyrazolo-1,3,5-triazine der Formel (1) 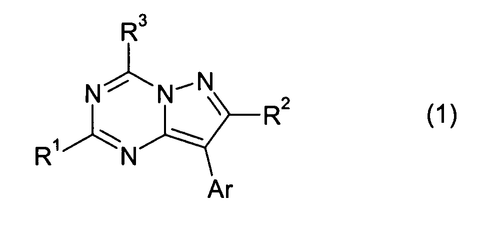 worin
Ar für Phenyl, Pyridyl oder 2,3-Dihydrobenzofuranyl steht, wobei jedes (Ar) wahlweise mit 1 bis 4 R⁴-Substituenten substituiert ist;
R¹ und R² jeweils unabhängig ausgewählt sind unter C₁-C₄-Alkyl;
R³ NR^{6a}R^{7a} ist;
R⁴ jeweils unabhängig ausgewählt ist unter C₁-C₁₀Alkyl, C₂-C₁₀-Alkenyl, C₂-C₁₀-Alkinyl, C₃-C₆-Cycloalkyl, C₄-C₁₂-Cycloalkylalkyl, NO₂, Halo, CN, C₁-C₄-Haloalkyl, NR⁶R⁷, NR⁸COR⁷, NR⁸CO₂R⁷, COR⁷, OR⁷, CONR⁶R⁷, CO(NOR⁹)R⁷, CO₂R⁷ oder S(O)ₙR⁷, ein jedes solches C₁-C₁₀Alkyl, C₂-C₁₀-Alkenyl, C₂-C₁₀-Alkinyl, C₃-C₆-Cycloalkyl und C₄-C₁₂-Cycloalkylalkyl wahlweise substituiert mit 1 bis 3 Substituenten, die jeweils unabhänig ausgewählt sind unter C₁-C₄-Alkyl, NO₂, Halo, CN, NR⁶R⁷, NR⁸COR⁷, NR⁸CO₂R⁷, COR⁷ OR⁷, CONR⁶R⁷, CO₂R⁷, CO(NOR⁹)R⁷ oder S(O)ₙR⁷;
R⁶ und R⁷, R^{6a} und R^{7a} jeweils unabhängig ausgewählt sind unter:
• H,
• C₁-C₁₀-Alkyl, C₃-C₁₀-Alkenyl, C₃-C₁₀-Alkinyl, C₁-C₁₀-Haloalkyl mit 1-10 Halogenen, C₂-C₈-Alkoxyalkyl, C₃-C₆-Cycloalkyl, C₄-C₁₂-Cycloalkylalkyl, C₅-C₁₀-Cycloalkenyl oder C₆-C₁₄-Cycloalkenylalkyl, jedes wahlweise substituiert mit 1 bis 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, Aryl, Heteroaryl oder Heterocyclyl,
• Aryl, Aryl(C₁-C₄-Alkyl), Heteroaryl, Heteroaryl(C₁-C₄-Alkyl), Heterocyclyl oder Heterocyclyl(C₁-C₄-Alkyl);
alternativerweise NR⁶R⁷ und NR^{6a}R^{7a} unabhängig für Piperidin, Pyrrolidin, Piperazin, N-Methylpiperazin, Morpholin oder Thiomorpholin stehen, jedes wahlweise mit 1 bis 3 C₁-C₄-Alkylgruppen substitutiert;
R⁸ jeweils unabhängig ausgewählt ist unter H oder C₁-C₄-Alkyl;
R⁹ und R¹⁰ jeweils unabhängig ausgewählt sind unter H, C₁-C₄-Alkyl, oder C₃-C₆-Cycloalkyl;
R¹¹ ausgewählt ist unter H, C₁-C₄-Alkyl, C₁-C₄-Haloalkyl, oder C₃-C₆-Cycloalkyl;
R¹² für C₁-C₄-Alkyl oder C₁-C₄-Haloalkyl steht;
R¹³ ausgewählt ist unter C₁-C₄-Alkyl, C₁-C₄-Haloalkyl, C₂-C₈-Alkoxyalkyl, C₃-C₆-Cycloalkyl, C₄-C₁₂-Cycloalkylalkyl, Aryl, Aryl(C₁-C₄-Alkyl)-, Heteroaryl oder Heteroaryl(C₁-C₄-Alkyl)-;
R¹⁴ ausgewählt ist unter C₁-C₁₀-Akyl, C₃-C₁₀-Alkenyl, C₃-C₁₀-Alkinyl, C₃-C₈-Cycloalkyl, oder C₄-C₁₂-Cycloalkylalkyl, jedes wahlweise substituiert mit 1 bis 3 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵, CONR¹⁶R¹⁵, und C₁-C₆-Alkylthio, C₁-C₆-Alkylsulfinyl und C₁-C₆-Alkylsulfonyl;
R¹⁵ und R¹⁶ jeweils unabhängig ausgewählt sind unter H, C₁-C₆-Alkyl, C₃-C₁₀-Cycloalkyl, C₄-C₁₆-Cycloalkylalkyl, mit der Maßgabe, dass R¹⁵ in S(O)ₙR¹⁵ nicht H sein kann;
wobei Aryl Phenyl oder Naphthyl ist, wobei jedes wahlweise substituiert ist mit 1 bis 5 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵ und CONR¹⁶R¹⁵;
Heteroaryl Pyridyl, Pyrimidinyl, Triazinyl, Furanyl, Pyranyl, Chinolinyl, Isochinolinyl, Thienyl, Imidazolyl, Thiazolyl, Indolyl, Pyrrolyl, Oxazolyl, Benzofuranyl, Benzothienyl, Benzothiazolyl, Isoxazolyl, Pyrazolyl, 2,3-Dihydrobenzothienyl oder 2,3-Dihydrobenzofuranyl ist, wobei jedes wahlweise substituiert ist mit 1 bis 5 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, -COR¹⁵, CO₂R¹⁵, CO(O)R¹⁵, NR⁸COR¹⁵, N(COR¹⁵)₂, NR⁸CONR¹⁶R¹⁵, NR⁸CO₂R¹⁵, NR¹⁶R¹⁵ und CONR¹⁶R¹⁵;
Heterocyclyl gesättigtes oder partiell gesättigtes Heteroaryl ist, wahlweise substituiert mit 1 bis 5 Substituenten, die jeweils unabhängig ausgewählt sind unter C₁-C₆-Alkyl, C₃-C₆-Cycloalkyl, Halo, C₁-C₄-Haloalkyl, Cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO_{2R}¹⁵, NR¹⁵R¹⁶ und CONR¹⁶R¹⁵;
n jeweils unabhängig 0, 1 oder 2 ist,
und geometrische Isomere davon, stereoisomere Formen davon, oder Gemische stereoisomerer Formen davon, und pharmazeutisch akzeptable Salze oder Propharmaka davon.

16. [1,5-a]-Pyrazolo-1,3,5-triazine nach Anspruch 15, wobei
Ar Phenyl, Pyridyl oder 2,3-Dihydrobenzofuranyl ist, wobei jedes (Ar) mit 1 bis 4 R⁴-Substituenten substituiert ist,
und geometrische Isomere davon, stereoisomere Formen davon, oder Gemische stereoisomerer Formen davon, und pharmazeutisch akzeptable Salze oder Propharmaka davon.

17. [1,5-a]-Pyrazolo-1,3,5-triazine nach Anspruch 15 oder 16, wobei
Ar Phenyl oder Pyridyl ist, wobei jedes (Ar) wahlweise mit 1 bis 4 R⁴-Substituenten substituiert ist,
und geometrische Isomere davon, stereoisomere Formen davon, oder Gemische stereoisomerer Formen davon, und pharmazeutisch akzeptable Salze oder Propharmaka davon.

18. [1,5-a]-Pyrazolo-1,3,5-triazine nach einem der Ansprüche 15 bis 17, wobei
R^{6a} und R^{7a} unabhängig H oder C₁-C₁₀-Alkyl sind,
und geometrische Isomere davon, stereoisomere Formen davon, oder Gemische stereoisomerer Formen davon, und pharmazeutisch akzeptable Salze oder Propharmaka davon.

19. Pharmazeutische Zusammensetzung, umfassend ein [1,5-a]-Pyrazolo-1,3,5-triazin der Formel (1) nach einem der Ansprüche 1 bis 18 oder ein geometrisches Isomer davon, eine stereoisomere Form davon, oder ein Gemisch stereoisomerer Formen davon, oder ein pharmazeutisch akzeptables Salz oder Propharmakon davon.

20. Verwendung eines [1,5-a]-Pyrazolo-1,3,5-triazins der Formel (1) nach einem der Ansprüche 1 bis 18 oder eines geometrischen Isomers davon, einer stereoisomeren Form davon, oder eines Gemisches stereoisomerer Formen davon, oder eines pharmazeutisch akzeptablen Salzes oder Propharmakons davon, bei der Herstellung eines Medikaments zur Behandlung von Gemütskrankheit, Angst, Depression, Kopfschmerz, Reizdarmsyndrom, post-traumatischer Stresserkrankung, supranukleärer Lähmung, Immunsuppression, Alzheimer-Krankheit, gastrointestinalen Erkrankungen, Anorexia nervosa und anderen Essstörungen, Drogenabhängigkeit, Drogen- oder Alkohol-Entzugssymptomen, entzündlichen Erkrankungen, cardiovaskulären oder das Herz betreffenden Erkrankungen, Fertilitätsproblemen, HIV-Infektionen, hämorrhagischem Stress, Obesität, Unfruchtbarkeit, Hirn- und Rückenmarkstraumata, Epilepsie, Schlaganfall, Geschwüren, Amyotropher Lateralsklerose oder Hypoglykämie.

21. Verwendung nach Anspruch 20, zur Behandlung von Gemütskrankheit, Angst, Depression, Reizdarmsyndrom, post-traumatischer Stresserkrankung, supranukleärer Lähmung, Immunsuppression, Alzheimer-Krankheit, gastrointestinaler Erkrankungen, Anorexia nervosa oder anderen Essstörungen, Drogenabhängigkeit, Drogen- oder Alkoholentzugssymptomen, entzündlichen Erkrankungen oder Fertilitätsproblemen.

22. Verwendung nach Anspruch 21, zur Behandlung von Angst.

## Revendications

1. [1,5-a]-pyrazolo-1,3,5-triazines de formule (1) 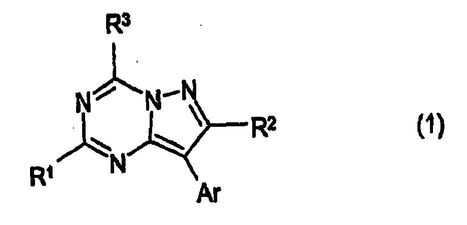 dans laquelle
Ar est phényle, pyridyle ou 2,3-dihydrobenzofuranyle, chaque (Ar) étant le cas échéant substitué par 1 à 4 substituants R⁴;
R¹ est, indépendamment, sélectionné à chaque occurrence parmi H, alkyle en C₁-C₄, alcényle en C₂-C₄, alcynyle en C₂-C₄, halogène, CN, halogénoalkyle en C₁-C₄, hydroxyalkyle en C₁-C₁₂, alcoxyalkyle en C₂-C₁₂, cyanoalkyle en C₂-C₁₀, cycloalkyle en C₃-C₆, cycloalkylalkyle en C₄-C₁₀, NR⁹R¹⁰, alkyle en C₁-C₄-NR⁹R¹⁰, NR⁹COR¹⁰, OR¹¹, SH ou S(O)ₙR¹²;
R² est sélectionné parmi H, alkyle en C₁-C₄, alcényle en C₂-C₄, alcynyle en C₂-C₄, cycloalkyle en C₃-C₆, cycloalkylalkyle en C₄-C₁₀, hydroxyalkyle en C₁-C₄, halogène, CN, -NR⁶R⁷, NR⁹COR¹⁰, -NR⁶S(O)ₙR⁷, S(O)ₙNR⁶R⁷, halogénoalkyle en C₁-C₄, -OR⁷, SH ou S(O)ₙR¹² ;
R³ est NR^{6a}R^{7a} ;
R⁴ est, indépendamment, sélectionné à chaque occurrence parmi : alkyle en C₁-C₁₀, alcényle en C₂-C₁₀, alcynyle en C₂-C₁₀, cycloalkyle en C₃-C₆, cycloalkylalkyle en C₄-C₁₂, NO₂, halogène, CN, halogénoalkyle en C₁-C₄, NR⁶R⁷, NR⁸COR⁷, NR⁶CO₂R⁷, COR⁷, OR⁷, CONR⁶R⁷, CO(NOR⁹)R⁷, CO₂R⁷, ou S(O)ₙR⁷, où chaque alkyle en C₁-C₁₀, alcényle en C₂-C₁₀, alcynyle en C₂-C₁₀, cycloalkyle en C₃-C₆ et cycloalkylalkyle en C₄-C₁₂ de ce type est le cas échéant substitué par 1 à 3 substituants indépendamment sélectionnés à chaque occurrence parmi alkyle en C₁-C₄, NO₂, halogène, CN, NR⁶R⁷, NR⁸COR⁷, NR⁸CO₂R⁷, COR⁷, OR⁷, CONR⁶R⁷, CO₂R⁷, CO(NOR⁹)R⁷, ou S(O)ₙR⁷ ;
R⁶ et R⁷, R^{6a} et R^{7a} sont indépendamment sélectionnés à chaque occurrence parmi :
• H,
• alkyle en C₁-C₁₀, alcényle en C₃-C₁₀, alcynyle en C₃-C₁₀, halogénoalkyle en C₁-C₁₀ avec 1 à 10 halogènes, alcoxyalkyle en C₂-C₈, cycloalkyle en C₃-C₆, cycloalkylalkyle en C₄-C₁₂, cycloalcényle en C₅-C₁₀ ou cycloalcénylalkyle en C₈-C₁₄, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³ , NR⁸COR¹⁵ , N(COR¹⁵)₂, NR⁸CONR¹⁶R¹⁵ , NR⁸CO₂R¹³, NR¹⁶R¹⁵ , CONR¹⁶R¹⁵, aryle, hétéroaryle ou hétérocyclyle,
• aryle, aryl(alkyle en C₁-C₄), hétéroaryle, hétéroaryl(alkyle en C₁-C₄), hétérocyclyle ou hétérocyclyl(alkyle en C₁-C₄) ;
en variante, NR⁶R⁷ et NR^{6a}R^{7a} sont indépendamment pipéridine, pyrrolidine, pipérazine, N-méthylpipérazine, morpholine ou thiomorpholine, chacun le cas échéant substitué par 1 à 3 groupes alkyle en C₁-C₄ ;
R⁸ est indépendamment sélectionné à chaque occurrence parmi H ou alkyle en C₁-C₄ ;
R⁹ et R¹⁰ sont, indépendamment, sélectionnés à chaque occurrence parmi H, alkyle en C₁-C₄ ou cycloalkyle en C₃-C₆;
R¹¹ est sélectionné parmi H, alkyle en C₁-C₄, halogénoalkyle en C₁-C₄ ou cycloalkyle en C₃-C₆ ;
R¹² est alkyle en C₁-C₄ ou halogénoalkyle en C₁-C₄;
R¹³ est sélectionné parmi alkyle en C₁-C₄, halogénoalkyle en C₁-C₄, alcoxyalkyle en C₂-C₈, cycloalkyle en C₃-C₆, cycloalkylalkyle en C₄-C₁₂, aryle, aryl(alkyle en C₁-C₄), hétéroaryle ou hétéroaryl(alkyle en C₁-C₄),
R¹⁴ est sélectionné parmi alkyle en C₁-C₁₀, alcényle en C₃-C₁₀, alcynyle en C₃-C₁₀, cycloalkyle en C₃-C₈ ou cycloalkylalkyle en C₄-C₁₂, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁸, NR⁸CO₂R¹⁵, NR¹⁶R¹⁵, CONR¹⁶R¹⁵ et alkylthio en C₁-C₆, alkylsulfinyle en C₁-C₆ et alkylsulfonyle en C₁-C₆;
R¹⁵ et R¹⁶ sont, indépendamment, sélectionnés à chaque occurrence parmi H, alkyle en C₁-C₆, cycloalkyle en C₃-C₁₀, cycloalkylalkyle en C₄-C₁₆, sauf que, pour S(O)ₙR¹⁵, R¹⁵ ne peut être H ;
aryle est phényle ou naphtyle, chacun le cas échéant substitué par 1 à 5 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵ et CONR¹⁶R¹⁵;
hétéroaryle est pyridyle, pyrimidinyle, triazinyle, furanyle, pyranyle, quinolinyle, isoquinolinyle, thiényle, imidazolyle, thiazolyle, indolyle, pyrrolyle, oxazolyle, benzofuranyle, benzothiényle, benzothiazolyle, isoxazolyle, pyrazolyle, 2,3-dihydrobenzothiényle ou 2,3-dihydrobenzofuranyle, chacun étant le cas échéant substitué par 1 à 5 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, -COR¹⁵, CO₂R¹⁵, CO(O)R¹⁵ NR⁸COR¹⁵, N(COR¹⁵)₂, NR⁸CONR¹⁶R¹⁵, NR⁸CO₂R¹⁵, NR¹⁶R¹⁵ et CONR¹⁶R¹⁵ ;
hétérocyclyle est un hétéroaryle saturé ou partiellement saturé, le cas échéant substitué par 1 à 5 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁵R¹⁶ et CONR¹⁶R¹⁵ ;
n est indépendamment à chaque occurrence 0, 1 ou 2,
à la condition que :
(1) quand R¹ est méthyle ou éthyle, R² est H et R³ est NH(n-C₄H₉) ou N(C₂H₅)₂, alors Ar n'est pas un phényle ou m-méthylphényle non substitué ; et
(2) quand R² est H et Ar est pyridyle et R³ est NR^{6a}R^{7a}, alors R^{6a} et R^{7a} ne sont pas H ou alkyle,
et leurs isomères géométriques, leurs formes stéréoisomériques ou mélanges de leurs formes stéréoisomériques et leurs sels pharmaceutiquement acceptables ou promédicaments.

2. [1,5-a]-pyrazolo-1,3,5-triazines selon la revendication 1, dans lesquelles
Ar est phényle, pyridyle ou 2,3-dihydrobenzofuranyle, chaque (Ar) étant substitué par 1 à 4 substituants R⁴,
et leurs isomères géométriques, leurs formes stéréoisomériques ou mélanges de leurs formes stéréoisomériques et leurs sels pharmaceutiquement acceptables ou promédicaments.

3. [1,5-a]-pyrazolo-1,3,5-triazines selon la revendication 1 ou 2, dans lesquelles
Ar est phényle ou pyridyle, chaque (Ar) étant le cas échéant substitué par 1 à 4 substituants R⁴,
et leurs isomères géométriques, leurs formes stéréoisomériques ou mélanges de leurs formes stéréoisomériques et leurs sels pharmaceutiquement acceptables ou promédicaments.

4. [1,5-a]-pyrazolo-1,3,5-triazines selon l'une quelconque des revendications 1 à 3, dans lesquelles
R¹ et R² sont, indépendamment, sélectionnés parmi H, alkyle en C₁-C₄, cycloalkyle en C₃-C₆, cycloalkylalkyle en C₄-C₁₀,
et leurs isomères géométriques, leurs formes stéréoisomériques ou mélanges de leurs formes stéréoisomériques et leurs sels pharmaceutiquement acceptables ou promédicaments.

5. [1,5-a]-pyrazolo-1,3,5-triazines selon la revendication 4, dans lesquelles
R¹ et R² sont, indépendamment, sélectionnés parmi H ou alkyle en C₁-C₄,
et leurs isomères géométriques, leurs formes stéréoisomériques ou mélanges de leurs formes stéréoisomériques et leurs sels pharmaceutiquement acceptables ou promédicaments.

6. [1,5-a]-pyrazolo-1,3,5-triazines selon la revendication 4, dans lesquelles
R¹ et R² sont, indépendamment, sélectionnés parmi alkyle en C₁-C₄,
et leurs isomères géométriques, leurs formes stéréoisomériques ou mélanges de leurs formes stéréoisomériques et leurs sels pharmaceutiquement acceptables ou promédicaments.

7. [1,5-a]-pyrazolo-1,3,5-triazines selon la revendication 4, dans lesquelles
R¹ est méthyle
et leurs isomères géométriques, leurs formes stéréoisomériques ou mélanges de leurs formes stéréoisomériques et leurs sels pharmaceutiquement acceptables ou promédicaments.

8. [1,5-a]-pyrazolo-1,3,5-triazines selon l'une quelconque des revendications 1 à 7, dans lesquelles
R^{6a} est indépendamment sélectionné parmi
• H,
• alkyle en C₁-C₁₀, alcényle en C₃-C₁₀, alcynyle en C₃-C₁₀, halogénoalkyle en C₁-C₁₀ avec 1 à 10 halogènes, alcoxyalkyle en C₂-C₈, cycloalkyle en C₃-C₆, cycloalkylalkyle en C₄-C₁₂, cycloalcényle en C₅-C₁₀ ou cycloalcénylalkyle en C₆-C₁₄, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryle, hétéroaryle ou hétérocyclyle,
• aryle, aryl(alkyle en C₁-C₄), hétéroaryle, hétéroaryl(alkyle en C₁-C₄)-, hétérocyclyle ou hétérocyclyl(alkyle en C₁-C₄)- ; et
R^{7a} est indépendamment sélectionné à chaque occurrence parmi
• H,
• alkyle en C₅-C₁₀, alcényle en C₃-C₁₀, alcynyle en C₃-C₁₀, halogénoalkyle en C₁-C₁₀ avec 1 à 10 halogènes, alcoxyalkyle en C₂-C₈, cycloalkyle en C₃-C₆, cycloalkylalkyle en C₄-C₁₂, cycloalcényle en C₅-C₁₀ ou cycloalcénylalkyle en C₆-C₁₄, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵*,* NR⁸CO₂R¹³, NR¹⁶R¹⁵*,* CONR¹⁶R¹⁵, aryle, hétéroaryle ou hétérocyclyle,
• aryle, aryl(alkyle en C₁-C₄)-, hétéroaryle, hétéroaryl(alkyle en C₁-C₄)-, hétérocyclyle ou hétérocyclyl(alkyle en C₁-C₄)- ; et
en variante, NR⁶R⁷ et NR^{6a}R^{7a} sont indépendamment pipéridine, pyrrolidine, pipérazine, N-méthylpipérazine, morpholine ou thiomorpholine, chacun le cas échéant substitué par 1 à 3 groupes alkyle en C₁-C₄,
et leurs isomères géométriques, leurs formes stéréoisomériques ou mélanges de leurs formes stéréoisomériques et leurs sels pharmaceutiquement acceptables ou promédicaments.

9. [1,5-a]-pyrazolo-1,3,5-triazines selon l'une quelconque des revendications 1 à 7, dans lesquelles
R^{6a} est sélectionné parmi
• H,
• alkyle en C₁-C₁₀, alcényle en C₃-C₁₀, alcynyle en C₃-C₁₀, halogénoalkyle en C₁-C₁₀ avec 1 à 10 halogènes, alcoxyalkyle en C₂-C₈, cycloalkyle en C₃-C₆, cycloalkylalkyle en C₄-C₁₂, cycloalcényle en C₅-C₁₀ ou cycloalcénylalkyle en C₆-C₁₄, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryle, hétéroaryle ou hétérocyclyle,
• aryle, aryl(alkyle en C₁-C₄)-, hétéroaryle, hétéroaryl(alkyle en C₁-C₄)-, hétérocyclyle ou hétérocyclyl(alkyle en C₁-C₄)- ;
R^{7a} est sélectionné parmi
• alkyle en C₁-C₄, et chaque alkyle en C₁-C₄ de ce type est substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryle, hétéroaryle ou hétérocyclyle,
et leurs isomères géométriques, leurs formes stéréoisomériques ou mélanges de leurs formes stéréoisomériques et leurs sels pharmaceutiquement acceptables ou promédicaments.

10. [1,5-a]-pyrazolo-1,3,5-triazines selon l'une quelconque des revendications 1 à 7, dans lesquelles
R^{6a} est H,
R^{7a} est sélectionné parmi
• alkyle en C₁-C₄, et chaque alkyle en C₁-C₄ de ce type est substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryle, hétéroaryle ou hétérocyclyle,
et leurs isomères géométriques, leurs formes stéréoisomériques ou mélanges de leurs formes stéréoisomériques et leurs sels pharmaceutiquement acceptables ou promédicaments.

11. [1,5-a]-pyrazolo-1,3,5-triazines selon l'une quelconque des revendications 1 à 7, dans lesquelles
R^{6a} et R^{7a} sont indépendamment H ou alkyle en C₁-C₁₀, chaque alkyle en C₁-C₁₀ de ce type est le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryle, hétéroaryle ou hétérocyclyle,
et leurs isomères géométriques, leurs formes stéréoisomériques ou mélanges de leurs formes stéréoisomériques et leurs sels pharmaceutiquement acceptables ou promédicaments.

12. [1,5-a]-pyrazolo-1,3,5-triazines selon l'une quelconque des revendications 1 à 7, dans lesquelles
R^{6a} et R^{7a} sont indépendamment H ou alkyle en C₁-C₁₀,
et leurs isomères géométriques, leurs formes stéréoisomériques ou mélanges de leurs formes stéréoisomériques et leurs sels pharmaceutiquement acceptables ou promédicaments.

13. [1,5-a]-pyrazolo-1,3,5-triazines selon la revendication 1 de formule (50) 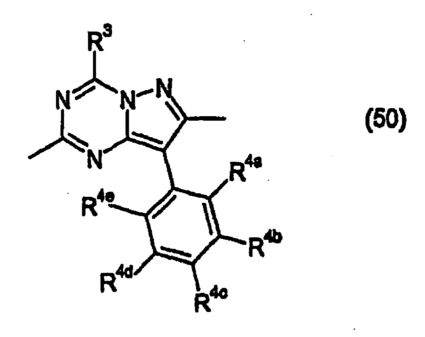 dans laquelle le composé est sélectionné dans le groupe constitué de :
• un composé de formule (50), dans laquelle R³ est -NHCH(n-Pr)₂, R^{4a} est Cl, R^{4b} est H, R^{4c} est Cl, R^{4d} est H et R^{4e} est H ;
• un composé de formule (50), dans laquelle R³ est -N(Et)(n-Bu), R^{4a} est Cl, R^{4b} est H, R^{4c} est Cl, R^{4d} est H et R^{4e} est H ;
• un composé de formule (50), dans laquelle R³ est -N(CH₂CH₂OMe)₂, R^{4a} est Cl, R^{4b} est H, R^{4c} est Cl, R^{4d} est H et R^{4e} est H ;
• un composé de formule (50), dans laquelle R³ est -NHCH(Et)(n-Bu), R^{4a} est Cl, R^{4b} est H, R^{4c} est Cl, R^{4d} est H et R^{4e} est H ;
• un composé de formule (50), dans laquelle R³ est NHCH(Et)(CH₂OMe), R^{4a} est Cl, R^{4b} est H, R^{4c} est Cl, R^{4d} est H et R^{4e} est H ;
• un composé de formule (50), dans laquelle R³ est -NHCH(CH₂OMe)₂, R^{4a} est Cl, R^{4b} est H, R^{4c} est Cl, R^{4d} est H et R^{4e} est H ;
• un composé de formule (50), dans laquelle R³ est -N(Et)₂, R^{4a} est Cl, R^{4b} est H, R^{4c} est Cl, R^{4d} est H et R^{4e} est H,
• un composé de formule (50), dans laquelle R³ est NHCH(CH₂OEt)₂, R^{4a} est Cl, R^{4b} est H, R^{4c} est Cl, R^{4d} est H et R^{4e} est H ;
• un composé de formule (50), dans laquelle R³ est NHCH(Et)₂, R^{4a} est Cl, R^{4b} est H, R^{4c} est Cl, R^{4d} est H et R^{4e} est H ;
• un composé de formule (50), dans laquelle R³ est -N(Me)(Ph), R^{4a} est Cl, R^{4b} est H, R^{4c} est Cl, R^{4d} est H et R^{4e} est H ;
• un composé de formule (50), dans laquelle R³ est -N(n-Pr)₂, R^{4a} est Cl, R^{4b} est H, R^{4c} est Cl, R^{4a} est H et R^{4e} est H ;
• un composé de formule (50), dans laquelle R³ est -NHCH(Et)(n-Pr), R^{4a} est Cl, R^{4b} est H, R^{4c} est Cl, R^{4d} est H et R^{4e} est H ;
• un composé de formule (50), dans laquelle R³ est -NHCH(CH₂OMe)₂, R^{4a} est Me, R^{4b} est H, R^{4c} est Me, R^{4d} est H et R^{4e} est Me ;
• un composé de formule (50), dans laquelle R³ est -NHCH(CH₂OMe)₂, R^{4a} est Me, R^{4b} est H, R^{4c} est Me, R^{4d} est H et R^{4e} est H ;
• un composé de formule (50), dans laquelle R³ est -N(CH₂CH₂OMe)₂, R^{4a} est Me, R^{4b} est H, R^{4c} est Me, R^{4d} est H et R^{4e} est H ;
• un composé de formule (50), dans laquelle R³ est -NHCH(Et)(CH₂OMe), R^{4a} est Me, R^{4b} est H, R^{4c} est Me, R^{4d} est H et R^{4e} est H ;
• un composé de formule (50), dans laquelle R³ est -NHCH(Et)₂, R^{4a} est Me, R^{4b} est H, R^{4c} est Me, R^{4d} est H et R^{4e} est H ;
• un composé de formule (50), dans laquelle R³ est -N(Et)₂, R^{4a} est Me, R^{4b} est H, R^{4c} est Me, R^{4d} est H et R^{4e} est H ;
• un composé de formule (50), dans laquelle R³ est -N(CH₂CN)₂, R^{4a} est Me, R^{4b} est H, R^{4c} est Me, R^{4a} est H et R^{4e} est H ;
• un composé de formule (50), dans laquelle R³ est -NHCH(Me)(CH₂OMe), R^{4a} est Me, R^{4b} est H, R^{4c} est Me, R^{4d} est H et R^{4e} est H ;
• un composé de formule (50), dans laquelle R³ est -N(n-Pr)(CH₂c-Pr), R^{4a} est Me, R^{4b} est H, R^{4c} est Me, R^{4d} est H et R^{4e} est H ;
• un composé de formule (50), dans laquelle R³ est -NHCH(Me)(CH₂N(Me)₂), R^{4a} est Me, R^{4b} est H, R^{4c} est Me, R^{4d} est H et R^{4e} est H ;
• un composé de formule (50), dans laquelle R³ est -N(c-Pr)(CH₂CH₂CN), R^{4a} est Me, R^{4b} est H, R^{4c} est Me, R^{4a} est H et R^{4e} est H ;
• un composé de formule (50), dans laquelle R³ est -N(n-Pr)(CH₂CH₂CN), R^{4a} est Me, R^{4b} est H, R^{4c} est Me, R^{4d} est H et R^{4e} est H ;
• un composé de formule (50), dans laquelle R³ est -N(n-Bu)(CH₂CN), R^{4a} est Me, R^{4b} est H, R^{4c} est Me, R^{4d} est H et R^{4e} est H ;
• un composé de formule (50), dans laquelle R³ est -NHCH(Et)(CH₂OMe), R^{4a} est Me, R^{4b} est H, R^{4c} est Me, R^{4d} est H et R^{4c} est Me ;
• un composé de formule (50), dans laquelle R³ est -NHCH(Et)₂, R^{4a} est Me, R^{4b} est H, R^{4c} est Me, R^{4d} est H et R^{4e} est Me ;
• un composé de formule (50), dans laquelle R³ est -N(CH₂CH₂OMe)₂, R^{4a} est Me, R^{4b} est H, R^{4c} est Me, R^{4d} est H et R^{4e} est Me ;
• un composé de formule (50), dans laquelle R³ est -NHCH(CH₂OMe)₂, R^{4a} est Br, R^{4b} est H, R^{4c} est OMe, R^{4d} est H et R^{4e} est H ;
• un composé de formule (50), dans laquelle R³ est -NHCH(Et)(CH₂OMe), R^{4a} est Br, R^{4b} est H, R^{4c} est OMe, R^{4d} est H et R^{4e} est H ;
• un composé de formule (50), dans laquelle R³ est -N(Et)₂, R^{4a} est Me, R^{4b} est H, R^{4c} est Me, R^{4d} est H et R^{4e} est Me;
• un composé de formule (50), dans laquelle R³ est -NHCH(CH₂OEt)₂, R^{4a} est Me, R^{4b} est H, R^{4c} est Me, R^{4d} est H et R^{4e} est Me ;
• un composé de formule (50), dans laquelle R³ est-NHCH(CH₂CH₂OMe)(CH₂OMe)₂, R^{4a} est Me, R^{4b} est H, R^{4c} est Me, R^{4d} est H et R^{4e} est Me ;
• un composé de formule (50), dans laquelle R³ est morpholino, R^{4a} est Me, R^{4b} est H, R^{4c} est Me, R^{4d} est H et R^{4e} est H ;
• un composé de formule (50), dans laquelle R³ est -N(CH₂CH₂OMe)₂, R^{4a} est Br, R^{4b} est H, R^{4c} est OMe, R^{4d} est H et R^{4e} est H ;
• un composé de formule (50), dans laquelle R³ est NHCH(Et)₂, R^{4a} est Br, R^{4b} est H, R^{4c} est OMe, R^{4d} est H et R^{4e} est H ;
• un composé de formule (50), dans laquelle R³ est -N(Et)₂, R^{4a} est Br, R^{4b} est H, R^{4c} est OMe, R^{4d} est H et R^{4e} est H ;
• un composé de formule (50), dans laquelle R³ est -NH(c-Pr), R^{4a} est Me, R^{4b} est H, R^{4c} est Me, R^{4d} est H et R^{4e} est H ;
• un composé de formule (50), dans laquelle R³ est -NHCH(CH₂OMe)₂, R^{4a} est CN, R^{4b} est H, R^{4c} est OMe, R^{4d} est H et R^{4e} est H ;
• un composé de formule (50), dans laquelle R³ est -N(c-Pr)(CH₂CH₂CN), R^{4a} est Me, R^{4b} est H, R^{4c} est Me, R^{4d} est H et R^{4e} est Me ;
• un composé de formule (50), dans laquelle R³ est -NCH(CH₂OMe)₂, R^{4a} est Me, R^{4b} est H, R^{4c} est Br, R^{4d} est H et R^{4e} est H ;
• un composé de formule (50), dans laquelle R³ est -NHCH(CH₂OMe) (CH₂CH₂OMe), R^{4a} est Me, R^{4b} est H, R^{4c} est Br, R^{4d} est H et R^{4e} est H ;
• un composé de formule (50), dans laquelle R³ est -NHCH(CH₂OMe)₂, R^{4a} est Me, R^{4b} est H, R^{4c} est OMe, R^{4d} est Me et R^{4e} est H ;
• un composé de formule (50), dans laquelle R³ est -N(CH₂CH₂OMe)₂, R^{4a} est Me, R^{4b} est H, R^{4c} est OMe, R^{4d} est Me et R^{4e} est H ;
• un composé de formule (50), dans laquelle R³ est -NHCH(Et)₂, R^{4a} est Me, R^{4b} est H, R^{4c} est OMe, R^{4d} est Me et R^{4e} est H ;
• un composé de formule (50), dans laquelle R³ est -N(Et)₂, R^{4a} est Me, R^{4b} est H, R^{4c} est OMe, R^{4d} est Me et R^{4e} est H ;
• un composé de formule (50), dans laquelle R³ est -NHCH(CH₂OMe)₂, R^{4a} est Cl, R^{4b} est H, R^{4c} est Me, R^{4d} est H et R^{4e} est H ;
• un composé de formule (50), dans laquelle R³ est -NHCH(Et)(CH₂OMe), R^{4a} est Cl, R^{4b} est H, R^{4c} est Me, R^{4d} est H et R^{4e} est H ;
• un composé de formule (50), dans laquelle R³ est -N(CH₂CH₂OMe)₂, R^{4a} est Cl, R^{4b} est H, R^{4c} est Me, R^{4d} est H et R^{4e} est H ;
• un composé de formule (50), dans laquelle R³ est -NHCH(CH₂OMe) (CH₂CH₂OMe), R^{4a} est Cl, R^{4b} est H, R^{4c} est Me, R^{4d} est H et R^{4e} est H ;
• un composé de formule (50), dans laquelle R³ est -N(c-Pr)(CH₂CH₂CN), R^{4a} est Me, R^{4b} est H, R^{4c} est OMe, R^{4d} est Me et R^{4e} est H ;
• un composé de formule (50), dans laquelle R³ est -N(c-Pr)(CH₂CH₂CN), R^{4a} est Cl, R^{4b} est H, R^{4c} est Cl, R^{4d} est H et R^{4e} est H ;
• un composé de formule (50), dans laquelle R³ est (S)-NHCH(CH₂OMe) (CH₂CH₂OMe), R^{4a} est Cl, R^{4b} est H, R^{4c} est Cl, R^{4d} est H et R^{4e} est H ;
• un composé de formule (50), dans laquelle R³ est -NHCH(CH₂OMe) (CH_{2C}H₂OMe), R^{4a} est Cl, R^{4b} est H, R^{4c} est Cl, R^{4d} est H et R^{4e} est H ;
• un composé de formule (50), dans laquelle R³ est -NHCH(Et)₂, R^{4a} est Me, R^{4b} est H, R^{4c} est Br, R^{4d} est H et R^{4e} est H ;
• un composé de formule (50), dans laquelle R³ est -N(CH₂CH₂OMe)₂ R^{4a} est Me, R^{4b} est H, R^{4c} est Br, R^{4d} est H et R^{4e} est H ;
• un composé de formule (50), dans laquelle R³ est -NH(CH₂OMe) (CH₂-I-Pr), R^{4a} est Me, R^{4b} est H, R^{4c} est Me, R^{4d} est H et R^{4e} est H ;
• un composé de formule (50), dans laquelle R³ est -N(CH₂CH₂OMe)₂, R^{4a} est Me, R^{4b} est H, R^{4c} est H, R^{4d} est H et R^{4e} est H ;
• un composé de formule (50), dans laquelle R³ est -N(CH₂CH₂OMe)₂, R^{4a} est Me, R^{4b} est H, R^{4c} est NMe₂, R^{4d} est H et R^{4e} est H ;
• un composé de formule (50), dans laquelle R³ est -NHCH(CH₂OMe)(n-Pr), R^{4a} est Me, R^{4b} est H, R^{4c} est Me, R^{4d} est H et R^{4e} est H ;
• un composé de formule (50), dans laquelle R³ est -NHCH(CH₂OEt)(Et), R^{4a} est Me, R^{4b} est H, R^{4c} est Me, R^{4d} est H et R^{4e} est H ;
• un composé de formule (50), dans laquelle R³ est -NHCH(CH₂OMe) (CH₂CH₂OMe), R^{4a} est Me, R^{4b} est H, R^{4c} est NMe₂, R^{4d} est H et R^{4e} est H ;
• un composé de formule (50), dans laquelle R³ est -N(Et)₂, R^{4a} est Me, R^{4b} est H, R^{4c} est Cl, R^{4d} est H et R^{4e} est H ;
• un composé de formule (50), dans laquelle R³ est -NHCH(Et)₂, R^{4a} est Me, R^{4b} est H, R^{4c} est Cl, R^{4d} est H et R^{4e} est H ;
• un composé de formule (50), dans laquelle R³ est -N(CH₂CH₂OMe)₂, R^{4a} est Me, R^{4b} est H, R^{4c} est Cl, R^{4d} est H et R^{4e} est H ;
• un composé de formule (50), dans laquelle R³ est -NHCH(CH₂OMe)₂, R^{4a} est Me, R^{4b} est H, R^{4c} est Cl, R^{4d} est H et R^{4e} est H ;
• un composé de formule (50), dans laquelle R³ est -N(Et)₂, R^{4a} est Me, R^{4b} est H, R^{4c} est Br, R^{4d} est H et R^{4e} est H ;
• un composé de formule (50), dans laquelle R³ est -N(Et)₂, R^{4a} est Cl, R^{4b} est H, R^{4c} est Me, R^{4d} est H et R^{4e} est H ;
• un composé de formule (50), dans laquelle R³ est -NHCH(Et)₂, R^{4a} est Cl, R^{4b} est H, R^{4c} est Me, R^{4d} est H et R^{4e} est H ;
• un composé de formule (50), dans laquelle R³ est -NHCH(Et)₂, R^{4a} est Me, R^{4b} est H, R^{4c} est NMe₂, R^{4d} est H et R^{4e} est H ;
• un composé de formule (50), dans laquelle R³ est (S)-NHCH(CH₂OMe) (CH₂CH₂OMe), R^{4a} est Me, R^{4b} est H, R^{4c} est Me, R^{4d} est H et R^{4e} est H ;
• un composé de formule (50), dans laquelle R³ est -NHCH(CH₂OMe) (CH₂CH₂OMe), R^{4a} est Me, R^{4b} est H, R^{4c} est Me, R^{4d} est H et R^{4e} est H ;
• un composé de formule (50), dans laquelle R³ est (S)-NHCH(CH₂OMe) (CH₂CH₂OMe), R^{4a} est Me, R^{4b} est H, R^{4c} est Cl, R^{4d} est H et R^{4e} est H ;
• un composé de formule (50), dans laquelle R³ est -NHCH(CH₂OMe) (CH₂CH₂OMe), R^{4a} est Me, R^{4b} est H, R^{4c} est Cl, R^{4d} est H et R^{4e} est H ;
• un composé de formule (50), dans laquelle R³ est -N(c-Pr)(CH₂CH₂CN), R^{4a} est Me, R^{4b} est H, R^{4c} est Cl, R^{4d} est H et R^{4e} est H ;
• un composé de formule (50), dans laquelle R³ est -NH(Et)(CH₂CN), R^{4a} est Me, R^{4b} est H, R^{4c} est Cl, R^{4d} est H et R^{4e} est H ;
• un composé de formule (50), dans laquelle R³ est -N(Et)₂, R^{4a} est Me, R^{4b} est Me, R^{4c} est OMe, R^{4d} est H et R^{4e} est H ;
• un composé de formule (50), dans laquelle R³ est -N(CH₂CH₂OMe) (CH₂CH₂OH), R^{4a} est Cl, R^{4b} est H, R^{4c} est Cl, R^{4d} est H et R^{4e} est H;
• un composé de formule (50), dans laquelle R³ est -N(CH₂CH₂OMe)₂, R^{4a} est Me, R^{4b} est Me, R^{4c} est OMe, R^{4d} est H et R^{4e} est H;
• un composé de formule (50), dans laquelle R³ est -NHCH(Et)₂, R^{4a} est Me, R^{4b} est Me, R^{4c} est OMe, R^{4d} est H et R^{4e} est H;
• un composé de formule (50), dans laquelle R³ est -N(CH₂c-Pr)(n-Pr), R^{4a} est Me, R^{4b} est H, R^{4c} est Cl, R^{4d} est H et R^{4e} est H;
• un composé de formule (50), dans laquelle R³ est -N(c-Pr)(CH₂CH₂CN), R^{4a} est Me, R^{4b} est Me, R^{4c} est OMe, R^{4d} est H et R^{4e} est H;
• un composé de formule (50), dans laquelle R³ est -NHCH(Et)₂, R^{4a} est Cl, R^{4b} est H, R^{4c} est OMe, R^{4d} est H et R^{4e} est H ;
• un composé de formule (50), dans laquelle R³ est -N(Et)₂, R^{4a} est Cl, R^{4b} est H, R^{4c} est OMe, R^{4d} est H et R^{4e} est H ;
• un composé de formule (50), dans laquelle R³ est -N(CH₂CH₂OMe)₂, R^{4a} est Cl, R^{4b} est H, R^{4c} est OMe, R^{4d} est H et R^{4e} est H ;
• un composé de formule (50), dans laquelle R³ est -NHCH(Et)(CH₂OMe), R^{4a} est Cl, R^{4b} est H, R^{4c} est OMe, R^{4d} est H et R^{4e} est H ;
• un composé de formule (50), dans laquelle R³ est -N(Et)₂, R^{4a} est Cl, R^{4b} est H, R^{4c} est CN, R^{4d} est H et R^{4e} est H ;
• un composé de formule (50), dans laquelle R³ est -N(c-Pr)(CH₂CH₂CN), R^{4a} est Cl, R^{4b} est H, R^{4c} est OMe, R^{4d} est H et R^{4e} est H ;
• un composé de formule (50), dans laquelle R³ est -NHCH(CH₂OH)₂, R^{4a} est Cl, R^{4b} est H, R^{4c} est Cl, R^{4a} est H et R^{4e} est H ; et
• un composé de formule (50), dans laquelle R³ est -N (CH₂CH₂OMe)₂, R^{4a} est Me, R^{4b} est H, R^{4c} est OMe, R^{4d} est H et R^{4e} est H,
et leurs isomères géométriques, leurs formes stéréoisomériques ou mélanges de leurs formes stéréoisomériques et leurs sels pharmaceutiquement acceptables ou promédicaments.

14. [1,5-a]-pyrazolo-1,3,5-triazines selon la revendication 1, dans lesquelles
Ar est 2-chloro-5-fluoro-méthoxyphényle,
R¹ et R² sont méthyles ; et
R³ est NHCH(Et)₂,
et leurs isomères géométriques, leurs formes stéréoisomériques ou mélanges de leurs formes stéréoisomériques et leurs sels pharmaceutiquement acceptables ou promédicaments.

15. [1,5-a]-pyrazolo-1,3,5-triazines de formule (1) 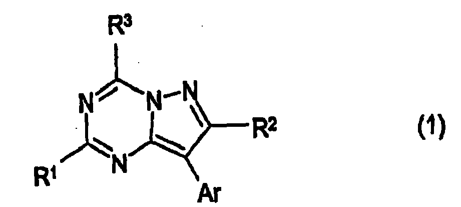 dans laquelle
Ar est phényle, pyridyle ou 2,3-dihydrobenzofuranyle, chaque (Ar) étant le cas échéant substitué par 1 à 4 substituants R⁴ ;
R¹ et R² sont, indépendamment, sélectionné parmi alkyle en C₁-C₄ ;
R³ est NR^{6a}R^{7a} ;
R⁴ est, indépendamment, sélectionné à chaque occurrence parmi : alkyle en C₁-C₁₀, alcényle en C₂-C₁₀, alcynyle en C₂-C₁₀, cycloalkyle en C₃-C₆, cycloalkylalkyle en C₄-C₁₂, NO₂, halogène, CN, halogénoalkyle en C₁-C₄, NR⁶R⁷, NR⁸COR⁷, NR⁶CO₂R⁷, COR⁷, OR⁷, CONR⁶R⁷, CO(NOR⁹)R⁷, CO₂R⁷, ou S(O)ₙR⁷, où chaque alkyle en C₁-C₁₀, alcényle en C₂-C₁₀, alcynyle en C₂-C₁₀, cycloalkyle en C₃-C₆ et cycloalkylalkyle en C₄-C₁₂ de ce type est le cas échéant substitué par 1 à 3 substituants indépendamment sélectionnés à chaque occurrence parmi alkyle en C₁-C₄, NO₂, halogène, CN, NR⁶R⁷, NR⁸COR⁷, NR⁸CO₂R⁷, COR⁷, OR⁷, CONR⁶R⁷, CO₂R⁷, CO(NOR⁹)R⁷, ou S(O)ₙR⁷ ;
R⁶ et R⁷, R^{6a} et R^{7a} sont indépendamment sélectionnés à chaque occurrence parmi :
• H,
• alkyle en C₁-C₁₀, alcényle en C₃-C₁₀, alcynyle en C₃-C₁₀, halogénoalkyle en C₁-C₁₀ avec 1 à 10 halogènes, alcoxyalkyle en C₂-C₈, cycloalkyle en C₃-C₆, cycloalkylalkyle en C₄-C₁₂, cycloalcényle en C₅-C₁₀ ou cycloalcénylalkyle en C₈-C₁₄, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹³, COR¹⁵, CO₂R¹⁵, OC(O)R¹³, NR⁸COR¹⁵, N(COR¹⁵)₂, NR⁸CONR¹⁶R¹⁵, NR⁸CO₂R¹³, NR¹⁶R¹⁵, CONR¹⁶R¹⁵, aryle, hétéroaryle ou hétérocyclyle,
• aryle, aryl(alkyle en C₁-C₄), hétéroaryle, hétéroaryl(alkyle en C₁-C₄), hétérocyclyle ou hétérocyclyl(alkyle en C₁-C₄) ;
en variante, NR⁶R⁷ et NR^{6a}R^{7a} sont indépendamment pipéridine, pyrrolidine, pipérazine, N-méthylpipérazine, morpholine ou thiomorpholine, chacun le cas échéant substitué par 1 à 3 groupes alkyle en C₁-C₄ ;
R⁸ est indépendamment sélectionné à chaque occurrence parmi H ou alkyle en C₁-C₄;
R⁹ et R¹⁰ sont, indépendamment, sélectionnés à chaque occurrence parmi H, alkyle en C₁-C₄ ou cycloalkyle en C₃-C₆ ;
R¹¹ est sélectionné parmi H, alkyle en C₁-C₄, halogénoalkyle en C₁-C₄ ou cycloalkyle en C₃-C₆ ;
R¹² est alkyle en C₁-C₄ ou halogénoalkyle en C₁-C₄ ;
R¹³ est sélectionné parmi alkyle en C₁-C₄, halogénoalkyle en C₁-C₄, alcoxyalkyle en C₂-C₈, cycloalkyle en C₃-C₆, cycloalkylalkyle en C₄-C₁₂, aryle, aryl(alkyle en C₁-C₄), hétéroaryle ou hétéroaryl(alkyle en C₁-C₄),
R¹⁴ est sélectionné parmi alkyle en C₁-C₁₀, alcényle en C₃-C₁₀, alcynyle en C₃-C₁₀, cycloalkyle en C₃-C₈ ou cycloalkylalkyle en C₄-C₁₂, chacun étant le cas échéant substitué par 1 à 3 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁸, NR⁸CO₂R¹⁵, NR¹⁶R¹⁵, CONR¹⁶R¹⁵ et alkylthio en C₁-C₆, alkylsulfinyle en C₁-C₆ et alkylsulfonyle en C₁-C₆;
R¹⁵ et R¹⁶ sont, indépendamment, sélectionnés à chaque occurrence parmi H, alkyle en C₁-C₆, cycloalkyle en C₃-C₁₀, cycloalkylalkyle en C₄-C₁₆, sauf que, pour S(O)ₙR¹⁵, R¹⁵ ne peut être H ;
aryle est phényle ou naphtyle, chacun le cas échéant substitué par 1 à 5 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁶R¹⁵ et CONR¹⁶R¹⁵ ;
hétéroaryle est pyridyle, pyrimidinyle, triazinyle, furanyle, pyranyle, quinolinyle, isoquinolinyle, thiényle, imidazolyle, thiazolyle, indolyle, pyrrolyle, oxazolyle, benzofuranyle, benzothiényle, benzothiazolyle, isoxazolyle, pyrazolyle, 2,3-dihydrobenzothiényle ou 2,3-dihydrobenzofuranyle, chacun étant le cas échéant substitué par 1 à 5 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, -COR¹⁵, CO₂R¹⁵, CO(O)R¹⁵, NR⁸COR¹⁵, N(COR¹⁵)₂, NR⁸CONR¹⁶R¹⁵, NR⁸CO₂R¹⁵, NR¹⁶R¹⁵ et CONR¹⁶R¹⁵ ;
hétérocyclyle est un hétéroaryle saturé ou partiellement saturé, le cas échéant substitué par 1 à 5 substituants indépendamment sélectionnés à chaque occurrence parmi alkyle en C₁-C₆, cycloalkyle en C₃-C₆, halogène, halogénoalkyle en C₁-C₄, cyano, OR¹⁵, SH, S(O)ₙR¹⁵, COR¹⁵, CO₂R¹⁵, OC(O)R¹⁵, NR⁸COR¹⁵, N(COR¹⁵)₂, NR⁸CONR¹⁶R¹⁵, NR⁸CO₂R¹⁵, NR¹⁵R¹⁶ et CONR¹⁶R¹⁵ ;
n est indépendamment à chaque occurrence 0, 1 ou 2,
et leurs isomères géométriques, leurs formes stéréoisomériques ou mélanges de leurs formes stéréoisomériques et leurs sels pharmaceutiquement acceptables ou promédicaments.

16. [1,5-a]-pyrazolo-1,3,5-triazines selon la revendication 15, dans lesquelles
Ar est phényle, pyridyle ou 2,3-dihydrobenzofuranyle, chaque (Ar) étant substitué par 1 à 4 substituants R⁴,
et leurs isomères géométriques, leurs formes stéréoisomériques ou mélanges de leurs formes stéréoisomériques et leurs sels pharmaceutiquement acceptables ou promédicaments.

17. [1,5-a]-pyrazolo-1,3,5-triazines selon la revendication 15 ou 16, dans lesquelles
Ar est phényle ou pyridyle, chaque (Ar) étant le cas échéant substitué par 1 à 4 substituants R⁴,
et leurs isomères géométriques, leurs formes stéréoisomériques ou mélanges de leurs formes stéréoisomériques et leurs sels pharmaceutiquement acceptables ou promédicaments.

18. [1,5-a]-pyrazolo-1,3,5-triazines selon l'une quelconque des revendications 15 à 17, dans lesquelles
R^{6a} et R^{7a} sont indépendamment H ou alkyle en C₁-C₁₀,

19. Composition pharmaceutique, comprenant une [1,5-a]-pyrazolo-1,3,5-triazine de formule (1) selon l'une quelconque des revendications 1 à 18 ou son isomère géométrique, sa forme stéréoisomérique, ou un mélange de ses formes stéréoisomériques, ou son sel pharmaceutiquement acceptable ou promédicament.

20. Utilisation des [1,5-a]-pyrazolo-1,3,5-triazines de formule (1) selon l'une quelconque des revendications 1 à 18 ou son isomère géométrique, sa forme stéréoisomérique, ou un mélange de ses formes stéréoisomériques, ou son sel pharmaceutiquement acceptable ou promédicament, dans la fabrication d'un médicament destiné à traiter les troubles affectifs, l'anxiété, la dépression, les migraines, le syndrome de l'intestin irritable, les troubles de stress post-traumatique, la paralysie supranucléaire, l'immunosuppression, la maladie d'Alzheimer, les maladies gastro-intestinales, l'anorexie nerveuse ou autres troubles de l'alimentation, la dépendance aux drogues, les symptômes de sevrage des drogues ou de l'alcool, les maladies inflammatoires, les maladies cardiovasculaires ou associées au coeur, les problèmes de fertilité, les infections dues au virus de l'immunodéficience humaine, le stress hémorragique, l'obésité, l'infertilité, les traumas du cerveau et de la moelle épinière, l'épilepsie, les attaques, les ulcères, la sclérose latérale amyotrophique ou l'hypoglycémie.

21. Utilisation selon la revendication 20, pour traiter les troubles affectifs, l'anxiété, la dépression, les migraines, le syndrome de l'intestin irritable, les troubles de stress post-traumatique, la paralysie supranucléaire, l'immunosuppression, la maladie d'Alzheimer, les maladies gastro-intestinales, l'anorexie nerveuse ou autres troubles de l'alimentation, la dépendance aux drogues, les symptômes de sevrage des drogues ou de l'alcool, les maladies inflammatoires ou les problèmes de fertilité.

22. Utilisation selon la revendication 21, pour traiter l'anxiété.
